(19) Europäisches Patentamt
European Patent Office
Office européen des brevets

(11) EP 1 489 168 A1

(12) EUROPEAN PATENT APPLICATION
published in accordance with Art. 158(3) EPC

(43) Date of publication:
**22.12.2004 Bulletin 2004/52**

(21) Application number: **02703964.3**

(22) Date of filing: **11.03.2002**

(51) Int Cl.[7]: **C12N 15/09**, C07K 14/475, C12N 5/10, A61K 38/17, A61P 43/00, A61K 35/14, A61K 35/12

(86) International application number:
**PCT/JP2002/002265**

(87) International publication number:
**WO 2003/076613 (18.09.2003 Gazette 2003/38)**

(84) Designated Contracting States:
**AT BE CH CY DE DK ES FI FR GB GR IE IT LI LU MC NL PT SE TR**
Designated Extension States:
**AL LT LV MK RO SI**

(71) Applicants:
- **ReproCELL Inc.**
**Tokyo 100-0011 (JP)**
- **Ema, Hideo**
**Ushiku-shi, Ibaraki 300-1203 (JP)**

(72) Inventors:
- **EMA, Hideo**
**Ushiku-shi, Ibaraki 300-1203 (JP)**
- **NAKAUCHI, Hiromitsu**
**Inashiki-gun, Ibaraki 300-1243 (JP)**
- **OSAWA, Mitsujiro**
**Tsukuba-shi, Ibaraki 305-0821 (JP)**

(74) Representative: **Holliday, Louise Caroline**
**D Young & Co**
**120 Holborn**
**London EC1N 2DY (GB)**

(54) **PROTEIN SUSTAINING UNDIFFERENTIATED STEM CELLS AS SUCH**

(57) The sustaining stem cells for example, hematopoietic stem cells while holding in the undifferentiated state and maintaining pluripotency by providing a polypeptide having a WIF domain for example, WIF-1 to the stem cells has been successfully achieved. This polypeptide preferably also contains an EGF-like repeat. This result has been achieved by providing a polypeptide preferably having the WIF domain together with a stem cell survival factor such as SCF. Thus, it becomes possible to easily provide a large amount of stem cells for example, hematopoietic stem cells containing functional precursor cells. It has also become possible to more efficiently transfer a gene into the stem cells. Because it is homogeneous and almost free from impurities, the stem cell composition can remarkably relieve side effects for example, infection, which occur in conventional therapies using hematopoietic stem cells.

EP 1 489 168 A1

SEQUENCE LISTING

```
<110> ReproCELL Inc., and EMA, Hideo

<120> PROTEIN SUSTAINING UNDIFFERENTIATED STEM CELLS

<130> TR001PCT

<140> PCT/JP02/02265
<141> 2002-3-11

<160> 25

<170> PatentIn Ver. 2.1

<210> 1
<211> 1140
<212> DNA
<213> Mus musculus

<400> 1
atggctcgga gaagagcctt ccctgctttc gcgctccggc tctggagcat cctaccttgc 60
ctgctcctgc tgcgagcgga tgcagggcag ccacctgagg agagcttgta cctgtggatc 120
gacgcccatc aggctagagt gctcatagga tttgaagaag acattctgat tgtctcggag 180
gggaaaatgg ccccctttac acatgatttc aggaaagccc aacaaagaat gccagccatt 240
cctgtcaata tccactccat gaattttacc tggcaagctg cggggcaggc agaatacttc 300
tacgagttcc tgtctctgcg ctccctggat aaaggcatca tggcagatcc aactgtcaat 360
gtccctttgc tgggaacagt gcctcacaag gcatcagttg ttcaagttgg tttcccgtgt 420
ctcggcaaac aagacggggt agcagcattt gaagtgaatg tgattgtcat gaattctgaa 480
ggcaacacca tccttaggac ccctcagaat gccatcttct ttaaaacatg tcaacaagct 540
gagtgtcccg gagggtgtcg aaatggaggc ttttgtaacg aaaggcgggt ctgcgagtgt 600
ccggatgggt tctacgggcc tcactgtgag aaagccctgt gcataccccg atgtatgaac 660
ggtggtctgt gtgtcactcc tggcttctgc atctgccccc ctggattcta cggtgtcaac 720
tgtgacaaag caaactgctc aaccacctgc tttaatggag ggacctgctt ttacccggga 780
aaatgtattt gccctcctgg actcgaggga gagcagtgtg aactcagcaa atgcccccaa 840
ccctgccgaa atggaggtaa atgcattggt aaaagcaagt gtaagtgccc gaaaggttac 900
caaggagacc tgtgctctaa gcccgtctgc gagcctggct gtggtgccca cggaacctgc 960
cacgaaccca acaagtgcca gtgtcgagag ggctggcacg gcagacactg caataagagg 1020
tatggagcca gcctcatgca tgccccgagg ccagcaggcg ccgggctgga gcgacacacg 1080
ccttcactta aaaaggctga ggatagaagg gatccacctg aatccaatta catctggtga 1140


<210> 2
<211> 379
<212> PRT
<213> Mus musculus

<400> 2
Met Ala Arg Arg Arg Ala Phe Pro Ala Phe Ala Leu Arg Leu Trp Ser
1               5                   10                  15

Ile Leu Pro Cys Leu Leu Leu Leu Arg Ala Asp Ala Gly Gln Pro Pro
            20                  25                  30

Glu Glu Ser Leu Tyr Leu Trp Ile Asp Ala His Gln Ala Arg Val Leu
        35                  40                  45

Ile Gly Phe Glu Glu Asp Ile Leu Ile Val Ser Glu Gly Lys Met Ala
    50                  55                  60

Pro Phe Thr His Asp Phe Arg Lys Ala Gln Gln Arg Met Pro Ala Ile
65                  70                  75                  80

Pro Val Asn Ile His Ser Met Asn Phe Thr Trp Gln Ala Ala Gly Gln
                85                  90                  95

Ala Glu Tyr Phe Tyr Glu Phe Leu Ser Leu Arg Ser Leu Asp Lys Gly
            100                 105                 110

Ile Met Ala Asp Pro Thr Val Asn Val Pro Leu Leu Gly Thr Val Pro
        115                 120                 125

His Lys Ala Ser Val Val Gln Val Gly Phe Pro Cys Leu Gly Lys Gln
    130                 135                 140

Asp Gly Val Ala Ala Phe Glu Val Asn Val Ile Val Met Asn Ser Glu
145                 150                 155                 160

Gly Asn Thr Ile Leu Arg Thr Pro Gln Asn Ala Ile Phe Phe Lys Thr
                165                 170                 175
```

```
Cys Gln Gln Ala Glu Cys Pro Gly Gly Cys Arg Asn Gly Gly Phe Cys
            180                 185                 190

Asn Glu Arg Arg Val Cys Glu Cys Pro Asp Gly Phe Tyr Gly Pro His
        195                 200                 205

Cys Glu Lys Ala Leu Cys Ile Pro Arg Cys Met Asn Gly Gly Leu Cys
    210                 215                 220

Val Thr Pro Gly Phe Cys Ile Cys Pro Pro Gly Phe Tyr Gly Val Asn
225                 230                 235                 240

Cys Asp Lys Ala Asn Cys Ser Thr Thr Cys Phe Asn Gly Gly Thr Cys
                245                 250                 255

Phe Tyr Pro Gly Lys Cys Ile Cys Pro Pro Gly Leu Glu Gly Glu Gln
            260                 265                 270

Cys Glu Leu Ser Lys Cys Pro Gln Pro Cys Arg Asn Gly Gly Lys Cys
        275                 280                 285

Ile Gly Lys Ser Lys Cys Lys Cys Pro Lys Gly Tyr Gln Gly Asp Leu
    290                 295                 300

Cys Ser Lys Pro Val Cys Glu Pro Gly Cys Gly Ala His Gly Thr Cys
305                 310                 315                 320

His Glu Pro Asn Lys Cys Gln Cys Arg Glu Gly Trp His Gly Arg His
                325                 330                 335

Cys Asn Lys Arg Tyr Gly Ala Ser Leu Met His Ala Pro Arg Pro Ala
            340                 345                 350

Gly Ala Gly Leu Glu Arg His Thr Pro Ser Leu Lys Lys Ala Glu Asp
        355                 360                 365

Arg Arg Asp Pro Pro Glu Ser Asn Tyr Ile Trp
    370                 375


<210> 3
<211> 1140
<212> DNA
<213> Homo sapiens

<400> 3
atggcccgga ggagcgcctt ccctgccgcc gcgctctggc tctggagcat cctcctgtgc 60
ctgctggcac tgcgggcgga ggccgggccg ccgcaggagg agagcctgta cctatggatc 120
gatgctcacc aggcaagagt actcatagga tttgaagaag atatcctgat tgtttcagag 180
gggaaaatgg caccttttac acatgatttc agaaaagcgc aacagagaat gccagctatt 240
cctgtcaata tccattccat gaattttacc tggcaagctg cagggcaggc agaatacttc 300
tatgaattcc tgtccttgcg ctccctggat aaaggcatca tggcagatcc aaccgtcaat 360
gtccctctgc tgggaacagt gcctcacaag gcatcagttg ttcaagttgg tttcccatgt 420
cttggaaaac aggatggggt ggcagcattt gaagtggatg tgattgttat gaattctgaa 480
ggcaacacca ttctccaaac acctcaaaat gctatcttct ttaaaacatg tctacaagct 540
gagtgcccag gcgggtgccg aaatggaggc ttttgtaatg aaagacgcat ctgcgagtgt 600
cctgatgggt tccacggacc tcactgtgag aaagcccttt gtaccccacg atgtatgaat 660
ggtggacttt gtgtgactcc tggtttctgc atctgcccac ctggattcta tggagtgaac 720
tgtgacaaag caaactgctc aaccacctgc tttaatggag ggacctgttt ctaccctgga 780
aaatgtattt gccctccagg actagaggga gagcagtgtg aaatcagcaa atgcccacaa 840
ccctgtcgaa atggaggtaa atgcattggt aaaagcaaat gtaagtgttc caaaggttac 900
cagggagacc tctgttcaaa gcctgtctgc gagcctggct gtggtgcaca tggaacctgc 960
catgaaccca acaaatgcca atgtcaagaa ggttggcatg gaagacactg caataaaagg 1020
tacgaagcca gcctcataca tgccctgagg ccagcaggcg cccagctcag gcagcacacg 1080
ccttcactta aaaaggccga ggagcggcgg gatccacctg aatccaatta catctggtga 1140


<210> 4
<211> 379
<212> PRT
<213> Homo sapiens

<400> 4
Met Ala Arg Arg Ser Ala Phe Pro Ala Ala Ala Leu Trp Leu Trp Ser
1               5                   10                  15

Ile Leu Leu Cys Leu Leu Ala Leu Arg Ala Glu Ala Gly Pro Pro Gln
            20                  25                  30
```

```
Glu Glu Ser Leu Tyr Leu Trp Ile Asp Ala His Gln Ala Arg Val Leu
        35                  40                  45

Ile Gly Phe Glu Glu Asp Ile Leu Ile Val Ser Glu Gly Lys Met Ala
    50                  55                  60

Pro Phe Thr His Asp Phe Arg Lys Ala Gln Gln Arg Met Pro Ala Ile
65                  70                  75                  80

Pro Val Asn Ile His Ser Met Asn Phe Thr Trp Gln Ala Ala Gly Gln
                85                  90                  95

Ala Glu Tyr Phe Tyr Glu Phe Leu Ser Leu Arg Ser Leu Asp Lys Gly
            100                 105                 110

Ile Met Ala Asp Pro Thr Val Asn Val Pro Leu Leu Gly Thr Val Pro
        115                 120                 125

His Lys Ala Ser Val Val Gln Val Gly Phe Pro Cys Leu Gly Lys Gln
    130                 135                 140

Asp Gly Val Ala Ala Phe Glu Val Asp Val Ile Val Met Asn Ser Glu
145                 150                 155                 160

Gly Asn Thr Ile Leu Gln Thr Pro Gln Asn Ala Ile Phe Phe Lys Thr
                165                 170                 175

Cys Leu Gln Ala Glu Cys Pro Gly Gly Cys Arg Asn Gly Gly Phe Cys
            180                 185                 190

Asn Glu Arg Arg Ile Cys Glu Cys Pro Asp Gly Phe His Gly Pro His
        195                 200                 205

Cys Glu Lys Ala Leu Cys Thr Pro Arg Cys Met Asn Gly Gly Leu Cys
    210                 215                 220

Val Thr Pro Gly Phe Cys Ile Cys Pro Pro Gly Phe Tyr Gly Val Asn
225                 230                 235                 240

Cys Asp Lys Ala Asn Cys Ser Thr Thr Cys Phe Asn Gly Gly Thr Cys
                245                 250                 255

Phe Tyr Pro Gly Lys Cys Ile Cys Pro Pro Gly Leu Glu Gly Glu Gln
            260                 265                 270

Cys Glu Ile Ser Lys Cys Pro Gln Pro Cys Arg Asn Gly Gly Lys Cys
        275                 280                 285

Ile Gly Lys Ser Lys Cys Lys Cys Ser Lys Gly Tyr Gln Gly Asp Leu
    290                 295                 300

Cys Ser Lys Pro Val Cys Glu Pro Gly Cys Gly Ala His Gly Thr Cys
305                 310                 315                 320

His Glu Pro Asn Lys Cys Gln Cys Gln Glu Gly Trp His Gly Arg His
                325                 330                 335

Cys Asn Lys Arg Tyr Glu Ala Ser Leu Ile His Ala Leu Arg Pro Ala
            340                 345                 350

Gly Ala Gln Leu Arg Gln His Thr Pro Ser Leu Lys Lys Ala Glu Glu
        355                 360                 365

Arg Arg Asp Pro Pro Glu Ser Asn Tyr Ile Trp
    370                 375
```

<210> 5
<211> 1098
<212> DNA
<213> Rattus norvegicus

<400> 5

```
atggcccgga gaagagcctt ccctgctttc gtgctccggc tctggagcat cctaccttgc 60
ctgctcctgc tacgagcgga tgcagggcag ccgccagagg agagcttgta cctgtggatc 120
gacgcccatc aggccagagt actcatagga tttgaagaag atattctgat tgtctcggag 180
gggaaaatgg cccccttac acatgatttc aggaaagccc aacaaagaat gccagccatt 240
cccgtcaata tccactccat gaattttacc tggcaagctt cagggcaggc agagtacttc 300
tatgagttcc tgtcgctgcg ctcgctggat aaaggcatca tggcagaccc aactgtcaat 360
gtccctcggc tgggaacagt gcctcacaag gcatcagttg ttcaagttgg tttcccgtgt 420
```

```
ctcggcaaac aggatggggt ggcagcattt gaagtgaatg tgattgtcat gaattctgaa 480
ggcaacccca tccttcggac ccctcaaaat gctatcttct ttaaaacatg tcaacaagct 540
gagtgcccag gagggtgtcg aaatggaggc ttttgtaacg aaaggcgggt ctgcgagtgt 600
cccgatgggt tctatggacc tcactgtgag aaagccctct gcatacctcg atgtatgaac 660
ggtggtctgt gtgtcactcc tggcttctgc atctgcccgc ctggattcta cggtgtcaac 720
tgtgacaaag caaactgctc ggccacctgc tttaatggag ggacctgttt ttacccagga 780
aaatgtattt gccctccagg acttgaggga gagcagtgtg aactcagcaa gtgcccccaa 840
ccctgccgaa acggaggtaa atgcattggt aaaagcaagt ctgtctgcga gcctggctgc 900
ggtgcccatg gaacctgcca cgaacccaac aaatgccagt gtcgagaggg ctggcatggg 960
agacactgca ataaaaggta cggagccagc ctcatgcatg ccccgaggcc agcaggcgcc 1020
gggctggagc ggcacacgcc ttcacttaaa aaggctgagg ggcggaggga tccacctgaa 1080
tccaattaca tctggtga                                               1098
```

<210> 6
<211> 365
<212> PRT
<213> Rattus norvegicus

<400> 6

```
Met Ala Arg Arg Arg Ala Phe Pro Ala Phe Val Leu Arg Leu Trp Ser
1               5                   10                  15

Ile Leu Pro Cys Leu Leu Leu Leu Arg Ala Asp Ala Gly Gln Pro Pro
            20                  25                  30

Glu Glu Ser Leu Tyr Leu Trp Ile Asp Ala His Gln Ala Arg Val Leu
        35                  40                  45

Ile Gly Phe Glu Glu Asp Ile Leu Ile Val Ser Glu Gly Lys Met Ala
    50                  55                  60

Pro Phe Thr His Asp Phe Arg Lys Ala Gln Gln Arg Met Pro Ala Ile
65                  70                  75                  80

Pro Val Asn Ile His Ser Met Asn Phe Thr Trp Gln Ala Ser Gly Gln
                85                  90                  95

Ala Glu Tyr Phe Tyr Glu Phe Leu Ser Leu Arg Ser Leu Asp Lys Gly
            100                 105                 110

Ile Met Ala Asp Pro Thr Val Asn Val Pro Arg Leu Gly Thr Val Pro
        115                 120                 125

His Lys Ala Ser Val Val Gln Val Gly Phe Pro Cys Leu Gly Lys Gln
    130                 135                 140

Asp Gly Val Ala Ala Phe Glu Val Asn Val Ile Val Met Asn Ser Glu
145                 150                 155                 160

Gly Asn Pro Ile Leu Arg Thr Pro Gln Asn Ala Ile Phe Phe Lys Thr
                165                 170                 175

Cys Gln Gln Ala Glu Cys Pro Gly Gly Cys Arg Asn Gly Gly Phe Cys
            180                 185                 190

Asn Glu Arg Arg Val Cys Glu Cys Pro Asp Gly Phe Tyr Gly Pro His
        195                 200                 205

Cys Glu Lys Ala Leu Cys Ile Pro Arg Cys Met Asn Gly Gly Leu Cys
    210                 215                 220

Val Thr Pro Gly Phe Cys Ile Cys Pro Pro Gly Phe Tyr Gly Val Asn
225                 230                 235                 240

Cys Asp Lys Ala Asn Cys Ser Ala Thr Cys Phe Asn Gly Gly Thr Cys
                245                 250                 255

Phe Tyr Pro Gly Lys Cys Ile Cys Pro Pro Gly Leu Glu Gly Glu Gln
            260                 265                 270

Cys Glu Leu Ser Lys Cys Pro Gln Pro Cys Arg Asn Gly Gly Lys Cys
        275                 280                 285

Ile Gly Lys Ser Lys Ser Val Cys Glu Pro Gly Cys Gly Ala His Gly
    290                 295                 300

Thr Cys His Glu Pro Asn Lys Cys Gln Cys Arg Glu Gly Trp His Gly
305                 310                 315                 320
```

Arg His Cys Asn Lys Arg Tyr Gly Ala Ser Leu Met His Ala Pro Arg
325 330 335

Pro Ala Gly Ala Gly Leu Glu Arg His Thr Pro Ser Leu Lys Lys Ala
340 345 350

Glu Gly Arg Arg Asp Pro Pro Glu Ser Asn Tyr Ile Trp
355 360 365

<210> 7
<211> 1125
<212> DNA
<213> Xenopus sp.

<400> 7
atgtctctaa caggctactt tgcagctccg ctatgcagca tctttctgtt tattcttgca 60
catgcagatg ctgggcagca ggaggacagt ttgtacatgt ggattgatgc tcaccaagcc 120
agagtattaa taggctttga ggaggatatt ctgattgttg cagaggggaa aatggcacca 180
tttacgcatg attttagaaa agcccagcag cgaatgccag ccatacctgt caatatccat 240
gccatgaatt ttacttggca ggcaacaggg caggcagaat acttttatga gtttttatca 300
ctgcggtcac tagataaagg aatcatggct gatccaactg tgaatatgcc attactggga 360
acagtgccgc acaaagctac agttatacag gttgggtttc cttgccttgg aaatcaggac 420
ggtgttgctg cctttgaggt gaatgtaatt gttatgaact cagaaggcaa tgtgattctt 480
cagactccac agaatgctat ctttttcaag acttgccagc aagcaaaatg tacaggagga 540
tgcagaaatg gaggtttctg caatgatagg catgtctgtg aatgtcctga tggcttttat 600
ggcccacatt gtgagaaagc actctgcatg ccacgatgta tgaatggtgg gctctgtgta 660
actcctggat tgtgcatctg tccacctggc tattatggca tcaactgtga taaagtaaac 720
tgcaccacac attgtctgaa tggggaaccc tgcttctatc caggaaagtg catttgcccc 780
tcaggatatg aaggagaaca gtgtgaaaca agtaaatgcc agcaaccctg taggaatggt 840
ggaaaatgta gtggaaaaaa caaatgcaag tgttccaagg gataccaagg agatctgtgt 900
tcaaaacctg ttgtgaaccc ttcttgtgga gctcatggaa cctgcattga acccaacaag 960
tgtcagtgta aagaaggctg gaatggaaga tactgcaata agaaatacgg atccaatctc 1020
atgaatgccc taaggccaac agggtccaga aacagacagc acacgccctc accaaaacgg 1080
actgaagaca ggcaagccct acccgaatcc aattatatct ggtga 1125

<210> 8
<211> 374
<212> PRT
<213> Xenopus sp.

<400> 8
Met Ser Leu Thr Gly Tyr Phe Ala Ala Pro Leu Cys Ser Ile Phe Leu
1 5 10 15

Phe Ile Leu Ala His Ala Asp Ala Gly Gln Gln Glu Asp Ser Leu Tyr
20 25 30

Met Trp Ile Asp Ala His Gln Ala Arg Val Leu Ile Gly Phe Glu Glu
35 40 45

Asp Ile Leu Ile Val Ala Glu Gly Lys Met Ala Pro Phe Thr His Asp
50 55 60

Phe Arg Lys Ala Gln Gln Arg Met Pro Ala Ile Pro Val Asn Ile His
65 70 75 80

Ala Met Asn Phe Thr Trp Gln Ala Thr Gly Gln Ala Glu Tyr Phe Tyr
85 90 95

Glu Phe Leu Ser Leu Arg Ser Leu Asp Lys Gly Ile Met Ala Asp Pro
100 105 110

Thr Val Asn Met Pro Leu Leu Gly Thr Val Pro His Lys Ala Thr Val
115 120 125

Ile Gln Val Gly Phe Pro Cys Leu Gly Asn Gln Asp Gly Val Ala Ala
130 135 140

Phe Glu Val Asn Val Ile Val Met Asn Ser Glu Gly Asn Val Ile Leu
145 150 155 160

Gln Thr Pro Gln Asn Ala Ile Phe Phe Lys Thr Cys Gln Gln Ala Lys
165 170 175

Cys Thr Gly Gly Cys Arg Asn Gly Gly Phe Cys Asn Asp Arg His Val
180 185 190

Cys Glu Cys Pro Asp Gly Phe Tyr Gly Pro His Cys Glu Lys Ala Leu

195 200 205

Cys Met Pro Arg Cys Met Asn Gly Gly Leu Cys Val Thr Pro Gly Leu
210 215 220

Cys Ile Cys Pro Pro Gly Tyr Tyr Gly Ile Asn Cys Asp Lys Val Asn
225 230 235 240

Cys Thr Thr His Cys Leu Asn Gly Gly Thr Cys Phe Tyr Pro Gly Lys
245 250 255

Cys Ile Cys Pro Ser Gly Tyr Glu Gly Glu Gln Cys Glu Thr Ser Lys
260 265 270

Cys Gln Gln Pro Cys Arg Asn Gly Gly Lys Cys Ser Gly Lys Asn Lys
275 280 285

Cys Lys Cys Ser Lys Gly Tyr Gln Gly Asp Leu Cys Ser Lys Pro Val
290 295 300

Cys Glu Pro Ser Cys Gly Ala His Gly Thr Cys Ile Glu Pro Asn Lys
305 310 315 320

Cys Gln Cys Lys Glu Gly Trp Asn Gly Arg Tyr Cys Asn Lys Lys Tyr
325 330 335

Gly Ser Asn Leu Met Asn Ala Leu Arg Pro Thr Gly Ser Arg Asn Arg
340 345 350

Gln His Thr Pro Ser Pro Lys Arg Thr Glu Asp Arg Gln Ala Leu Pro
355 360 365

Glu Ser Asn Tyr Ile Trp
370

<210> 9
<211> 1137
<212> DNA
<213> Danio rerio

<400> 9
atggctttca ggacgcctgc tgttcagctg caccttaaag cgtgcgtcct tctgctttta 60
ggggggtctgc tggaagctgc ctatcaagag cgaggaacca tgtacatgtg gattgatgcc 120
aatcaagcaa gaatattaat tggttttgaa gaggatattt taatagtctc tgaggggaaa 180
atggctccat tcacacacga cttcaggaaa gcacagcaga gaatgccagc gatacctgtc 240
aatatccatc acgtcaactt cacctggcaa gccactgatc aggcagagta tttttatgag 300
ttccagactc tgcgttcttt ggataaagac attatggatg atcctactgt caatgttcct 360
cttttgggat cagtgcctca caaagcatct gtggttcagg tgggctttcc atgcagaggt 420
gaccaggacg gtgtggcagc atttgaggtg accatcctgg tgatggatgc tggaggaaat 480
atcatcctga ggacgccaca caatgccatc ttcttcaaaa catgccagag agcaaagtgt 540
cctggaggtt gtcgaaatgg aggctactgc aatgaaaggc aggtctgcga gtgtcaggat 600
gggttttatg gcgttcactg tgagaaagcc ctgtgctctc caagatgcct aaatggtggt 660
ctgtgtatga gtccaggcgt gtgtatttgt ccaccgggct actttggatc cagttgtgag 720
agagcaaact gcagcaccac ctgtttgaat ggagggacgt gtttccatcc tggcaaatgc 780
atctgcgccg tgagctttga aggggtccgc tgtgagctca gtaaatgtcg acagccttgc 840
aggaatggag gcaaatgcac agggagaaac aaatgcaagt gcagcaaagg atatcatgga 900
gatctgtgct ccaaagctgt ctgcgagccc agctgtggag cacacgggac ctgcgtggag 960
cccaacaggt gccagtgtcg ggaaggctgg cacggacgtc actgcaataa gagatttcgc 1020
ggaggagttt ctaacagcca gcgggtctct ccatccaaac acaagtctcc gtctgtggcg 1080
gcggcgaagg aagcgcctga aaccagtcag ccgtctgaaa ccaactatgt ggtttaa 1137

<210> 10
<211> 378
<212> PRT
<213> Danio rerio

<400> 10
Met Ala Phe Arg Thr Pro Ala Val Gln Leu His Leu Lys Ala Cys Val
1 5 10 15

Leu Leu Leu Leu Gly Gly Leu Leu Glu Ala Ala Tyr Gln Glu Arg Gly
20 25 30

Thr Met Tyr Met Trp Ile Asp Ala Asn Gln Ala Arg Ile Leu Ile Gly
35 40 45

Phe Glu Glu Asp Ile Leu Ile Val Ser Glu Gly Lys Met Ala Pro Phe
50 55 60

Thr His Asp Phe Arg Lys Ala Gln Gln Arg Met Pro Ala Ile Pro Val
65 70 75 80

Asn Ile His His Val Asn Phe Thr Trp Gln Ala Thr Asp Gln Ala Glu
85 90 95

Tyr Phe Tyr Glu Phe Gln Thr Leu Arg Ser Leu Asp Lys Asp Ile Met
100 105 110

Asp Asp Pro Thr Val Asn Val Pro Leu Leu Gly Ser Val Pro His Lys
115 120 125

Ala Ser Val Val Gln Val Gly Phe Pro Cys Arg Gly Asp Gln Asp Gly
130 135 140

Val Ala Ala Phe Glu Val Thr Ile Leu Val Met Asp Ala Gly Gly Asn
145 150 155 160

Ile Ile Leu Arg Thr Pro His Asn Ala Ile Phe Phe Lys Thr Cys Gln
165 170 175

Arg Ala Lys Cys Pro Gly Gly Cys Arg Asn Gly Gly Tyr Cys Asn Glu
180 185 190

Arg Gln Val Cys Glu Cys Gln Asp Gly Phe Tyr Gly Val His Cys Glu
195 200 205

Lys Ala Leu Cys Ser Pro Arg Cys Leu Asn Gly Gly Leu Cys Met Ser
210 215 220

Pro Gly Val Cys Ile Cys Pro Pro Gly Tyr Phe Gly Ser Ser Cys Glu
225 230 235 240

Arg Ala Asn Cys Ser Thr Thr Cys Leu Asn Gly Gly Thr Cys Phe His
245 250 255

Pro Gly Lys Cys Ile Cys Ala Val Ser Phe Glu Gly Val Arg Cys Glu
260 265 270

Leu Ser Lys Cys Arg Gln Pro Cys Arg Asn Gly Gly Lys Cys Thr Gly
275 280 285

Arg Asn Lys Cys Lys Cys Ser Lys Gly Tyr His Gly Asp Leu Cys Ser
290 295 300

Lys Ala Val Cys Glu Pro Ser Cys Gly Ala His Gly Thr Cys Val Glu
305 310 315 320

Pro Asn Arg Cys Gln Cys Arg Glu Gly Trp His Gly Arg His Cys Asn
325 330 335

Lys Arg Phe Arg Gly Gly Val Ser Asn Ser Gln Arg Val Ser Pro Ser
340 345 350

Lys His Lys Ser Pro Ser Val Ala Ala Ala Lys Glu Ala Pro Glu Thr
355 360 365

Ser Gln Pro Ser Glu Thr Asn Tyr Val Val
370 375

<210> 11
<211> 26
<212> DNA
<213> Artificial Sequence

<220>
<223> Description of Artificial Sequence:Artificial Sequence

<400> 11
tgyathtgyc cnccnggntw bwvbcd 26

<210> 12
<211> 26
<212> DNA
<213> Artificial Sequence

<220>
<223> Description of Artificial Sequence:Artificial
Sequence

<400> 12
rcgnctnccn ccrtcnskrc angbcd 26

<210> 13
<211> 4817
<212> DNA
<213> Artificial Sequence

<220>
<223> Description of Artificial Sequence:pCAGGS 6xHis construct

<400> 13
gtcgacattg attattgact agttattaat agtaatcaat tacggggtca ttagttcata 60
gcccatatat ggagttccgc gttacataac ttacggtaaa tggcccgcct ggctgaccgc 120
ccaacgaccc ccgcccattg acgtcaataa tgacgtatgt tcccatagta acgccaatag 180
ggactttcca ttgacgtcaa tgggtggact atttacggta aactgcccac ttggcagtac 240
atcaagtgta tcatatgcca agtacgcccc ctattgacgt caatgacggt aaatggcccg 300
cctggcatta tgcccagtac atgaccttat gggactttcc tacttggcag tacatctacg 360
tattagtcat cgctattacc atgggtcgag gtgagcccca cgttctgctt cactctcccc 420
atctccccc cctccccacc cccaattttg tatttattta tttttaatt attttgtgca 480
gcgatggggg cggggggggg ggggcgcgc gccaggcggg gcggggcggg gcgaggggcg 540
gggcggggcg aggcggagag gtgcggcggc agccaatcag agcggcgcgc tccgaaagtt 600
tcctttatg gcgaggcggc ggcggcggcg gccctataaa aagcgaagcg cgcggcgggc 660
gggagtcgct gcgttgcctt cgccccgtgc cccgctccgc gccgcctcgc gccgcccgcc 720
ccggctctga ctgaccgcgt tactcccaca ggtgagcggg cgggacggcc cttctcctcc 780
gggctgtaat tagcgcttgg tttaatgacg gctcgtttct tttctgtggc tgcgtgaaag 840
ccttaaaggg ctccgggagg gccctttgtg cgggggggag cggctcgggg ggtgcgtgcg 900
tgtgtgtgtg cgtggggagc gccgcgtgcg gcccgcgctg cccggcggct gtgagcgctg 960
cgggcgcggc gcggggcttt gtgcgctccg cgtgtgcgcg aggggagcgc ggccgggggc 1020
ggtgccccgc ggtgcggggg ggctgcgagg ggaacaaagg ctgcgtgcgg ggtgtgtgcg 1080
tgggggggtg agcagggggt gtgggcgcgg cggtcgggct gtaacccccc cctgcacccc 1140
cctccccgag ttgctgagca cggcccggct tcgggtgcgg ggctccgtgc ggggcgtggc 1200
gcggggctcg ccgtgccggg cgggggggtgg cggcaggtgg gggtgccggg cggggcgggg 1260
ccgcctcggg ccggggaggg ctcgggggag gggcgcggcg gccccggagc gccggcggct 1320
gtcgaggcgc ggcgagccgc agccattgcc ttttatggta atcgtgcgag agggcgcagg 1380
gacttccttt gtcccaaatc tggcggagcc gaaatctggg aggcgccgcc gcaccccctc 1440
tagcgggcgc gggcgaagcg gtgcggcgcc ggcaggaagg aaatgggcgg ggagggcctt 1500
cgtgcgtcgc cgcgccgccg tccccttctc catctccagc ctcggggctg ccgcaggggg 1560
acggctgcct tcggggggga cggggcaggg cggggttcgg cttctggcgt gtgaccggcg 1620
gctctagagc ctctgctaac catgttcatg ccttcttctt tttcctacag ctcctgggca 1680
acgtgctggt tgttgtgctg tctcatcatt ttggcaaaga attcctcgag gctagcccat 1740
catcatcatc atcattgaaa ttcactcctc aggtgcaggc tgcctatcag aaggtggtgg 1800
ctggtgtggc caatgccctg gctcacaaat accactgaga tctttttccc tctgccaaaa 1860
attatgggga catcatgaag ccccttgagc atctgacttc tggctaataa aggaaattta 1920
ttttcattgc aatagtgtgt tggaattttt tgtgtctctc actcggaagg acatatggga 1980
gggcaaatca tttaaaacat cagaatgagt atttggttta gagtttggca acatatgcca 2040
tatgctggct gccatgaaca aaggtggcta taaagaggtc atcagtatat gaaacagccc 2100
cctgctgtcc attccttatt ccatagaaaa gccttgactt gaggttagat tttttttata 2160
ttttgttttg tgttattttt ttctttaaca tccctaaaat ttccttaca tgttttacta 2220
gccagatttt tcctcctctc ctgactactc ccagtcatag ctgtccctct tctcttatga 2280
agatccctcg acctgcagcc caagcttggc gtaatcatgg tcatagctgt ttcctgtgtg 2340
aaattgttat ccgctcacaa ttccacacaa catacgagcc ggaagcataa agtgtaaagc 2400
ctggggtgcc taatgagtga gctaactcac attaattgcg ttgcgctcac tgcccgcttt 2460
ccagtcggga aacctgtcgt gccagcggat ccgcatctca attagtcagc aaccatagtc 2520
ccgcccctaa ctccgcccat cccgccccta actccgccca gttccgccca ttctccgccc 2580
catggctgac taattttttt tatttatgca gaggccgagg ccgcctcggc ctctgagcta 2640
ttccagaagt agtgaggagg cttttttgga ggcctaggct tttgcaaaaa gctaacttgt 2700
ttattgcagc ttataatggt tacaaataaa gcaatagcat cacaaatttc acaaataaag 2760
cattttttc actgcattct agttgtggtt tgtccaaact catcaatgta tcttatcatg 2820
tctggatccg ctgcattaat gaatcggcca acgcgcgggg agaggcggtt tgcgtattgg 2880
gcgctcttcc gcttcctcgc tcactgactc gctgcgctcg gtcgttcggc tgcggcgagc 2940
ggtatcagct cactcaaagg cggtaatacg gttatccaca gaatcagggg ataacgcagg 3000
aaagaacatg tgagcaaaag gccagcaaaa ggccaggaac cgtaaaaagg ccgcgttgct 3060
ggcgtttttc cataggctcc gcccccctga cgagcatcac aaaaatcgac gctcaagtca 3120
gaggtggcga aacccgacag gactataaag ataccaggcg tttccccctg gaagctccct 3180
cgtgcgctct cctgttccga ccctgccgct taccggatac ctgtccgcct ttctcccttc 3240
gggaagcgtg gcgctttctc aatgctcacg ctgtaggtat ctcagttcgg tgtaggtcgt 3300
tcgctccaag ctgggctgtg tgcacgaacc ccccgttcag cccgaccgct gcgccttatc 3360
cggtaactat cgtcttgagt ccaacccggt aagacacgac ttatcgccac tggcagcagc 3420
cactggtaac aggattagca gagcgaggta tgtaggcggt gctacagagt tcttgaagtg 3480
gtggcctaac tacggctaca ctagaaggac agtatttggt atctgcgctc tgctgaagcc 3540
agttaccttc ggaaaaagag ttggtagctc ttgatccggc aaacaaacca ccgctggtag 3600
cggtggtttt tttgtttgca agcagcagat tacgcgcaga aaaaaaggat ctcaagaaga 3660
tcctttgatc ttttctacgg ggtctgacgc tcagtggaac gaaaactcac gttaagggat 3720
tttggtcatg agattatcaa aaaggatctt cacctagatc ttttaaatt aaaaatgaag 3780

ttttaaatca atctaaagta tatatgagta aacttggtct gacagttacc aatgcttaat 3840
cagtgaggca cctatctcag cgatctgtct atttcgttca tccatagttg cctgactccc 3900
cgtcgtgtag ataactacga tacgggaggg cttaccatct ggccccagtg ctgcaatgat 3960
accgcgagac ccacgctcac cggctccaga tttatcagca ataaaccagc cagccggaag 4020
ggccgagcgc agaagtggtc ctgcaacttt atccgcctcc atccagtcta ttaattgttg 4080
ccgggaagct agagtaagta gttcgccagt taatagtttg cgcaacgttg ttgccattgc 4140
tacaggcatc gtggtgtcac gctcgtcgtt tggtatggct tcattcagct ccggttccca 4200
acgatcaagg cgagttacat gatcccccat gttgtgcaaa aaagcggtta gctccttcgg 4260
tcctccgatc gttgtcagaa gtaagttggc cgcagtgtta tcactcatgg ttatggcagc 4320
actgcataat tctcttactg tcatgccatc cgtaagatgc ttttctgtga ctggtgagta 4380
ctcaaccaag tcattctgag aatagtgtat gcggcgaccg agttgctctt gcccggcgtc 4440
aatacgggat aataccgcgc cacatagcag aactttaaaa gtgctcatca ttggaaaacg 4500
ttcttcgggg cgaaaactct caaggatctt accgctgttg agatccagtt cgatgtaacc 4560
cactcgtgca cccaactgat cttcagcatc ttttactttc accagcgttt ctgggtgagc 4620
aaaaacagga aggcaaaatg ccgcaaaaaa gggaataagg gcgacacgga aatgttgaat 4680
actcatactc ttcctttttc aatattattg aagcatttat cagggttatt gtctcatgag 4740
cggatacata tttgaatgta tttagaaaaa taaacaaata ggggttccgc gcacatttcc 4800
ccgaaaagtg ccacctg 4817

<210> 14
<211> 1140
<212> DNA
<213> Artificial Sequence

<220>
<223> Description of Artificial Sequence:Artificial Sequence

<400> 14
atggctcgga gaagagcctt ccctgctttc gcgctccggc tctggagcat cctaccttgc 60
ctgctcctgc tgcgagcgga tgcagggcag ccacctgagg agagcttgta cctgtggatc 120
gacgcccatc aggctagagt gctcatagga tttgaagaag accttctgat tgtctcggag 180
gggaaaatgg cccccctttac acatgatttc aggaaagccc aacaaagaat gccagccatt 240
cctgtcaata tccactccat gaattttacc tggcaagctg cggggcaggc agaatacttc 300
tacgagttcc tgtctctgcg ctccctggat aaaggcatca tggcagatcc aactgtcaat 360
gtccctttgc tgggaacagt gcctcacaag gcatcagttg ttcaagttgg tttcccgtgt 420
ctcggcaaac aagacggggt agcagcattt gaagtgaatg tgattgtcat gaattctgaa 480
ggcaacacca tccttaggac ccctcagaat gccatcttct ttaaaacatg tcaacaagct 540
gagtgtcccg gagggtgtcg aaatggaggc ttttgtaacg aaaggcgggt ctgcgagtgt 600
ccggatgggt tctacgggcc tcactgtgag aaagccctgt gcataccccg atgtatgaac 660
ggtggtctgt gtgtcactcc tggcttctgc atctgccccc ctggattcta cggtgtcaac 720
tgtgacaaag caaactgctc aaccacctgc tttaatggag ggacctgctt ttacccggga 780
aaatgtattt gccctcctgg actcgaggga gagcagtgtg aactcagcaa atgcccccaa 840
ccctgccgaa atggaggtaa atgcattggt aaaagcaagt gtaagtgccc gaaaggttac 900
caaggagacc tgtgctctaa gcccgtctgc gagcctggct gtggtgccca cggaacctgc 960
cacgaaccca acaagtgcca gtgtcgagag ggctggcacg gcagacactg caataagagg 1020
tatggagcca gcctcatgca tgccccgagg ccagcaggcg ccgggctgga gcgacacacg 1080
ccttcactta aaaaggctga ggatagaagg gatccacctg aatccaatta catctggtga 1140

<210> 15
<211> 379
<212> PRT
<213> Artificial Sequence

<220>
<223> Description of Artificial Sequence:Artificial Sequence

<400> 15
Met Ala Arg Arg Arg Ala Phe Pro Ala Phe Ala Leu Arg Leu Trp Ser
1 5 10 15

Ile Leu Pro Cys Leu Leu Leu Leu Arg Ala Asp Ala Gly Gln Pro Pro
20 25 30

Glu Glu Ser Leu Tyr Leu Trp Ile Asp Ala His Gln Ala Arg Val Leu
35 40 45

Ile Gly Phe Glu Glu Asp Leu Leu Ile Val Ser Glu Gly Lys Met Ala
50 55 60

Pro Phe Thr His Asp Phe Arg Lys Ala Gln Gln Arg Met Pro Ala Ile
65 70 75 80

Pro Val Asn Ile His Ser Met Asn Phe Thr Trp Gln Ala Ala Gly Gln
85 90 95

Ala Glu Tyr Phe Tyr Glu Phe Leu Ser Leu Arg Ser Leu Asp Lys Gly

```
                100                 105                 110

Ile Met Ala Asp Pro Thr Val Asn Val Pro Leu Leu Gly Thr Val Pro
        115                 120                 125

His Lys Ala Ser Val Val Gln Val Gly Phe Pro Cys Leu Gly Lys Gln
    130                 135                 140

Asp Gly Val Ala Ala Phe Glu Val Asn Val Ile Val Met Asn Ser Glu
145                 150                 155                 160

Gly Asn Thr Ile Leu Arg Thr Pro Gln Asn Ala Ile Phe Phe Lys Thr
                165                 170                 175

Cys Gln Gln Ala Glu Cys Pro Gly Gly Cys Arg Asn Gly Gly Phe Cys
            180                 185                 190

Asn Glu Arg Arg Val Cys Glu Cys Pro Asp Gly Phe Tyr Gly Pro His
        195                 200                 205

Cys Glu Lys Ala Leu Cys Ile Pro Arg Cys Met Asn Gly Gly Leu Cys
    210                 215                 220

Val Thr Pro Gly Phe Cys Ile Cys Pro Pro Gly Phe Tyr Gly Val Asn
225                 230                 235                 240

Cys Asp Lys Ala Asn Cys Ser Thr Thr Cys Phe Asn Gly Gly Thr Cys
                245                 250                 255

Phe Tyr Pro Gly Lys Cys Ile Cys Pro Pro Gly Leu Glu Gly Glu Gln
            260                 265                 270

Cys Glu Leu Ser Lys Cys Pro Gln Pro Cys Arg Asn Gly Gly Lys Cys
        275                 280                 285

Ile Gly Lys Ser Lys Cys Lys Cys Pro Lys Gly Tyr Gln Gly Asp Leu
    290                 295                 300

Cys Ser Lys Pro Val Cys Glu Pro Gly Cys Gly Ala His Gly Thr Cys
305                 310                 315                 320

His Glu Pro Asn Lys Cys Gln Cys Arg Glu Gly Trp His Gly Arg His
                325                 330                 335

Cys Asn Lys Arg Tyr Gly Ala Ser Leu Met His Ala Pro Arg Pro Ala
            340                 345                 350

Gly Ala Gly Leu Glu Arg His Thr Pro Ser Leu Lys Lys Ala Glu Asp
        355                 360                 365

Arg Arg Asp Pro Pro Glu Ser Asn Tyr Ile Trp
    370                 375
```

<210> 16
<211> 1140
<212> DNA
<213> Artificial Sequence

<220>
<223> Description of Artificial Sequence:Artificial
Sequence

<400> 16

```
atggctcgga gaagagcctt ccctgctttc gcgctccggc tctggagcat cctaccttgc 60
ctgctcctgc tgcgagcgga tgcagggcag ccacctgagg agagcttgta cctgtggatc 120
gacgcccatc aggctagagt gctcatagga tttgaagaag acattctgat tgtctcggag 180
gggaaaatgg ccccctttac acatgatttc aggaaagccc aacaaagaat gccagccatt 240
cctgtcaata tccactccat gaattttacc tggcaagctg cggggcaggc agaatacttc 300
tacgagttcc tgtctctgcg ctccattgat aaaggcatca tggcagatcc aactgtcaat 360
gtccctttgc tgggaacagt gcctcacaag gcatcagttg ttcaagttgg tttcccgtgt 420
ctcggcaaac aagacggggt agcagcattt gaagtgaatg tgattgtcat gaattctgaa 480
ggcaacacca tccttaggac ccctcagaat gccatcttct ttaaaacatg tcaacaagct 540
gagtgtcccg gagggtgtcg aaatggaggc ttttgtaacg aaaggcgggt ctgcgagtgt 600
ccggatgggt tctacgggcc tcactgtgag aaagccctgt gcataccccg atgtatgaac 660
ggtggtctgt gtgtcactcc tggcttctgc atctgccccc ctggattcta cggtgtcaac 720
tgtgacaaag caaactgctc aaccacctgc tttaatggag ggacctgctt ttacccggga 780
aaatgtattt gccctcctgg actcgaggga gagcagtgtg aactcagcaa atgcccccaa 840
ccctgccgaa atggaggtaa atgcattggt aaaagcaagt gtaagtgccc gaaaggttac 900
caaggagacc tgtgctctaa gcccgtctgc gagcctggct gtggtgccca cggaacctgc 960
```

cacgaaccca acaagtgcca gtgtcgagag ggctggcacg gcagacactg caataagagg 1020
tatggagcca gcctcatgca tgccccgagg ccagcaggcg ccgggctgga gcgacacacg 1080
ccttcactta aaaaggctga ggatagaagg gatccacctg aatccaatta catctggtga 1140

<210> 17
<211> 379
<212> PRT
<213> Artificial Sequence

<220>
<223> Description of Artificial Sequence:Artificial Sequence

<400> 17
Met Ala Arg Arg Arg Ala Phe Pro Ala Phe Ala Leu Arg Leu Trp Ser
1 5 10 15

Ile Leu Pro Cys Leu Leu Leu Leu Arg Ala Asp Ala Gly Gln Pro Pro
20 25 30

Glu Glu Ser Leu Tyr Leu Trp Ile Asp Ala His Gln Ala Arg Val Leu
35 40 45

Ile Gly Phe Glu Glu Asp Ile Leu Ile Val Ser Glu Gly Lys Met Ala
50 55 60

Pro Phe Thr His Asp Phe Arg Lys Ala Gln Gln Arg Met Pro Ala Ile
65 70 75 80

Pro Val Asn Ile His Ser Met Asn Phe Thr Trp Gln Ala Ala Gly Gln
85 90 95

Ala Glu Tyr Phe Tyr Glu Phe Leu Ser Leu Arg Ser Ile Asp Lys Gly
100 105 110

Ile Met Ala Asp Pro Thr Val Asn Val Pro Leu Leu Gly Thr Val Pro
115 120 125

His Lys Ala Ser Val Val Gln Val Gly Phe Pro Cys Leu Gly Lys Gln
130 135 140

Asp Gly Val Ala Ala Phe Glu Val Asn Val Ile Val Met Asn Ser Glu
145 150 155 160

Gly Asn Thr Ile Leu Arg Thr Pro Gln Asn Ala Ile Phe Phe Lys Thr
165 170 175

Cys Gln Gln Ala Glu Cys Pro Gly Gly Cys Arg Asn Gly Gly Phe Cys
180 185 190

Asn Glu Arg Arg Val Cys Glu Cys Pro Asp Gly Phe Tyr Gly Pro His
195 200 205

Cys Glu Lys Ala Leu Cys Ile Pro Arg Cys Met Asn Gly Gly Leu Cys
210 215 220

Val Thr Pro Gly Phe Cys Ile Cys Pro Pro Gly Phe Tyr Gly Val Asn
225 230 235 240

Cys Asp Lys Ala Asn Cys Ser Thr Thr Cys Phe Asn Gly Gly Thr Cys
245 250 255

Phe Tyr Pro Gly Lys Cys Ile Cys Pro Pro Gly Leu Glu Gly Glu Gln
260 265 270

Cys Glu Leu Ser Lys Cys Pro Gln Pro Cys Arg Asn Gly Gly Lys Cys
275 280 285

Ile Gly Lys Ser Lys Cys Lys Cys Pro Lys Gly Tyr Gln Gly Asp Leu
290 295 300

Cys Ser Lys Pro Val Cys Glu Pro Gly Cys Gly Ala His Gly Thr Cys
305 310 315 320

His Glu Pro Asn Lys Cys Gln Cys Arg Glu Gly Trp His Gly Arg His
325 330 335

Cys Asn Lys Arg Tyr Gly Ala Ser Leu Met His Ala Pro Arg Pro Ala
340 345 350

Gly Ala Gly Leu Glu Arg His Thr Pro Ser Leu Lys Lys Ala Glu Asp
355 360 365

Arg Arg Asp Pro Pro Glu Ser Asn Tyr Ile Trp
370 375

<210> 18
<211> 1140
<212> DNA
<213> Artificial Sequence

<220>
<223> Description of Artificial Sequence:Artificial Sequence

<400> 18
atggctcgga gaagagcctt ccctgctttc gcgctccggc tctggagcat cctaccttgc 60
ctgctcctgc tgcgagcgga tgcagggcag ccacctgagg agagcttgta cctgtggatc 120
gacgcccatc aggctagagt gctcatagga tttgaagaag acattctgat tgtctcggag 180
gggaaaatgg ccccctttac acatgatttc aggaaagccc aacaaagaat gccagccatt 240
cctgtcaata tccactccat gaattttacc tggcaagctg cggggcaggc agaatacttc 300
tacgagttcc tgtctctgcg ctccctggat aaaggcatca tggcagatcc aactgtcaat 360
gtccctttgc tgggaacagt gcctcacaag gcatcagttg ttcaagttgg tttcccgtgt 420
ctcggcaaac aagacggggt agcagcattt gaagtgaatg tgattgtcat gaattctgaa 480
ggcaacacca tccttaggac ccctcagaat gccatcttct ttaaaacatg tcaacaagct 540
gagtgtcccg gagggtgtcg aaatggaggc ttttgtaacg aaaggcgggt ctgcgagtgt 600
ccggatgggt tctacgggcc tcactgtgag aaagccctgt gcataccccg atgtatgaac 660
ggtggtctgt gtgtcactcc tggcttctgc atctgccccc ctggattcta cggtgtcaac 720
tgtgacaaag taaactgctc aaccacctgc tttaatggag ggacctgctt ttacccggga 780
aaatgtattt gccctcctgg actcgaggga gagcagtgtg aactcagcaa atgcccccaa 840
ccctgccgaa atggaggtaa atgcattggt aaaagcaagt gtaagtgccc gaaaggttac 900
caaggagacc tgtgctctaa gcccgtctgc gagcctggct gtggtgccca cggaacctgc 960
cacgaaccca acaagtgcca gtgtcgagag ggctggcacg gcagacactg caataagagg 1020
tatggagcca gcctcatgca tgccccgagg ccagcaggcg ccgggctgga gcgacacacg 1080
ccttcactta aaaaggctga ggatagaagg gatccacctg aatccaatta catctggtga 1140

<210> 19
<211> 379
<212> PRT
<213> Artificial Sequence

<220>
<223> Description of Artificial Sequence:Artificial Sequence

<400> 19
Met Ala Arg Arg Arg Ala Phe Pro Ala Phe Ala Leu Arg Leu Trp Ser
1 5 10 15

Ile Leu Pro Cys Leu Leu Leu Leu Arg Ala Asp Ala Gly Gln Pro Pro
20 25 30

Glu Glu Ser Leu Tyr Leu Trp Ile Asp Ala His Gln Ala Arg Val Leu
35 40 45

Ile Gly Phe Glu Glu Asp Ile Leu Ile Val Ser Glu Gly Lys Met Ala
50 55 60

Pro Phe Thr His Asp Phe Arg Lys Ala Gln Gln Arg Met Pro Ala Ile
65 70 75 80

Pro Val Asn Ile His Ser Met Asn Phe Thr Trp Gln Ala Ala Gly Gln
85 90 95

Ala Glu Tyr Phe Tyr Glu Phe Leu Ser Leu Arg Ser Leu Asp Lys Gly
100 105 110

Ile Met Ala Asp Pro Thr Val Asn Val Pro Leu Leu Gly Thr Val Pro
115 120 125

His Lys Ala Ser Val Val Gln Val Gly Phe Pro Cys Leu Gly Lys Gln
130 135 140

Asp Gly Val Ala Ala Phe Glu Val Asn Val Ile Val Met Asn Ser Glu
145 150 155 160

Gly Asn Thr Ile Leu Arg Thr Pro Gln Asn Ala Ile Phe Phe Lys Thr

165 170 175

Cys Gln Gln Ala Glu Cys Pro Gly Gly Cys Arg Asn Gly Gly Phe Cys
180 185 190

Asn Glu Arg Arg Val Cys Glu Cys Pro Asp Gly Phe Tyr Gly Pro His
195 200 205

Cys Glu Lys Ala Leu Cys Ile Pro Arg Cys Met Asn Gly Gly Leu Cys
210 215 220

Val Thr Pro Gly Phe Cys Ile Cys Pro Pro Gly Phe Tyr Gly Val Asn
225 230 235 240

Cys Asp Lys Val Asn Cys Ser Thr Thr Cys Phe Asn Gly Gly Thr Cys
245 250 255

Phe Tyr Pro Gly Lys Cys Ile Cys Pro Pro Gly Leu Glu Gly Glu Gln
260 265 270

Cys Glu Leu Ser Lys Cys Pro Gln Pro Cys Arg Asn Gly Gly Lys Cys
275 280 285

Ile Gly Lys Ser Lys Cys Lys Cys Pro Lys Gly Tyr Gln Gly Asp Leu
290 295 300

Cys Ser Lys Pro Val Cys Glu Pro Gly Cys Gly Ala His Gly Thr Cys
305 310 315 320

His Glu Pro Asn Lys Cys Gln Cys Arg Glu Gly Trp His Gly Arg His
325 330 335

Cys Asn Lys Arg Tyr Gly Ala Ser Leu Met His Ala Pro Arg Pro Ala
340 345 350

Gly Ala Gly Leu Glu Arg His Thr Pro Ser Leu Lys Lys Ala Glu Asp
355 360 365

Arg Arg Asp Pro Pro Glu Ser Asn Tyr Ile Trp
370 375

<210> 20
<211> 1140
<212> DNA
<213> Artificial Sequence

<220>
<223> Description of Artificial Sequence:Artificial Sequence

<400> 20
atggctcgga gaagagcctt ccctgctttc gcgctccggc tctggagcat cctaccttgc 60
ctgctcctgc tgcgagcgga tgcagggcag ccacctgagg agagcttgta cctgtggatc 120
gacgcccatc aggctagagt gctcatagga tttgaagaag acattctgat tgtctcggag 180
gggaaaatgg cccccttac acatgatttc aggaaagccc aacaaagaat gccagccatt 240
cctgtcaata tccactccat gaatttacc tggcaagctg cggggcaggc agaatacttc 300
tacgagttcc tgtctctgcg ctccctggat aaaggcatca tggcagatcc aactgtcaat 360
gtccctttgc tgggaacagt gcctcacaag gcatcagttg ttcaagttgg tttcccgtgt 420
ctcggcaaac aagacggggt agcagcattt gaagtgaatg tgattgtcat gaattctgaa 480
ggcaacacca tccttaggac ccctcagaat gccatcttct ttaaaacatg tcaacaagct 540
gagtgtcccg gagggtgtcg aaatggaggc ttttgtaacg aaaggcggt ctgcgagtgt 600
ccggatgggt tctacgggcc tcactgtgag aaagccctgt gcataccccg atgtatgaac 660
ggtggtctgt gtgtcactcc tggcttctgc atctgccccc ctggattcta cggtgtcaac 720
tgtgacaaag caaactgctc aaccacctgc tttaatggag ggacctgctt ttacccggga 780
aaatgtattt gccctcctgg actcgaggga gatcagtgtg aactcagcaa atgcccccaa 840
ccctgccgaa atggaggtaa atgcattggt aaaagcaagt gtaagtgccc gaaaggttac 900
caaggagacc tgtgctctaa gcccgtctgc gagcctggct gtggtgccca cggaacctgc 960
cacgaaccca acaagtgcca gtgtcgagag ggctggcacg gcagacactg caataagagg 1020
tatggagcca gcctcatgca tgccccgagg ccagcaggcg ccgggctgga gcgacacacg 1080
ccttcactta aaaaggctga ggatagaagg gatccacctg aatccaatta catctggtga 1140

<210> 21
<211> 379
<212> PRT
<213> Artificial Sequence

<220>
<223> Description of Artificial Sequence:Artificial

Sequence

<400> 21
Met Ala Arg Arg Arg Ala Phe Pro Ala Phe Ala Leu Arg Leu Trp Ser
1               5                   10                  15

Ile Leu Pro Cys Leu Leu Leu Leu Arg Ala Asp Ala Gly Gln Pro Pro
            20                  25                  30

Glu Glu Ser Leu Tyr Leu Trp Ile Asp Ala His Gln Ala Arg Val Leu
        35                  40                  45

Ile Gly Phe Glu Glu Asp Ile Leu Ile Val Ser Glu Gly Lys Met Ala
    50                  55                  60

Pro Phe Thr His Asp Phe Arg Lys Ala Gln Gln Arg Met Pro Ala Ile
65                  70                  75                  80

Pro Val Asn Ile His Ser Met Asn Phe Thr Trp Gln Ala Ala Gly Gln
                85                  90                  95

Ala Glu Tyr Phe Tyr Glu Phe Leu Ser Leu Arg Ser Leu Asp Lys Gly
            100                 105                 110

Ile Met Ala Asp Pro Thr Val Asn Val Pro Leu Leu Gly Thr Val Pro
        115                 120                 125

His Lys Ala Ser Val Val Gln Val Gly Phe Pro Cys Leu Gly Lys Gln
    130                 135                 140

Asp Gly Val Ala Ala Phe Glu Val Asn Val Ile Val Met Asn Ser Glu
145                 150                 155                 160

Gly Asn Thr Ile Leu Arg Thr Pro Gln Asn Ala Ile Phe Phe Lys Thr
                165                 170                 175

Cys Gln Gln Ala Glu Cys Pro Gly Gly Cys Arg Asn Gly Gly Phe Cys
            180                 185                 190

Asn Glu Arg Arg Val Cys Glu Cys Pro Asp Gly Phe Tyr Gly Pro His
        195                 200                 205

Cys Glu Lys Ala Leu Cys Ile Pro Arg Cys Met Asn Gly Gly Leu Cys
    210                 215                 220

Val Thr Pro Gly Phe Cys Ile Cys Pro Pro Gly Phe Tyr Gly Val Asn
225                 230                 235                 240

Cys Asp Lys Ala Asn Cys Ser Thr Thr Cys Phe Asn Gly Gly Thr Cys
                245                 250                 255

Phe Tyr Pro Gly Lys Cys Ile Cys Pro Pro Gly Leu Glu Gly Asp Gln
            260                 265                 270

Cys Glu Leu Ser Lys Cys Pro Gln Pro Cys Arg Asn Gly Gly Lys Cys
        275                 280                 285

Ile Gly Lys Ser Lys Cys Lys Cys Pro Lys Gly Tyr Gln Gly Asp Leu
    290                 295                 300

Cys Ser Lys Pro Val Cys Glu Pro Gly Cys Gly Ala His Gly Thr Cys
305                 310                 315                 320

His Glu Pro Asn Lys Cys Gln Cys Arg Glu Gly Trp His Gly Arg His
                325                 330                 335

Cys Asn Lys Arg Tyr Gly Ala Ser Leu Met His Ala Pro Arg Pro Ala
            340                 345                 350

Gly Ala Gly Leu Glu Arg His Thr Pro Ser Leu Lys Lys Ala Glu Asp
        355                 360                 365

Arg Arg Asp Pro Pro Glu Ser Asn Tyr Ile Trp
    370                 375

<210> 22
<211> 558
<212> DNA
<213> Artificial Sequence

<220>
<223> Description of Artificial Sequence:Artificial
Sequence

<400> 22
atggctcgga gaagagcctt ccctgctttc gcgctccggc tctggagcat cctaccttgc 60
ctgctcctgc tgcgagcgga tgcagggcag ccacctgagg agagcttgta cctgtggatc 120
gacgcccatc aggctagagt gctcatagga tttgaagaag acattctgat tgtctcggag 180
gggaaaatgg cccccttac acatgatttc aggaaagccc aacaaagaat gccagccatt 240
cctgtcaata tccactccat gaattttacc tggcaagctg cggggcaggc agaatacttc 300
tacgagttcc tgtctctgcg ctccctggat aaaggcatca tggcagatcc aactgtcaat 360
gtccctttgc tgggaacagt gcctcacaag gcatcagttg ttcaagttgg tttcccgtgt 420
ctcggcaaac aagacggggt agcagcattt gaagtgaatg tgattgtcat gaattctgaa 480
ggcaacacca tccttaggac ccctcagaat gccatcttct ttaaaacaca gctagcccat 540
catcatcatc atcattga 558

<210> 23
<211> 185
<212> PRT
<213> Artificial Sequence

<220>
<223> Description of Artificial Sequence:Artificial
Sequence

<400> 23
Met Ala Arg Arg Arg Ala Phe Pro Ala Phe Ala Leu Arg Leu Trp Ser
1 5 10 15

Ile Leu Pro Cys Leu Leu Leu Leu Arg Ala Asp Ala Gly Gln Pro Pro
20 25 30

Glu Glu Ser Leu Tyr Leu Trp Ile Asp Ala His Gln Ala Arg Val Leu
35 40 45

Ile Gly Phe Glu Glu Asp Ile Leu Ile Val Ser Glu Gly Lys Met Ala
50 55 60

Pro Phe Thr His Asp Phe Arg Lys Ala Gln Gln Arg Met Pro Ala Ile
65 70 75 80

Pro Val Asn Ile His Ser Met Asn Phe Thr Trp Gln Ala Ala Gly Gln
85 90 95

Ala Glu Tyr Phe Tyr Glu Phe Leu Ser Leu Arg Ser Leu Asp Lys Gly
100 105 110

Ile Met Ala Asp Pro Thr Val Asn Val Pro Leu Leu Gly Thr Val Pro
115 120 125

His Lys Ala Ser Val Val Gln Val Gly Phe Pro Cys Leu Gly Lys Gln
130 135 140

Asp Gly Val Ala Ala Phe Glu Val Asn Val Ile Val Met Asn Ser Glu
145 150 155 160

Gly Asn Thr Ile Leu Arg Thr Pro Gln Asn Ala Ile Phe Phe Lys Thr
165 170 175

Gln Leu Ala His His His His His His
180 185

<210> 24
<211> 717
<212> DNA
<213> Artificial Sequence

<220>
<223> Description of Artificial Sequence:Artificial
Sequence

<400> 24
atggctcgga gaagagcctt ccctgctttc gcgctccggc tctggagcat cctaccttgc 60
ctgctcctgc tgctcgactg tcaacaagct gagtgtcccg gagggtgtcg aaatggaggc 120
ttttgtaacg aaaggcgggt ctgcgagtgt ccggatgggt tctacgggcc tcactgtgag 180
aaagccctgt gcataccccg atgtatgaac ggtggtctgt gtgtcactcc tggcttctgc 240
atctgccccc ctggattcta cggtgtcaac tgtgacaaag caaactgctc aaccacctgc 300

```
tttaatggag ggacctgctt ttacccggga aaatgtattt gccctcctgg actcgaggga 360
gagcagtgtg aactcagcaa atgcccccaa ccctgccgaa atggaggtaa atgcattggt 420
aaaagcaagt gtaagtgccc gaaaggttac caaggagacc tgtgctctaa gcccgtctgc 480
gagcctggct gtggtgccca cggaacctgc cacgaaccca acaagtgcca gtgtcgagag 540
ggctggcacg gcagacactg caataagagg tatggagcca gcctcatgca tgccccgagg 600
ccagcaggcg ccgggctgga gcgacacacg ccttcactta aaaaggctga ggatagaagg 660
gatccacctg aatccaatta catctggcag ctagcccatc atcatcatca tcattga    717
```

<210> 25
<211> 238
<212> PRT
<213> Artificial Sequence

<220>
<223> Description of Artificial Sequence:Artificial Sequence

<400> 25

```
Met Ala Arg Arg Arg Ala Phe Pro Ala Phe Ala Leu Arg Leu Trp Ser
  1               5                  10                  15

Ile Leu Pro Cys Leu Leu Leu Leu Leu Asp Cys Gln Gln Ala Glu Cys
             20                  25                  30

Pro Gly Gly Cys Arg Asn Gly Gly Phe Cys Asn Glu Arg Arg Val Cys
         35                  40                  45

Glu Cys Pro Asp Gly Phe Tyr Gly Pro His Cys Glu Lys Ala Leu Cys
     50                  55                  60

Ile Pro Arg Cys Met Asn Gly Gly Leu Cys Val Thr Pro Gly Phe Cys
 65                  70                  75                  80

Ile Cys Pro Pro Gly Phe Tyr Gly Val Asn Cys Asp Lys Ala Asn Cys
                 85                  90                  95

Ser Thr Thr Cys Phe Asn Gly Gly Thr Cys Phe Tyr Pro Gly Lys Cys
            100                 105                 110

Ile Cys Pro Pro Gly Leu Glu Gly Glu Gln Cys Glu Leu Ser Lys Cys
        115                 120                 125

Pro Gln Pro Cys Arg Asn Gly Gly Lys Cys Ile Gly Lys Ser Lys Cys
    130                 135                 140

Lys Cys Pro Lys Gly Tyr Gln Gly Asp Leu Cys Ser Lys Pro Val Cys
145                 150                 155                 160

Glu Pro Gly Cys Gly Ala His Gly Thr Cys His Glu Pro Asn Lys Cys
                165                 170                 175

Gln Cys Arg Glu Gly Trp His Gly Arg His Cys Asn Lys Arg Tyr Gly
            180                 185                 190

Ala Ser Leu Met His Ala Pro Arg Pro Ala Gly Ala Gly Leu Glu Arg
        195                 200                 205

His Thr Pro Ser Leu Lys Lys Ala Glu Asp Arg Arg Asp Pro Pro Glu
    210                 215                 220

Ser Asn Tyr Ile Trp Gln Leu Ala His His His His His His
225                 230                 235
```

36

/36

## Description

TECHNICAL FIELD

The present invention is related to an agent for controlling life or death of a stem cell. More specifically, it relates to a protein suppressing the process for death of a hematopoietic stem cell, and controlling the mechanism for promoting the process for life.

BACKGROUND ART

Treatment of diseases by regenerative medicine has recently been receiving interest. However, such regenerative medicine is not completed so that it is routinely used for a number of patients having organ or tissue dysfunction. To date, in order to treat such patients, organ transplantation as well as assistance systems with medical devices are used for only a few patients with such disorders. However, these treatments have problems such as donor shortage, immunological rejection, infection, lifetime or the like. Particularly, donor shortage is a serious problem, and in the case of bone marrow transplantation, it is difficult to provide a limited amount of samples to a number of patients, even if bone marrow banks or umbilical cord blood banks have been developed. Accordingly, demands have been increasing for regenerative medicine such as stem cell therapy and the applications thereof in order to overcome these problems.

As used herein, "regenerative" refers to the phenomenon where an injured tissue or organ is recovered to its original state, and can interchangeably be referred to as pathological regeneration. The body of a living organism may lose a part of an organ or receive a heavy injury by trauma or diseases during the life thereof. In such a case, whether or not such an injured organ can be regenerated depends on the type of such an organ (or animal species). Regenerative medicine is a medicine which attempts to regenerate an organ (or tissue) not capable of naturally regenerating by itself, and to recover the function thereof. Whether or not a tissue is regenerated can be determined by monitoring the improvement of the function thereof. A mammal has some power to regenerate a tissue or organ to some extent (e.g. regeneration of the skin, liver and blood). However, organs such as the heart, lung, brain and the like has little regenerative power, and it has been considered that once such organs have been injured, they cannot regenerate by themselves. Accordingly, to date, there is no effective treatment except for organ transplantation when an organ receives such an injury.

It has been speculated that organs with high regenerative power contain stem cells. This idea has been demonstrated to be true by experimental bone marrow transplantation using animal models. Further, subsequent studies have shown that such stem cells in the bone marrow are the source for all blood cell regeneration. Stem cells have been shown to be present in organs with high regenerative power such as bone marrow, skin and the like. Further, it has also been shown that stem cells exist even in the brain which has long been considered to be non-regenerative. That is, all the organs in the body have stem cells, and it has been shown that such stem cells play a role in controlling regeneration of each organ. Further, stem cells present in each tissue has more plasticity than expected, and it is indicated that stem cells in one organ can be used for regeneration of the other organ.

There is the possibility that stem cells can be used for regeneration of an organ which to date cannot be regenerated. Therefore regenerative medicine such as stem cell therapy is increasingly more interesting. Recently, the need for the study of regeneration in the medical fields has increased therefore findings on stem cells or organogenesis are increasing. For example, establishment of embryonic stem (ES) cells having totipotency and the production of a cloned individual from an adult somatic cell are gaining more and more interest. This is because technology regarding development and regeneration can be used for stem cell therapy. Regenerative medicine using stem cells are in the process of clinical application in each field. There are some fields which have already practiced such regenerative medicine.

The origin of regenerative medicine is the presence of reconstructive biology. Reconstructive biology is originated when Wilson demonstrated that separated sea sponge cells processed by pipetting and remixing formed an individual in 1907 (Wilson, H. V., J. Exp. Zool. 5: 245-248, 1907). With respect to tissue reconstruction in higher biological organisms, amphibian embryo reconstitution was documented by Townes and Holtfreter in 1955. They found that amphibian embryo treated with dilute alkali solution and then by mixing separated cells a reconstituted organism was formed. Thereafter, a number of trials have been challenged for such reconstitution, however, no significant development has been noted until 1980's.

Regenerative medicine was first generally acknowledged when it was applied to burn injuries. In 1981, Bell produced artificial skin and applied it to the therapy of burn injuries (Bell, E. et al. Science, 211: 1052-54, 1981). Bell, E. et al., embedded skin fibroblast cells within a collagen gel, and cultured epidermal cells in the surface thereof to produce an artificial skin. Here, the idea to reconstruct an organ in a three-dimensional manner using cells and extracellular matrices as material was noted.

In regenerative medicine, it is most important to reconstruct organs. There are roughly two methods for reconstructing organs: organs are reconstructed *ex vivo;* and organs are reconstructed *in vivo.* In either case, stem cells are required for reconstruction of organs. Pluripotency and self-replication ability are importantly required for stem cells used in the above-described application.

Stem cells are roughly divided into two categories: embryonic stem cells and somatic (tissue) stem cells. Among somatic stem cells, hematopoietic stem cells have attracted attention since a long time ago. The self-replication ability and pluripotency of hematopoietic stem cells are required for maintaining mature blood cells having a short life time during the life-span of a human. This has already been proposed in 1961 (Till, J.E., et al., Radiat. Res. 14: 213-222). In 1972, a bone marrow transplantation method using a mouse model was established (Micklem, H.S., et al.: J. Cell Physiol., 79: 293-298, 1972), thereby making it possible to detect hematopoietic stem cells by investigating reconstruction of the hematopoietic stem cell system. Since then, hematopoietic stem cell research has made dramatic progress. Subsequent studies revealed the presence of hematopoietic stem cells having self-replication ability and pluripotency (Dick, J.E., et al.; Cell 42; 71-79, 1985). However, hematopoietic stem cells are present at a low rate of several in about 100,000 bone marrow cells even in the case of mice. Therefore, in actual research or clinical application, hematopoietic stem cells need to be concentrated or purified.

Conventionally, hematopoietic stem cell transplantation therapies have been carried out using naturally-occurring cells, leading to various side effects. For example, a side effect (RRT) is produced by pretreatment before transplantation using a large dose of an anticancer drug or radiation. There are also other side effects as follows: bacterial or fungal infectious diseases and hemorrhage due to suppression of bone marrow; in the case of allotransplantation, when donor's leucocytes survive and the number of the cells is increased, they recognize recipient organs as foreign matter and attack them (graft versus host diseases (GVHD)); various pulmonary complications, mainly including cytomegalovirus (CMV) pneumonia; various visceral disorders due to disorders of vascular endothelial cells (cells lining the inner wall of blood vessels); various infectious diseases during immune suppression prolonged after survival of transplanted cells (at least 1-2 years); prolonged chronic GVHD exhibiting various symptoms; late-onset disorders (e.g., secondary cancers, gonad dysfunction, infertility, etc.); and the like.

Due to the above-described complications, transplantation often temporarily worsens systemic conditions. The death rate of patients due to complications is about 10 to 20% in autotransplantation and about 20 to 40% in allotransplantation. Even if patients overcome complications, some patients may relapse. Thus, the current transplantation therapies are inadequate.

To prevent early death due to complications after bone marrow transplantation, a therapeutic method has been developed in which stem cells are separated and purified and precursor cells are produced in large quantities from the stem cells, and the precursor cells as well as stem cells are transplanted. The method has already entered clinical trials.

On the other hand, gene therapy, in which a gene is introduced into stem cells which are in turn transplanted into a patient, has been tried. For example, it has been reported that a stem cell (CD34 positive bone marrow cell) having introduced IL-2 receptor y chain was transplanted into patients with X-linked severe combined immunodeficiency, and as a result, the clinical condition of the patients was improved (Cavazzana-Calvo, M. et al., Science, 288: 669-672, 2000). When introducing a gene into a stem cell, it is necessary to culture such a stem cell for a short period of time, and it is crucial to the success of the gene therapy whether or not such a stem cell is controlled not to survive without differentiation during such a culture period.

Fluorescence activated cell sorting (FACS) was developed in the 1980's. Since then, techniques utilizing FACS have been employed for enrichment and purification of hematopoietic stem cells. It has been revealed that high-purity hematopoietic stem cells are obtained by separating CD34-KSL cells from multiply stained bone marrow cells (Osawa, M. et al., Science, 273: 242-245, 1996). As described above, pluripotency and self-replication ability are the essential features of stem cells. To exploit these abilities, it is important to enrich and purify stem cells and expand the cells by *ex vivo* culture.

Various proteins have an important role in regulating the expansion and differentiation of stem cells. For example, stem cell factor (SCF) (also called steel factor) in hematopoietic stem cells has attracted attention.

SCF is produced by bone marrow stromal cells and acts on pluripotent stem cells, bone marrow cells such as CFU-M, CFU-Meg or the like, and lymphocyte precursory cells to support their expansion and differentiation. That is, it is believed that SCF acts on cells from hematopoietic stem cells to precursory cells so as to aid other cytokines which induce differentiation toward the final stage (S. Kitamura, Saitokain-no-Saizensen [Frontline of Cytokine], Yodosha, edited by T. Hirano, pp. 174-187, 2000).

However, the action of a sole SCF seems to be so weak that it cannot work well unless it operates in cooperation with other factors. For example, SCF induces the differentiation and expansion of hematopoietic stem cells strongly in the presence of other cytokines, such as interleukin IL-3, IL-6, IL-11, granulocyte colony stimulating factor (G-CSF), or the like. SCF also induces the differentiation and expansion of mast cells, erythroblast precursory cells, granulocyte macrophage precursory cells, megakaryocyte precursory cells, and the like.

Therefore, it is considered that SCF does not directly control expansion and differentiation, but enhances the responsiveness of a number of kinds of hematopoietic cells to various cytokines while supporting the survival of the cells.

Thrombopoietin (TPO) has also attracted attention. This factor supports the differentiation of megakaryocytes and the production of platelets as well as acting on stem cells to induce their expansion and differentiation. Also, it has been found that TPO is involved in the self replication of stem cells.

Thus, conventional factors can promote the differentiation of stem cells in an uncontrollable manner, but not in a controllable manner.

Therefore, it is an object of the present invention to provide a method and substance to maintain the pluripotency of a stem cell maintaining an undifferentiated state (without differentiation).

SUMMARY OF INVENTION

Accordingly, the present invention provides a protein having a Wnt-inhibitory factor (WIF) domain. More specifically, the protein having the WIF domain is WIF-1 (for example, SEQ ID NOS: 2, 4, 6, 8, 10 or the like). A protein having a WIF domain such as WIF-1 can provide to stem cells the ability to optionally reserve pluripotency without differentiation when provided to the stem cell in combination with an agent for supporting stem cell survival such as a SCF. Previously, proteins having a WIF domain have been shown to inhibit the function of WNT protein. However, it is not known that such a protein acts on a stem cell to maintain the undifferentiated state and pluripotency and to stop differentiation. Therefore, this fact can be recognized to be a significant effect.

Therefore the present invention provides the following:

1. A polypeptide having a WIF domain which maintains pluripotency without differentiating a stem cell.
2. The polypeptide according to item 1, wherein the WIF domain comprises at least five amino acids among about position 30 to about position 180 of the sequence set forth in SEQ ID NO: 4.
3. The polypeptide according to item 1, wherein the WIF domain comprises the sequence of about position 30 to about position 180 of the sequence set forth in SEQ ID NO: 4.
4. The polypeptide according to item 1, wherein the polypeptide further includes an Epidermal Growth Factor (EGF) like repeat.
5. The polypeptide according to item 4, wherein the EGF like repeat comprises at least one repeat consisting of $CX_3CX_5CX_5CXCX_8CX_4$ where C is cysteine and X is any amino acid.
6. The polypeptide according to item 1, wherein the polypeptide has the sequence set forth in SEQ ID NO: 4.
7. The polypeptide according to item 1, wherein the stem cell is a hematopoietic stem cell.
8. A composition for maintaining pluripotency without differentiating a stem cell comprising a polypeptide having a WIF domain.
9. The composition according to item 8, wherein the polypeptide further has an EGF like repeat.
10. The composition according to item 8 wherein the polypeptide has the sequence set forth in SEQ ID NO: 4.
11. The composition according to item 8 further comprising a stem cell survival agent.
12. The composition according to item 8, wherein the stem cell survival agent is stem cell factor (SCF).
13. A stem cell which does not differentiate in vitro and maintains pluripotency.
14. The stem cell according to item 13 which is a hematopoietic stem cell.
15. The stem cell according to item 13 wherein the period of said pluripotency maintenance is at least six days.
16. The stem cell according to item 13, wherein the pluripotency comprises a capability of differentiating into blood cells.
17. A long period pluripotency maintaining cell composition comprising a stem cell and a polypeptide having a WIF domain.
18. The long period pluripotency maintaining cell composition according to item 17, wherein the polypeptide further comprises an EGF like repeat.
19. The long period pluripotency maintaining cell composition according to item 17, wherein the stem cell is hematopoietic cell.
20. The long period pluripotency maintaining cell composition according to item 17, wherein at least $10^2$ cells of the stem cell exist therein.
21. The long period pluripotency maintaining cell composition according to item 17, wherein the polypeptide having the WIF domain comprises the sequence set forth in SEQ ID NO: 4.
22. The long period pluripotency maintaining cell composition according to item 17, wherein the polypeptide having the WIF domain is present at least at 0.1 ng/ml therein.
23. The long period pluripotency maintaining cell composition according to item 17 further comprising a stem cell survival agent.
24. The long period pluripotency maintaining cell composition according to item 23, wherein the stem cell survival agent is SCF.

25. The long period pluripotency maintaining cell composition according to item 23, wherein the stem cell survival agent is FLT-3 ligand.

26. The long period pluripotency maintaining cell composition according to item 23, wherein the stem cell survival agent is present at least at 1 ng/ml therein.

27. The long period pluripotency maintaining cell composition according to item 23 for preparing differentiated cells wherein the differentiated cells are used for treating disorders of blood cells.

28. A method for maintaining pluripotency without differentiating a stem cell, comprising the step of:

1) providing the stem cell with a polypeptide having a WIF domain.

29. The method according to item 28 wherein the polypeptide further comprises an EGF like domain.

30. The method according to item 28 wherein the stem cell is a hematopoietic cell.

31. The method according to item 28 wherein the stem cell is present at least at $10^2$ cells.

32. The method according to item 28 wherein the polypeptide having the WIF domain comprises the sequence set forth in SEQ ID NO: 4.

33. The method according to item 27 wherein the polypeptide having the WIF domain is present at least at 0.1 ng/ml.

34. The method according to item 27 further comprising the step of:

2) providing a stem cell survival agent with the stem cell.

35. The method according to item 34, wherein the stem cell survival agent is SCF.

36. The method according to item 34, wherein the stem cell survival agent is FLT-3 ligand.

37. The method according to item 34 wherein the stem cell survival agent is present at least at 1 ng/ml.

38. A method for producing a long period pluripotency maintaining cell composition comprising the steps of:

1) providing a stem cell;
2) treating the stem cell with a polypeptide having a WIF domain; and
3) collecting the stem cell treated.

39. The method according to item 38 wherein the polypeptide having the WIF domain further comprises an EGF like repeat.

40. The method according to item 38 wherein the stem cell is a hematopoietic stem cell.

41. The method according to item 38 wherein the stem cell is present at least at $10^2$ cells.

42. The method according to item 38, wherein the polypeptide having the WIF domain comprises the sequence set forth in SEQ ID NO: 4.

43. The method according to item 38, wherein the polypeptide having the WIF domain is present at least at 0.1 ng/ml.

44. The method according to item 38 further comprising the step of

2) providing the stem cell with a stem cell survival agent.

45. The method according to item 44 wherein the stem cell survival agent is SCF.

46. The method according to item 44 wherein the stem cell survival agent is flt-3 ligand.

47. The method according to item 44, wherein the stem cell survival agent is present at least at 1 ng/ml.

48. A method for treating a disease or disorder originating from a disorder of a differentiated cell, comprising the steps of:

1) administering a long period pluripotency maintaining cell composition to a subject wherein the long period pluripotency maintaining cell composition comprises:

a stem cell; and
a polypeptide having a WIF domain.

49. The method according to item 48, wherein the polypeptide having the WIF domain further comprises an EGF-like repeat.

50. The method according to item 48 wherein the differentiated cell is a blood cell.

51. The method according to item 48 wherein the stem cell is a hematopoietic cell.

52. The method according to item 48 wherein the stem cell is present at least at $10^2$ cells.
53. The method according to item 48 wherein the polypeptide having the WIF domain comprises the sequence set forth in SEQ ID NO: 4.
54. The method according to item 48 wherein the polypeptide having the WIF domain is present at least at 0.1 ng/ml.
55. The method according to item 48 wherein the long period pluripotency maintaining cell composition further comprises a stem cell survival agent.
56. The method according to item 55 wherein the stem cell survival agent is SCF.
57. The method according to item 55 wherein the stem cell survival agent is flt-3 ligand.
58. The method according to item 55, wherein the stem cell survival agent is present at least at 1 ng/ml.
59. The method according to item 48 further comprising the step of differentiating the stem cell.
60. The method according to item 48 wherein the subject is a human.
61. The method according to item 48 wherein the polypeptide having the WIF domain is a human recombinant WIF-1 comprising the sequence set forth in SEQ ID NO: 4.
62. A pharmaceutical composition for treating a disease or disorder originating from a disorder of a differentiated cell comprising:

a stem cell;
a polypeptide having a WIF domain; and
a pharmaceutically acceptable carrier.

63. A pharmaceutical composition according to item 62 wherein the polypeptide further comprises an EGF-like repeat.
64. The pharmaceutical composition according to item 62 wherein the differentiated cell is a blood cell.
65. The pharmaceutical composition according to item 62 wherein the stem cell is a hematopoietic cell.
66. The pharmaceutical composition according to item 62 wherein the stem cell is present at least at $10^2$ cells.
67. The pharmaceutical composition according to item 62 wherein the polypeptide having the WIF domain is WIF-1.
68. The pharmaceutical composition according to item 62 wherein the polypeptide having the WIF domain is present at least at 0.1 ng/ml.
69. The pharmaceutical composition according to item 62 further comprising a stem cell survival agent.
70. The pharmaceutical composition according to item 62, wherein the stem survival agent is SCF.
71. The pharmaceutical composition according to item 69, wherein the stem cell survival agent is flt-3 ligand.
72. The pharmaceutical composition according to item 69 wherein the stem cell survival agent is present at least at 1 ng/ml.
73. The pharmaceutical composition according to item 69 wherein the disease or disorder is a human disease or disorder.
74. The pharmaceutical composition according to item 74 wherein the polypeptide having the WIF domain is recombinant human WIF-1 comprising the sequence set forth in SEQ ID NO: 4.
75. Use of a polypeptide having a WIF domain for maintaining the pluripotency of a stem cell without differentiation.
76. The use according to item 75 wherein the polypeptide further comprises an EGF like repeat.
77. The use according to item 75, wherein the stem cell is a hematopoietic cell.
78. The use according to item 75, wherein the stem cell is present at least at $10^2$ cells.
79. The use according to item 75, wherein the polypeptide having the WIF domain comprises the sequence set forth in SEQ ID NO: 4.
80. The use according to item 75, wherein the polypeptide having the WIF domain is present at least at 0.1 ng/ml.
81. The use according to item 75, combined with the use of a stem cell survival agent.
82. The use according to item 81, wherein the stem cell survival agent is SCF.
83. The use according to item 81, wherein the stem cell survival agent is flt-3 ligand.
84. The use according to item 81, wherein the stem cell survival agent is present at least at 1 ng/ml.
85. The use according to item 75 wherein the disease or disorder is a human disease or disorder.
86. The use according to item 75 wherein the polypeptide having the WIF domain is human recombinant WIF-1 comprising the sequence set forth in SEQ ID NO: 4.

BRIEF DESCRIPTION OF DRAWINGS

**Figures 1** (**Figure 1A** and **Figure 1B**) shows the WIF-1 gene in which the domains thereof are shown separately. The sequences are shown for mouse, human, rat, Xenopus, Zebrafish (corresponding to SEQ ID Nos: 2, 4, 6, 8, and 10, respectively) in that order from the top, respectively. The amino acid residues from about position 30 to about position 180 correspond to the WIF domain as defined herein. After about position 180 of the amino acid residues is located an EGF-like repeat.

**Figure 2** shows a scheme showing a method for purifying CD34$^-$KSL cells using FACS.

**Figure 3** shows a FACS figure developed using Lin$^-$ and c-Kit$^+$/Sca-1$^+$.

**Figure 4** shows a scheme showing a single cell culture. The cell survival rate, cell division, and colony formation of the cells prepared by the method can be analyzed using the method described herein.

**Figure 5** shows a scheme for analyzing a stem cell using a mouse.

BEST MODE FOR CARRYING OUT THE INVENTION

Hereinafter, the present invention will be described. It should be understood throughout the present specification that articles for a singular form (e.g., "a", "an", "the", etc. in English; "ein", "der", "das", "die", etc. and their inflections in German; "un", "une", "le", "la", etc. in French; "un", "una", "el", "la", etc. in Spanish, and articles, adjectives, etc. in other languages) include the concept of their plurality unless otherwise mentioned. It should be also understood that the terms as used herein have definitions typically used in the art unless otherwise mentioned.

(Description of the sequences)

SEQ ID NO: 1 shows the full-length nucleotide sequence of murine WIF-1 of the present invention.

SEQ ID NO: 2 shows the full-length amino acid sequence of murine WIF-1 of the present invention.

SEQ ID NO: 3 shows the full-length nucleotide sequence of human WIF-1 of the present invention.

SEQ ID NO: 4 shows the full-length amino acid sequence of human WIF-1 of the present invention.

SEQ ID NO: 5 shows the full-length nucleotide sequence of rat WIF-1 of the present invention.

SEQ ID NO: 6 shows the full-length amino acid sequence of rat WIF-1 of the present invention.

SEQ ID NO: 7 shows the full-length nucleotide sequence of Xenopus WIF-1 of the present invention.

SEQ ID NO: 8 shows the full-length amino acid sequence of Xenopus WIF-1 of the present invention.

SEQ ID NO: 9 shows the full-length nucleotide sequence of Zebrafish WIF-1 of the present invention.

SEQ ID NO: 10 shows the full-length amino acid sequence of Zebrafish WIF-1 of the present invention.

SEQ ID NO: 11 shows a sequence of a degenerate primer (forward direction) used in Example 1.

SEQ ID NO: 12 shows a sequence of a degenerate primer (backward direction) used in Example 1.

As used herein, including SEQ ID NOS: 11 and 12, the nucleic acid references used are as follows: a: adenine; c: cytosine; g: guanine; t: thymine; u: uracil; m: a or c; r: g or a; w: a or t or u; s: g or c; y: t or u or c; k: g or t or u; v: a or g or c; h: a or c or t or u; d: a or g or t or u; b: g or c or t or u; n: (a or c or g or t or u) or (unknown or other base).

SEQ ID NO: 13 shows the sequence of pCAGGS-6His vector.

SEQ ID NOS; 14, 16, 18, and 20 show nucleotide sequences of conservatively substituted variants of the polypeptide containing the WIF domain of the present invention, and SEQ ID NOS: 15, 17, 19 and 21 show the amino acid sequence of the conservatively substituted variants of the polypeptide having the WIF domain of the present invention.

SEQ ID NO: 22 shows the nucleotide sequence of a variant comprising only the WIF domain.

SEQ ID NO: 23 shows the amino acid sequence of a variant comprising only the WIF domain.

SEQ ID NO: 24 shows the nucleotide sequence of a variant comprising only the EGF-like repeat.

SEQ ID NO: 25 shows the amino acid sequence of a variant comprising only the EGF-like repeat.

(Definition of the terms)

The term "cell" is herein used in its broadest sense in the art, referring to a structural unit of tissue of a multicellular organism, which is capable of self replicating, has genetic information and a mechanism for expressing it, and is surrounded by a membrane structure which isolates the cell from the outside.

As used herein, any cell may be used so long as the cell can obtain the capability of obtaining pluripotency and becoming undifferentiated cell by treating the cell with an agent of the present invention, although stem cells may usually be used. As used herein, the term "stem cell" refers to a cell capable of self replication and pluripotency. Typically, stem cells can regenerate an injured tissue. As used herein "life" of a stem cell refers to a phenomenon wherein survival or growth in an undifferentiated state without loss of pluripotency occurs. The "death" of a stem cell refers to a phenomenon leading to differentiation or apoptosis. Stem cells used herein may be, but are not limited to, embryonic stem (ES) cells or tissue stem cells (also called tissular stem cells, tissue-specific stem cells, or somatic stem cells). Embryonic stem cells are pluripotent stem cells derived from early embryos. An embryonic stem cell was first established in 1981, and has been applied to the production of knockout mice since 1989. In 1998, a human embryonic stem cell line was established, which is currently becoming available for regenerative medicine. Therefore in a preferable embodiment of the present invention, embryonic stem cells or embryonic germ cells may be used as the cell. In another preferable embodiment, tissue stem cells such as hematopoietic stem cells are used.

Tissue stem cells have a relatively limited level of differentiation unlike embryonic stem cells. Tissue stem cells are present in tissues and have an undifferentiated intracellular structure. Tissue stem cells have a higher nucleus/cytoplasm ratio and have few intracellular organelles. Most tissue stem cells have pluripotency, a long cell cycle, and proliferative ability beyond the life of the individual. Accordingly, in a preferable embodiment of the present invention, tissue stem cells which are directed to blood cells are used as the cell.

Tissue stem cells are separated into categories of sites from which the cells are derived, such as ectodermic, mesodermic and endodermic. Tissue stem cells in the dermal system include nervous stem cells present in the brain and epidermal stem cells, hair follicle stem cells and pigment stem cells found in the skin, and the like. Tissue stem cells in the mesoderm system include blood vessel stem cells, hematopoietic stem cells, and mesenchymal stem cells found in the bone marrow and blood. Tissue stem cells in the endoderm are mainly present in the organs and include hepatic stem cells, pancreatic stem cells, bowel and epidermal stem cells. In addition, germ line stem cells are present in the testis and ovary. In a preferable embodiment of the present invention, stem cells in the mesoderm system may be used. In a more preferable embodiment of the present invention, hematopoietic stem cells may be used.

Tissue stem cells are separated into categories of sites from which the cells are derived, such as the dermal system, the digestive system, the bone marrow system, the nervous system, and the like. Tissue stem cells in the dermal system include epidermal stem cells, hair follicle stem cells, and the like. Tissue stem cells in the digestive system include pancreatic (common) stem cells, hepatic stem cells, and the like. Tissue stem cells in the bone marrow system include hematopoietic stem cells, mesenchymal stem cells, and the like. Tissue stem cells in the nervous system include neural stem cells, retinal stem cells, and the like. In an embodiment of the present invention, hematopoietic stem cells may be used as the blood cells are derived from the hematopoietic stem cells.

Hematopoiteic stem cells are one of the most studied stem cells up to date. However, hematopoietic stem cells are not completely understood on a number of points such as the plasticity thereof. Mouse models are mostly used for studying hematopoietic stem cells. Hematopoietic stem cells are present at about 3 to 4 cells per 100,000 bone marrow cells (in the case of the B6 mouse). When hematopoietic stem cells are applied to experiments or therapy, it is necessary to separate them from the other bone marrow cells. The most efficient way at present for purifying such hematopoietic stem cells is a method for enriching hematopoietic stem cells comprising staining bone marrow cells using an antibodies labeled with a fluorescence labeled against a number of cell surface antigen, separating them into positive and negative cell fractions using FACS, and selecting the fractions containing hematopoietic stem cells.

Hematopoietic stem cells in the bone marrow are frequently present in CD34 negative or weakly positive, c-Kit positive, Sca-1 positive, lineage marker negative ($CD34^-$KSL) cell fractions. It is known that a competitive repopulation assay can be used to stimulate hematopoietic repopulation for a longer period of time at a ratio of about one per three recipient mice when a $CD34^-$ KSL cell is transplanted into a mouse. Therefore, one in three $CD34^-$KSL cells can be recognized to be a hematopoietic stem cell (Jikken Igaku = Experimental Medicine, Vol. 19, No. 15 (Suppl.) 55-59, 2001). As the level of repopulation is heterogeneous, hematopoietic stem cell population is not homogeneous. Such hematopoietic stem cell enriched populations can be well used in experimental and clinical applications.

In the present invention, such enriched or purified hematopoietic stem cells populations may be used as a cell source.

As used herein, the term "differentiated cell" refers to a cell having a specialized function and form (e.g., muscle cells, neurons, blood cells, etc.). Unlike stem cells, differentiated cells have no or little pluripotency. Examples of differentiated cells include epidermic cells, pancreatic parenchymal cells, pancreatic duct cells, hepatic cells, bile duct cells, blood cells (such as red blood cells, platelets, T cells, B cells or the like), cardiac muscle cells, skeletal muscle cells, osteoblasts, skeletal myoblasts, neurons, vascular endothelial cells, pigment cells, smooth muscle cells, adipocytes, bone cells, cartilage cells, and the like. Therefore, in an embodiment of the present invention, when a differentiated cell can be treated by an agent of the present invention to maintain an undifferentiated state and to obtain pluripotency, such a differentiated cell falls within the scope of the present invention. A differentiated cell prepared from the stem cell of the present invention can be used for the treatment and/or the prevention of a variety of diseases and disorders. Accordingly, such a differentiated cell and a composition comprising such a differentiated cell will also fall within the scope of the present invention. Such a differentiated cell can be prepared by the application of differentiation factors (for example, hematopoietic factors such as erythropoietin (EPO), a variety of interleukins (such as IL-3) or the like). As used herein the term "differentiation factor" is an agent (such as a polypeptide) for promoting differentiation by acting on a stem cell. Examples of such differentiation factors include cytokines including a variety of interleukins (such as IL-3, IL-6, IL-11 or the like), colony stimulating factor (such as EPO, thrombopoietin (TPO), granulocyte-colony stimulating factor (G-CSF) granulocyte-macrophage-colony stimulating factor (GM-CSF), macrophage-colony stimulating factor (M-CSF) or the like); a variety of growth factors including Hepatic growth factor (HGF), Epidermal Growth Factor (EGF), Vascular Endothelial growth factor (VEGF), Platelet-Derived Growth factor (PDGF), and Fibroblast Growth Factor (FGF).

As used herein, the term "isolated" refers to a status of a substance or a cell, and that the substance or cell is in a different status than that of the natural state, and means that at least one substance or cell, which naturally associates therewith, does not associate. As used herein, the term "concentration" or "enrichment" of a cell refers to elevating the occurrence of a specific cell present from that of the naturally occurring state. As used herein, the term "purification" of a cell refers to bringing the frequency of the cell to a higher level, and cells other than the cells of interest will be present in a manner such that the function of the cells of interest will be substantially unaffected. Accordingly, in a preferable state, purified cells or cell compositions comprise particular cells only. As used herein "purifying" a substance refers to bringing the frequency of the substance to a higher level, and substances other than the substance of interest will be present in a manner such that the function of the substances of interest will be substantially unaffected. Accordingly, in a preferable state, purified substance or compositions comprising the same will comprise a particular substance only.

Further, cells having pluripotency of the present invention can differentiate into at least a blood-lymphoid system cell (T cell, B cell, plasma cell, basophil, eosinophil, monocyte, macrophage, neutrophil, megakaryocyte, platelet, erythroblast, erythrocyte or the like), and preferably the cells having pluripotency of the present invention can differentiate into all the blood-lymphoid system cells. Accordingly, the preferable differentiated cells of the present invention may be a blood-lymphoid system cells such as T cells, B cells, plasma cells, megakaryocytes, platelets, erythroblasts, erythrocytes and the like.

As used herein, the terms "differentiation" or "cell differentiation" refers to a phenomenon that two or more types of cells having qualitative differences in form and/or function occur in a daughter cell population derived from the division of a single cell.

Therefore, "differentiation" includes a process during which a population (family tree) of cells, which do not originally have a specific detectable feature, acquires a feature, such as the production of a specific protein, or the like. At present, cell differentiation is generally considered to be a state of a cell in which a specific group of genes in the genome are expressed. Cell differentiation can be identified by searching for intracellular or extracellular agents or conditions which elicit the above-described state of gene expression. Differentiated cells are stable in principle. Particularly, animal cells which have been once differentiated are rarely differentiated into other types of cells. Therefore, the acquired pluripotent cells of the present invention are considerably useful. "Undifferentiated (state)" refers to a state of a cell in which the cell has no qualitative difference in forms or functions.

As used herein, the term "pluripotency" refers to a nature of a cell, i.e., an ability to differentiate into a variety of tissues or organs. Typically, the pluripotency of a cell is limited as the cell is developed, and in an adult, where the pluripotency is usually lost, cells constituting a tissue or organ rarely alter to different cells. Particularly, epithelial cells resist altering to other types of epithelial cells. Such alteration typically occurs in pathological conditions, and is called metaplasia. However, mesenchymal cells tend to easily undergo metaplasia, i.e., alter to other mesenchymal cells, with relatively simple stimuli. Therefore, mesenchymal cells have a high level of pluripotency. Therefore, the term "maintaining pluripotency" reters to maintenance of a pluripotency of cells such as stem cells. The present invention is a breakthrough invention which has attained the production of a stem cell which can maintain pluripotency for a longer period of time than a normal stem cell.

Cells used herein may be derived from any organism (e.g., vertebrates and invertebrates). Preferably, cells from a vertebrate are used and more preferably, cells from a mammal such as primates, rodentia etc. are used. Still more preferably, cells from primates are used. Most preferably, cells from humans are used.

As used herein the term "WIF domain" refers to a N-terminal region of the WIF-1 protein without the signal peptide thereof and extending until the EGF-like repeat starts. Therefore, in the case of the murine WIF domain, such a domain corresponds to the amino acid number about position 30 to about position 180 in the sequence of SEQ ID NO: 2. As used herein, the "WIF domain" will include, in addition to the identical sequence of the amino acid number about position 30 to at about position 180 of SEQ ID NO: 2, the identical sequence of the amino acid number at about position 30 to at about position 180 of human WIF domain as set forth in SEQ ID NO: 4, the identical sequence of the amino acid number at about position 30 to at about position 180 of the Xenopus WIF domain as set forth in SEQ ID NO: 8, the identical sequence of the amino acid number at about position 30 to at about position 180 of the Zebrafish WIF domain as set forth in SEQ ID NO: 10, as well as similar sequences thereof. As used herein, such similar sequences are preferably conservatively substituted variants of a sequence selected from the group consisting of the WIF domains of the mouse, human, Xenopus and Zebrafish sequences as described above. Conservative substitution is defined hereinbelow. In the present invention, a polypeptide having a WIF domain of the above-mentioned length is usually used, however, a polypeptide having a WIF domain having a shorter length than the WIF domain may be used. Such a polypeptide having a WIF domain having a shorter length than the WIF domain can be readily determined to have a function of the WIF domain by the use of an *in vitro* inhibition activity assay for the Wnt protein as set forth in Hsieh J.H., et al., Nature 398,431-436, 1999. Accordingly, such a shorter WIF domain will include, among the group of the identical sequence of the amino acid number at about position 30 to at about position 180 of SEQ ID NO: 2, the identical sequence of the amino acid number at about position 30 to at about position 180 of human WIF domain as set forth in SEQ ID NO: 4, the identical sequence of the amino acid number at about position 30 to at about position 180 of the Xenopus WIF domain as set forth in SEQ ID NO: 8, and the identical sequence of the amino acid number at about position 30 to at about position 180 of the Zebrafish WIF domain as set forth in SEQ ID NO: 10, at least 5 amino acids, at least 6 amino acids, at least 7 amino acids, at least 8 amino acids, at least 9 amino acids, at least 10 amino acids, at least 11 amino acids, at least 12 amino acids, at least 13 amino acids, at least 14 amino acids, at least 15 amino acids, at least 20 amino acids, at least 25 amino acids, at least 30 amino acids, at least 40 amino acids, at least 50 amino acids, at least 60 amino acids, at least 70 amino acids, at least 80 amino acids, at least 90 amino acids, at least 100 amino acids, at least 110 amino acids, at least 120 amino acids, at least 130 amino acids, at least 140 amino acids, or at least 150 amino acids of this group. As used herein, a preferable polypeptide having a WIF domain is WIF-1. The amino acid sequences of the WIF-1 are, as used herein, SEQ ID NOs : 2, 4, 6, 8 and 10 (murine, human, rat, *Xenopus,* and Zebrafish, respectively), and these are well conserved among the species (see Figure 1).

As used herein, the term "EGF like repeat" refers to a specific domain in Epidermal Growth Factor. It is a repeated sequence having six cysteines in the repeat unit, and in the EGF, it has nine repeats consisting of the repeated unit"$CX_7CX_{4-5}CX_{10-13}CXCX_8C$" wherein X refers to any amino acid. A useful polypeptide for the present invention preferably comprises at least one EGF-like repeat, and more preferably, may have two, three, four or five EGF-like repeats. WIF-1 has five repeats consisting of the repeating unit"$CX_3CX_5CX_5CXCX_8X_4$" wherein X refers to any amino acid. WIF-1 of the present invention has five EGF-like repeats, and the location thereof is as follows: in the case of murine WIF-1 polypeptide (SEQ ID NO: 2), amino acid numbers 182-213, 213-245, 245-277, 278-309 and 310-341. Polypeptides having an EGF-like repeat include notch-1, notch-2, jagged-1, D11-1, DLK and the like. A polypeptide having an EGF-like repeat has been shown to be involved in controlling differentiation of a stem cell in a general sense (WO 97/31467A1), however, no activity has been shown for maintaining pluripotency with blocking or delaying differentiation (i.e. maintaining the undifferentiated state) until the present invention discloses such an activity in WIF-1, and therefore, the significance of the present invention should be acknowledged.

The cysteine residues included in the WIF-1 polypeptide are located at amino acid positions: 140, 177, 182, 186, 192, 198, 200, 209, 214, 218, 224, 230, 232, 241, 246, 250, 256, 262, 264, 273, 278, 282, 288, 294, 296, 305, 310, 314, 320, 326, 328 and 337 of SEQ ID NO: 2. These residues are conserved among species.

Portions to which carbohydrate chains can be added may be a portion to which an N-acetyl-D-glucosamine can be bound by N-glucoside binding, and include positions 120, 158, 170, 193, 220, 240, 245, 252, 284, 324 and 338. These positions are also conserved among many species. Further, a portion to which an N-acetyl-D-galactosamine can be bound by O-glucoside binding, include a portion in which serine or threonine residues are often present. These proteins with carbohydrates added are usually stable against degradation *in vivo* and may have more potent physiological activity. Accordingly, these polypeptide with carbohydrate added are also within the scope of the present invention.

The polypeptides of the present invention may be from any organism. Preferably, the organism is a vertebrate (such as mammals, reptiles, amphibians, fish, avifauna etc.), more preferably, mammals (for example, rodentia (mouse, rat, etc.), primates (such as humans) etc.). In another embodiment, the polypeptide of the present invention may be synthesized. Methods for synthesizing amino acids are well known in the art. Such a synthesizing method includes methods utilizing peptide synthesizers (such as those available from Applied Biosystems and the like).

The polypeptide of the present invention may use polymeric (such as dimeric or higher) type polypeptides having more potent specific activity comprising a fusion protein produced by a method for forming dimers using disulfide bonds by expressing a fusion protein with a single hinge region portion of an antibody, or a fusion protein produced to be expressed at the C-terminus, N-terminus, or other site in a form such that a disulfide bond is formed by any different method not affecting the activity of the polypeptide. Further, a method for producing a polymeric structure by aligning a sequence of the present invention such as SEQ ID NO: 2 in tandem, may be used. Accordingly, any dimers or polymers higher order oligomer produced by genetic engineering technology is also within the scope of the present invention.

As used herein, the term "stem cell survival agent" is an agent considered to be essential for survival of a stem cell. Conventionally, the survival (and differentiation) of stem cells has been thought to require SCF. However, Ornitz M.,et al.(Immunity,Vol.10,173-182,1999) discovered a pluripotent hematopoietic stem cell which does not express c-Kit which is a receptor for SCF. Therefore, SCF *per se* is not required for survival of all stem cells. Thus, those skilled in the art understand that a stem cell survival agent may vary depending on the stem cell of interest.

Accordingly, those skilled in the art will recognize that stem cell survival agents will vary depending on the stem cell used. Stem cell survival agents include SCF, TPO, flt-3 ligand and the like. In one embodiment, a stem cell survival agent may be SCF (also known as steel factor). SCF was identified as an agent for controlling stem cell function. However, as described herein, SCF per se has no activity relating to self-replication of stem cells, and rather it became apparent that SCF supports the survival of cells having the c-Kit receptor, and directs cells to differentiation as a result. Accordingly, SCF is considered to serve as a species of stem cell survival agent and is a preferred example of stem cell survival agent herein.

Further, conventionally, a positive CD34 indication is considered to be an important marker for pluripotency, and the expression thereof has been considered to be indicative of having pluripotency. However, it has herein become apparent that such a positive indication is not always correlated with pluripotency. Thus, in the present invention, the surprising finding that a protein having WIF domain has a function of maintaining pluripotency without inducing differentiation of stem cells has been made.

As a ligand of Notch, Jagged-1 is known (J.Exp.Med. 192,1365-1372,2000). Jagged-1 has been shown to have relation with hematopoietic cells, however, it has not been shown that Jagged-1 alone or in combination with SCF has a function of maintaining pluripotency with an undifferentiated state.

Hematopoietic factors such as TPO have been shown to be related to differentiation control in hematopoietic stem cells. Blood, 1 October 2001, Vol. 98, No. 7, pp. 2091-2100 showed the roles of survivin, which is a member of the family of apoptosis suppression factors, in normal umbilical cord blood and bone marrow CD34 positive cells in relation to hematopoietic factors. The combination of TPO, Flt3 ligand (FL) and SCF was shown to upregulate the expression of survivin in CD34+ cells within 24 hours. Further, it was shown that the expression of survivin and the entry into the cell cycle coincide with each other. However, no relationship has been studied with cell differentiation.

As used herein, the terms "protein", "polypeptide" and "peptide" are interchangeably used to refer to a macromolecule (polymer) consisting of a series of amino acids. Amino acid refers to an organic molecule having carboxyl and amino groups on a carbon atom. As used herein, preferably, such an amino acid may be, but is not limited to, one of the twenty naturally-occurring amino acids.

The terms "polynucleotide", "oligonucleotide", and "nucleic acid" as used herein have the same meaning and refer to a nucleotide polymer having any length. Nucleotide refers to a nucleoside in which a portion is replaced with phosphate ester. The base portion thereof includes pyrimidine bases or purine bases (such as pyrimidine nucleotides and purine nucleotides). Polynucleotides include DNA and RNA.

A method for producing the polypeptide of the present invention includes, for example, a method for culturing primary cells or cell lines producing the polypeptide and isolating or purifying the protein from the culture supernatant thereof. Alternatively, it is possible to use genetic engineering methods, to incorporate a gene encoding the polypeptide into an appropriate expression vector, to transform an expression host with the vector and to obtain a recombinant physiologically active substance (for example, cell growth factor) from the culture supernatant of the transformed cells. The host cell includes but is not limited to any cell so long as the cell expresses the polypeptide, and those skilled in the art can use a variety of host cells that can be used in the art for genetic engineering, such as *E. coli,* yeast, plant cells, insect cells, animal cells, and the like. The polypeptide thus obtained can be used so long as the polypeptide has the substantially similar activity and can include one or more amino acid substitutions, additions and/or deletions in an amino acid sequence, and one or more carbohydrate chain can be substituted, added and/or deleted.

A given amino acid may be substituted with another amino acid in a protein structure, such as a cationic region or a substrate molecule binding site, without a clear reduction or loss of interactive binding ability. A given biological function of a protein is defined by the interactive ability or other property of the protein. Therefore, a particular amino acid substitution may be performed in an amino acid sequence, or at the DNA code sequence level, to produce a protein which maintains the original property after the substitution. Therefore, various modifications of peptides as disclosed herein and DNA encoding such peptides may be performed without clear losses of biological usefulness.

When the above-described modifications are designed, the hydrophobicity indices of amino acids may be taken into consideration. The hydrophobic amino acid indices play an important role in providing a protein with an interactive biological function, which is generally recognized in the art (Kyte, J. and Doolittle, R.F., J. Mol. Biol. 157(1): 105-132, 1982). The hydrophobic property of an amino acid contributes to the secondary structure of a protein and then regulates interactions between the protein and other molecules (e.g., enzymes, substrates, receptors, DNA, antibodies, antigens, etc.). Each amino acid is given a hydrophobicity index based on the hydrophobicity and charge properties thereof as follows: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamic acid (-3.5); glutamine (-3.5); aspartic acid (-3.5); asparagine (-3.5); lysine (-3.9); and arginine (-4.5).

It is well known that if a given amino acid is substituted with another amino acid having a similar hydrophobicity index, the resultant protein may still have a biological function similar to that of the original protein (e.g., a protein having an equivalent enzymatic activity). For such an amino acid substitution, the hydrophobicity index is preferably within $\pm 2$, more preferably within $\pm 1$, and even more preferably within $\pm 0.5$. It is understood in the art that such an amino acid substitution based on hydrophobicity is efficient. As described in US Patent No. 4,554,101, amino acid residues are given the following hydrophilicity indices: arginine (+3.0); lysine (+3.0); aspartic acid ($+3.0\pm 1$); glutamic acid ($+3.0\pm 1$); serine (+0.3); asparagine (+0.2); glutamine (+0.2); glycine (0); threonine (-0.4); proline ($-0.5\pm 1$); alanine (-0.5); histidine (-0.5); cysteine (-1.0); methionine (-1.3); valine (-1.5); leucine (-1.8); isoleucine (-1.8); tyrosine (-2.3); phenylalanine (-2.5); and tryptophan (-3.4). It is understood that an amino acid may be substituted with another amino acid which has a similar hydrophilicity index and can still provide a biological equivalent. For such an amino acid substitution, the hydrophilicity index is preferably within $\pm 2$, more preferably $\pm 1$, and even more preferably $\pm 0.5$.

The term "conservative substitution" as used herein refers to amino acid substitution in which a substituted amino acid and a substituting amino acid have similar hydrophilicity indices or/and hydrophobicity indices as described above. Examples of conservative substitution include, but are not limited to, substitutions within each of the following residue pairs: arginine and lysine; glutamic acid and aspartic acid; serine and threonine; glutamine and asparagine; and valine, leucine, and isoleucine, and the like, which are well known to those skilled in the art.

In order to prepare functionally equivalent polypeptides, amino acid additions, deletions, or modifications can be performed in addition to amino acid substitutions. Amino acid substitution(s) refers to the replacement of at least one amino acid of an original peptide chain with different amino acids, such as the replacement of 1 to 10 amino acids, preferably 1 to 5 amino acids, and more preferably 1 to 3 amino acids with different amino acids. Amino acid addition (s) refers to the addition of at least one amino acid to an original peptide chain, such as the addition of 1 to 10 amino acids, preferably 1 to 5 amino acids, and more preferably 1 to 3 amino acids to an original peptide chain. Amino acid deletion(s) refers to the deletion of at least one amino acid, such as the deletion of 1 to 10 amino acids, preferably 1 to 5 amino acids, and more preferably 1 to 3 amino acids. Amino acid modification includes, but is not limited to, amidation, carboxylation, sulfation, halogenation, truncation, lipidation, alkylation, glycosylation, phosphorylation, hydroxylation, acylation (e.g., acetylation), and the like. Amino acids to be substituted or added may be naturally-occurring or nonnaturally-occurring amino acids, or amino acid analogs. Naturally-occurring amino acids are preferable. Those skilled in the art can determine whether or not such a variant is functionally equivalent by evaluating whether or not the variant has a similar function to the indicator polypeptide using any assay described herein.

As used herein, the term "peptide analog" refers to a compound which is different from a peptide but has at least one chemical or biological function equivalent to the peptide. Therefore, a peptide analog includes one that has at least one amino acid analog or amino acid derivative addition or substitution with respect to the original peptide. A peptide analog has the above-described addition or substitution so that the function thereof is substantially the same as the function of the original peptide (e.g., a similar pKa value, a similar functional group, a similar binding manner to ligands, a similar water-solubility, and the like). Such a peptide analog can be prepared using a technique well known in the art.

Such a nucleic acid molecule encoding a polypeptide used in the present invention includes one in which a part of the sequence of the nucleic acid is deleted or is substituted with other base(s), or an additional nucleic acid sequence is inserted, as long as a polypeptide expressed by the nucleic acid has substantially the same activity as that of the polypeptide of the present invention, as described above. Alternatively, an additional nucleic acid may be linked to the 5' terminus and/or 3' terminus of the nucleic acid. The nucleic acid molecule may include one that is hybridizable to a gene encoding a polypeptide under stringent conditions and encodes a polypeptide having substantially the same function. Such a method for producing such a nucleic acid is known in the art and can be used in the present invention.

The above-described nucleic acid can be obtained by a well-known PCR method, i.e., chemical synthesis. This method may be combined with, for example, site-specific mutagenesis, hybridization, or the like.

Molecular biological techniques, biochemical techniques, and microorganism techniques as used herein are well known in the art and commonly used, and are described in, for example, Ausubel F.A. et al., ed., (1988), Current Protocols in Molecular Biology.Wiley.New York, NY; Sambrook J. et al., (1987) Molecular Cloning: A Laboratory Manual, 2nd Ed., Cold Spring Harbor Laboratory Press, Cold Spring Harbor, NY; Special issue, Jikken Igaku [Experimental Medicine] "Idenshi Donyu & Hatsugenkaiseki Jikkenho [Experimental Methods for Gene Introduction & Expression Analysis]", Yodo-sha, 1997, and the like.

As used herein, the term "fragment" refers to a polynucleotide having a sequence length ranging from 1 to n-1 with respect to the full length of the reference polynucleotide (of length n). The length of the fragment can be appropriately changed depending on the purpose. For example, in the case of polypeptides, the lower limit of the length of the fragment includes 3, 4, 5, 6, 7, 8, 9, 10, 15, 20, 25, 30, 40, 50 or more amino acids. Lengths represented by integers which are not herein specified (e.g., 11 and the like) may be appropriate as a lower limit. For example, in the case of polynucleotides, the lower limit of the length of the fragment includes 5, 6, 7, 8, 9, 10, 15, 20, 25, 30, 40, 50, 75, 100 or more nucleotides. Lengths represented by integers which are not herein specified (e.g., 11 and the like) may be appropriate as a lower limit.

In order to use a fragment of a nucleic acid molecule as a selective promoter or selective primer against the original nucleic acid from which it is derived, it is required to be a fragment which specifically hybridizes thereto. As used herein, DNAs which "specifically hybridize" refers to the capability of distinguishably detecting or amplifying a second nucleic acid molecule. A selective probe may be typically at least 10 nucleotides in length, preferably at least 15 nucleotides in length, more preferably at least 20 nucleotides in length, still more preferably at least 30, 40 or 50 nucleotides in length, and a length of more than 50 nucleotides can also be used. A selective probe is available as a PCR amplified product using such selective primers. When using a selective primer as at least one of a pair of primers for PCR, such a selective primer may be at least 9 nucleotides in length, preferably at least 10 nucleotides in length, more preferably at least 15 nucleotides in length, still more preferably at least 17, 18, 19, 20, 21, 22, 23, 24, 25, 30 or 50 nucleotides in length.

As used herein, "homology" of a gene refers to the proportion of identity between two or more gene sequences. Therefore, the greater the homology between two given genes, the greater the identity or similarity between their sequences. Whether or not two genes have homology is determined by comparing their sequences directly or by a hybridization method under stringent conditions. When two gene sequences are directly compared with each other, these genes have homology if the DNA sequences of the genes have representatively at least 50% identity, preferably at least 70% identity, more preferably at least 80%, 90%, 95%, 96%, 97%, 98%, or 99% identity with each other.

The similarity, identity and homology of amino acid sequences and base sequences are herein compared using BLAST (sequence analyzing tool) with the default parameters.

As used herein, "polynucleotides hybridizing under stringent conditions" refers to conditions commonly used and well known in the art. Such a polynucleotide can be obtained by conducting colony hybridization, plaque hybridization, southern blot hybridization, or the like using a polynucleotide selected from the polynucleotides of the present invention. Specifically, a filter on which DNA derived from a colony or plaque is immobilized is used to conduct hybridization at 65°C in the presence of 0.7 to 1.0 M NaCl. Thereafter, a 0.1 to 2-fold concentration SSC (saline-sodium citrate) solution (1-fold concentration SSC solution is composed of 150 mM sodium chloride and 15 mM sodium citrate) is used to wash the filter at 65°C. Polynucleotides identified by this method are referred to as "polynucleotides hybridizing under stringent conditions". Hybridization can be conducted in accordance with a method described in, for example, Molecular Cloning 2nd ed., Current Protocols in Molecular Biology, Supplement 1-38, DNA Cloning 1: Core Techniques, A Practical Approach, Second Edition, Oxford University Press (1995), and the like. Here, sequences hybridizing under stringent conditions exclude, preferably, sequences containing only A (adenine) or T (thymine).

As used herein, "hybridizable polynucleotide" refers to a polynucleotide which can hybridize to other polynucleotides under the above-described hybridization conditions. Specifically, the hybridizable polynucleotide includes at least a polynucleotide having a homology of at least 60% to the base sequence of DNA encoding a polypeptide having an amino acid sequence as set forth in SEQ ID NO:2, 4, 6 or 8, preferably a polynucleotide having a homology of at least 80%, and more preferably a polynucleotide having a homology of at least 95%. The homology of a nucleic acid sequence may be represented by similarity evaluated with a score using, for example, the search program BLAST which uses an algorithm developed by Altschul et al., J. Mol. Biol., 215, 403-410(1990)).

An "oligonucleotide derivative" includes a nucleotide derivative, or refers to an oligonucleotide having different linkages between nucleotides from typical linkages. Examples of such an oligonucleotide specifically include 2'-O-methyl-ribonucleotide, an oligonucleotide derivative in which a phosphodiester bond in an oligonucleotide is converted to a phosphorothioate bond, an oligonucleotide derivative in which a phosphodiester bond in an oligonucleotide is converted to a N3'-P5' phosphoroamidate bond, an oligonucleotide derivative in which a ribose and a phosphodiester bond in an oligonucleotide are converted to a peptide-nucleic acid bond, an oligonucleotide derivative in which uracil in an oligonucleotide is substituted with C-5 propynyl uracil, an oligonucleotide derivative in which uracil in an oligonucleotide is substituted with C-5 thiazole uracil, an oligonucleotide derivative in which cytosine in an oligonucleotide is substituted with C-5 propynyl cytosine, an oligonucleotide derivative in which cytosine in an oligonucleotide is substituted with phenoxazine-modified cytosine, an oligonucleotide derivative in which ribose in DNA is substituted with 2'-O-propyl ribose, and an oligonucleotide derivative in which ribose in an oligonucleotide is substituted with 2'-methoxyethoxy ribose.

As used herein, "amino acid" may be natural or unnatural as described above. "Amino acid derivative" refers to a substance which is different from a natural amino acid but has similar function to the original amino acid. Such an amino acid derivative is well known in the art.

As used herein, "nucleotide" may be natural or unnatural as described above. "Nucleotide derivative" refers to a substance which is different from a natural nucleotide but has similar function to the original nucleotide. Such a nucleotide derivative is well known in the art.

As used herein, the term "epitope" refers to an antigenic determinant whose structure is clear. Methods for determining epitopes are well known in the art, and those skilled in the art can determine such well known and commonly used technology to produce the same once the nucleic acid or amino acid primary sequence information is available. Generally, in order to use as an epitope such a sequence requires a length of at least 3 such amino acids, and preferably, at least a length of 4, 5, 6, 7, 8, 9, 10, 15, 20 or 25 such amino acids.

As used herein, the term "biological activity" refers to activity possessed by an agent (e.g., a polypeptide or protein etc.) within an organism, including activities exhibiting various functions. For example, when a given agent is an enzyme, the biological activity thereof includes the emzymatic activity thereof. In another example, when a given agent is a ligand, the biological activity thereof includes binding of the agent to a receptor for the ligand. In a protein having a WIF domain which is an embodiment of the present invention, such a biological activity includes at least one binding activity to a receptor of the Wnt family.

As used herein, the term "variant" refers to a substance, such as a polypeptide, polynucleotide, or the like, which differs partially from the original substance. Examples of such a variant include a substitution variant, an addition variant, a deletion variant, a truncated variant, an allelic variant, and the like. The term "allele" as used herein refers to a genetic variant located at a locus identical to a corresponding gene, where the two genes are distinguished from each other. Therefore, the term "allelic variant" as used herein refers to a variant which has an allelic relationship with a given gene. The term "species homolog" or "homolog" as used herein refers to one that has a nucleotide homology with the nucleotide sequence of a given reference nucleic acid. Homologs are typically polynucleotides which hybridize to the reference nucleic acid molecule under stringent conditions. In reference to a nucleic acid molecule of the present invention, "homolog" refers to a nucleic acid molecule having a nucleic acid sequence having homology with the nucleic acid sequence encoding the amino acid sequence of the protein, and has the same or similar biological function with that of the present invention. Accordingly, the "homolog" and "variant" is a concept which partially overlaps to each other. The term "homolog" as used herein refers to one that has an amino acid or nucleotide homology with a given gene in a given species (preferably at least 60% homology, more preferably at least 80%, at least 85%, at least 90%, and at least 95% homology). A method for obtaining such a homolog is understood from the description of the present specification. For example, a homolog of WIF-1 of the present invention can be a homologous gene of the same species or a gene corresponding to that of the other species. Thus, the protein having a WIF domain of the present invention includes all the homologs of WIF-1.

As used herein, when it is necessary to introduce a vector, a method for introducing a vector may be any method so long as the method can introduce a DNA into a cell. Such a method includes transfection, transduction, transformation, and the like (for example, electroporation methods, particle gun (gene gun) methods, or the like).

When a gene is mentioned herein, the term "vector" or "recombinant vector" refers to a vector capable of transferring a polynucleotide sequence of interest to a target cell. Such a vector includes those capable of self-replication or incorporation into a chromosome in a host cell (e.g., a prokaryotic cell, yeast, an animal cell, a plant cell, an insect cell, an individual animal, and an individual plant, etc.), or those containing a promoter at a site suitable for transcription of a polynucleotide of the present invention. Promoters may be those capable of enhancing expression in a host cell such an *E.coli,* yeast or the like. These will include trp promoter (Ptrp), lac promoter (Plac), PL promoter, PR promoter, PSE promoter or and the like, promoters derived from E.coli and phages and the like, SPO1 promoter, SPO2 promoter, penP promoter and the like. Artificially modified promoters such as those having two Ptrp tandemly located (Ptrp x2), tac promoter, lacT7 promoter, letI promoter and the like may be used.

A vector suitable for cloning is referred to as "cloning vector". Such a cloning vector ordinarily contains a multiple cloning site containing a plurality of restriction sites. Restriction sites and multiple cloning sites are well known in the art and may be appropriately or optionally used depending on the purpose. The technology is described in references as described herein (e.g., Sambrook et al. (*supra*)). Such vectors include, for example, plasmids.

"Recombinant vectors" for prokaryotic cells include pBTrp2, pBTac1, pBTac2 (both available from Roche Molecular Biochemicals), pKK233-2(Pharmacia), pSE280 (Invitrogen), pGEMEX-1 (Promega), pQE-8 (QIAGEN), pKYP10 (Japanese Laid-Open Publication No.: 58-110600), pKYP200 (Agric.Biol.Chem., 48,669(1984)), pLSA1 (Agric.Biol.Chem.,53,277(1989)), pGEL1 (Proc.Natl:Acad.Sci.USA,82,4306(1985)), pBluescript II SK+(Stratagene), pBluescript II SK(-) (Stratagene), pTrs30 (FERM BP-5407), pTrs32 (FERM BP-5408), pGHA2 (FERM BP-400), pGKA2 (FERM B-6798), pTerm2(Japanese Laid-Open Publication No.: 3-22979, US4686191, US4939094, US5160735), pEG400 (J.Bacteriol., 172,2392(1990)), pGEX (Pharmacia), pET systems (Novagen), pSupex, pUB110, pTP5, pC194, pTrxFus (Invitrogen), pMAL-c2 (New England Biolabs), pUC19 (Gene,33,103(1985)), pSTV28 (TaKaRa), pUC118 (TaKaRa), pPA1 (Japanese Laid-Open Publication No.: 63-233798), and the like.

"Recombinant vectors" for yeast cells include YEp13 (ATCC37115), YEp24 (ATCC37051). YCp50 (ATCC37419), pHS19, pHS15 and the like.

"Recmobinant vectors" for animal cells include pcDNAI/Amp, pcDNAI, pCDM8 (all from available from Funakoshi), pAGE107 (Japanese Laid-Open Publication No.: 3-229) (Invitrogen), pAGE103 (J.Biochem.,101,1307(1987)), pAMo, pAMoA (J. Biol. Chem., 268, 22782-22787(1993)) and the like.

Exemplary "recombinant vectors" for plant cells include Ti plasmid, tobacco mosaic virus vector and the like.

Exemplary "recombinant vectors" for insect cells include pVL1392, pVL1393, pBlueBacIII (all available from Invitrogen) and the like.

As used herein, the term "transformant" refers to the whole or a part of an organism, such as a cell, which is produced by transformation. Examples of a transformant include a prokaryotic cell, yeast, an animal cell, a plant cell, an insect cell, and the like. Transformants may be referred to as transformed cells, transformed tissue, transformed hosts, or the like, depending on the subject.

When a prokaryotic cell is used herein for genetic operations or the like, the prokaryotic cell may be of, for example, the genus *Escherichia,* the genus *Serratia,* the genus *Bacillus,* the genus *Brevibacterium,* the genus *Corynebacterium,* the genus *Microbacterium,* the genus *Pseudomonas,* or the like. Specifically, the prokaryotic cell is, for example, *Escherichia coli* XL1-Blue, *Escherichia coli* XL2-Blue, *Escherichia coli* DH1, *Escherichia coli* MC1000, *Escherichia coli* KY3276, *Escherichia coli* W1485, *Escherichia coli* JM109, *Escherichia coli* HB101, *Escherichia coli* No. 49, *Escherichia coli* W3110, *Escherichia coli* NY49, *Escherichia coli* BL21(DE3), *Escherichia coli* BL21(DE3)pLysS, *Escherichia coli* HMS174(DE3), *Escherichia coli* HMS174(DE3)pLysS, *Serratia ficaria, Serratia fonticola, Serratia liquefaciens, Serratia marcescens, Bacillus subtilis, Bacillus amyloliquefaciens, Brevibacterium ammmoniagenes, Brevibacterium immariophilum* ATCC14068, *Brevibacterium saccharolyticum* ATCC14066, *Corynebacterium glutamicum* ATCC13032, *Corynebacterium glutamicum* ATCC14067, *Corynebacterium glutamicum* ATCC13869, *Corynebacterium acetoacidophilum* ATCC13870, *Microbacterium ammoniaphilum* ATCC15354, *Pseudomonas* sp. D-0110 or the like.

When a yeast cell is used herein, such a cell includes yeast cells belonging to the genus *Saccharomyces,* genus *Schizosaccharomyces,* the genus *Kluyveromyces,* the genus *Trichosporonm,* the genus *Schwanniomyces,* and specifically include, for example, *Saccharomyces cerevisiae, Schizosaccharomyces pombe, Kluyveromyces lactis, Trichosporon pullulans, Schwanniomyces alluvius, Pichia pastoris* and the like. A method for introducing such a recombinant vector may be any method for introducing DNA into a yeast, and includes electroporation methods [Methods. Enzymol.,194,182(1990)], spheroplast methods [Proc.Natl.Acad.Sci.USA,84,1929(1978)], lithium acetate methods [J. Bacteriol.,153,163(1983)], a method described in Proc.Natl. Acad. Sci. USA, 75,1929 (197a) and the like.

Examples of an animal cell as used herein includes a mouse myeloma cell, a rat myeloma cell, a mouse hybridoma cell, a Chinese hamster ovary (CHO) cell, a baby hamster kidney (BHK) cell, an African green monkey kidney cell, a human leukemic cell, HBT5637 (Japanese Laid-Open Publication No. 63-299), a human colon cancer cell line, and the like. The mouse myeloma cells include ps20, NSO, and the like. The rat myeloma cells include YB2/0 and the like. Human embryo kidney cells include HEK293 (ATCC:CRL-1573) and the like. The human leukemic cells include BALL-1 and the like. The African green monkey kidney cells include COS-1, COS-7, and the like. The human colon cancer cell lines include HCT-15, and the like.

Plant cells include those of potato, tobacco, maize, rice, oilseed, soybean, tomato, carrot, wheat, barley, rye, alfalfa, linum and the like. A method for introducing such an expression vector may be any method for introducing DNA into a plant cell, and include, for example, Agrobacterium methods (Japanese Laid-Open Publication Nos.: 59-140885, 60-70080, WO 94/00977), electroporation methods (Japanese Laid-Open Publication No.: 60-251887), particle gun (gene gun) methods (Japanese Patent Nos. 2606856 and 2517813) and the like.

An insect cell includes a cultured cell derived from ovarian cells of *Spodoptera frugiperda,* or *Bombyx mori* ovary. *Spdoptera frugiperda* ovarian cells include Sf9, Sf21 (Baculovirus Expression Expression Vectors: A Laboratory Manual) or the like, *Trichoplusia ni* ovarian cells include High 5, BTI-TN-5B1-4 (Invitrogen) or the like, cultured cells derived from *Bombyx mori* ovary include as an example *Bombyx mori* N4.

A method for producing a polypeptide or the salt thereof can employ any well known technology in the art and include for example, a method wherein a transformant such as prokaryotic cell, yeast cell, animal cell, plant cell, insect cell and the like including a recombinant vector with a DNA encoding the polypeptide of the present invention incorporated therein is cultured according to an ordinary culture method, the polypeptide of the present invention is produced and accumulated, and the polypeptide of the present invention is obtained from the culture to produce the polypeptide of the present invention.

As used herein, the term "antibody" is used in its ordinary sense, which is well known to those skilled in the art. As used herein, the term "antibody" encompasses the whole molecule and a fragment thereof, a derivative thereof, a conjugate thereof, and the like. Preferably, antibodies, which are preferably used in the present invention, recognize a polypeptide of the present invention, more preferably in a specific manner. Such antibodies may be polyclonal antibodies or monoclonal antibodies.

The cell or cell composition prepared by using the agent of the present invention can be provided in any formulation format so long as the format is appropriate for introduction into the living organism. Such a formulation format includes for example a liquid agent, an injection agent, a sustained release agent or the like. Administration routes include oral administration, parental administration, direct administration to a diseased site, and the like

Injection drugs can be prepared using techniques well known in the art. For example, an agent of the present invention is dissolved in an appropriate solvent (physiological saline, buffer (e.g., PBS, etc.), sterilized water, etc.), followed by filter sterilization using a filter, or the like. Thereafter, the solution is placed into a sterile container (e.g., an ampoule, or the like). Thus, an injection drug can be prepared. The injection drug may contain a commonly used pharmaceutical carrier if required. An administration method using a non-invasive catheter may be employed.

In one embodiment, an agent of the present invention may be provided in a sustained-release form. When administered in a sustained-release form, an active component (e.g., a nucleic acid or a polypeptide) is effective if efficacy thereof can be expected over a long time due to sustained release. Any sustained-released dosage form may be used in the present invention. Examples of sustained-release dosage forms include, but are not limited to, rod-like formulations (e.g., pellet-like, cylinder-like, needle-like formulations, etc.), tablet formulations, disk-like formulations, sphere-like formulations, sheet-like formulations, and the like. Methods for preparing sustained-release dosage forms are well known in the art, as described in, for example, the Japanese Pharmacopeia, the U.S. Pharmacopeia, Pharmacopeias of other countries, and the like. Examples of a method for producing sustained-release drugs include, but are not limited to, a method using disaggregation of a drug from a complex, a method for preparing an aqueous suspended liquid drug, a method for preparing an oil injection liquid or oil suspended injection liquid, a method for preparing an emulsified injection liquid (o/w or w/o type emulsified injection liquid, or the like), and the like.

As used herein, polypeptide expression may be "detected" or "quantified" by an appropriate method, including mRNA measurement and immunological measurement methods. Examples of the molecular biological measurement methods include a Northern blotting method, a dot blotting method, a PCR method, and the like. Examples of the immunological measurement method include an ELISA method, an RIA method, a fluorescent antibody method, a Western blotting method, an immunohistological staining method, and the like, where a microtiter plate may be used. Examples of a quantification method include an ELISA method, an RIA method, and the like.

As used herein, the term "amount of expression" refers to the amount of a polypeptide or mRNA expressed in a cell of interest or the like. The amount of expression includes the amount of expression at the protein level of a polypeptide of the present invention evaluated by any appropriate method using an antibody of the present invention, including immunological measurement methods (e.g., an ELISA method, a RIA method, a fluorescent antibody method, a Western blotting method, an immunohistological staining method, and the like, or the amount of expression at the mRNA level of a polypeptide of the present invention evaluated by any appropriate method, including molecular biological measurement methods (e.g., a Northern blotting method, a dot blotting method, a PCR method, and the like). The term "change in the amount of expression" indicates an increase or decrease in the amount of expression at the protein or mRNA level of a polypeptide of the present invention evaluated by an appropriate method including the above-described immunological measurement method or molecular biological measurement method.

BEST MODE FOR CARRYING OUT THE INVENTION

In one aspect, the present invention provides a polypeptide having a WIF domain which maintains pluripotency without differentiating a stem cell. As used herein, the "maintenance of undifferentiation (or without or no differentiation)" refers to maintaining an undifferentiated state or not making differentiated. Therefore, no differentiation and maintaining undifferentiated as well as suppressing differentiation are used herein to have the same meaning. Further, no differentiation refers to a state in which each of cells or cell groups in the early developing period of an organism cannot be distinguished from a morphological or functional point of view.

In one embodiment, the polypeptide of the present invention is WIF-1. WIF-1 preferably comprises a sequence set forth in SEQ ID NOs: 2 (murine), 4 (human), 6 (rat), 8 (Xenopus) or 10 (Zebrafish). More preferably, the polypeptide of the present invention comprises a sequence set forth in SEQ ID NO: 4 or a variant sequence thereof. Still more preferably, the polypeptide of the present invention comprises a sequence set forth in SEQ ID NO: 4. WIF-1 is an abbreviation of Wnt inhibitory factor-1, and was reported in 1999 as a novel protein binding to Wnt protein and inhibiting the activity thereof (Hsieh J.H.,et al., Nature 398, 431-436, 1999). Hsieh et al., identified the gene product of WIF-1 and the expression pattern in human, mouse, *Xenopus* and Zebrafish, and proved the *in vitro* inhibitory activity of a Wnt protein. Hsieh et al., further reported that WIF-1 inhibited body segmentation and axis formation activity in *Xenopus.*

Wnt is known to be an agent controlling embryonic body axis formation and organ formation (Cadigan K.M., and Nusse R., Genes & Development 11:3286-3305). Wnt was found in separate studies in mouse (originally called as int-1), insect (Drosophila; originally called wg (wingless)) and later found in other organisms such as *Caenorhabitis elegans,* a wide range of vertebrates including mammals including humans, amphibians and the like. However, Wnt has about 20 kinds of homologs such as Wnt-1, and there are a number of functions for the homologs. Some Wnt are believed to mediate stabilization of beta-catenin in cytoplasm and the gene expression associated thereof, however, there are also other types of Wnt suggested to be involved in cancer formation. As such, the Wnt family as a whole is chaotic with respect to the functions thereof. As a protein interacting with Wnt, Fz (Frizzled) receptor is known, however, a number of functions are reported for the receptor and are not yet clarified.

Thus, WIF-1 was isolated as a factor having Wnt-inhibitory activity, however, the activity thereof has been unknown up to date. As WIF-1 has an EGF-like repeat as apparent from the sequence thereof, in the present invention the differentiation regulation activity of such an EGF-like repeat is noted. In another aspect, it is also noted that the WIF domain corresponds to about position 30 to about 180 of the amino acid sequence of mouse WIF-1 with respect to the differentiation regulation function thereof. In the present invention, it has been clarified that a protein having a WIF-domain optionally having an EGF-like repeat, has a function of expanding a cell while maintaining its pluripotency without differentiation optionally in the presence of an essential factor for survival such as SCF. Accordingly, in a preferable embodiment, the present polypeptide has a WIF domain and an EGF-like repeat. In another embodiment, the present polypeptide may be a different polypeptide having a EGF-like repeat. It has not been reported that a polypeptide having an EGF-like repeat has an activity suppressing differentiation without losing pluripotency of a hematopoietic stem cell. Therefore, by the present invention, it is surprising that a large volume of culture of hematopoietic stem cells, which are clinically applicable, has been achieved. Thus, in a preferable embodiment, a stem cell used in the present invention may be a hematopoietic stem cell.

The following methods may also be considered for cloning a gene of WIF-1. As WIF-1 has been found in a variety of organisms, it is considered that a portion of the amino acid sequence thereof is conserved in the process of evolution of living organisms. Accordingly, a DNA sequence corresponding to a conserved amino acid sequence (for example, a WIF domain or an EGF-like domain or the like) is designed for use as a primer in a RT-PCR (reverse transcription polymerase chain reaction) and proliferation of an equivalent of another living organism corresponding to the polypeptide of the present invention, may be obtained. Accordingly, such an equivalent is also within the scope of the present invention. Further, a sequence obtained by conducting a homology search against genome databases such as the human genome project, or gene information databases such as GenBank or the like using software such as BLAST, is also within the scope of the present invention.

A sequence obtained by screening a cDNA library of an organ using a fragment found in such an homology search as a probe to clone genes having a longer gene sequence, or full-length genes, is also within the scope of the present invention. Such a screening method includes a method using a radioisotope or not. Further, a method without using a library such as 5'-RACE, 3'-RACE or the like may be used. In another embodiment, a stem cell transformed with a vector containing a nucleic acid encoding a polypeptide according to the present invention is provided.

In another aspect, the present invention provides a composition for maintaining a pluripotency of a stem cell with maintaining an undifferentiated state thereof. The present composition includes a polypeptide having a WIF domain. A composition according to the present invention optionally comprises a stem cell survival agent. As used herein, the term "stem cell survival agent" is an agent considered to be essential for survival of a stem cell. Conventionally, the survival (and differentiation) of stem cells has been thought to require SCF. However, Ornitz M.,et al.(Immunity, Vol.10,173-182,1999) discovered a pluripotent hematopoietic stem cell which does not express c-Kit which is a receptor to SCF. Therefore, SCF *per se* is not required for survival of all the stem cells. Further, SCF *per se* has no differentiation activity. Accordingly, a stem cell used herein may be propagated with maintenance of the undifferentiated state thereof and preserving the pluripotency thereof in the presence of a polypeptide having a WIF domain so long as the cell is in the circumstances enabling the survival thereof. Usually, a stem cell requires SCF as described above, preferably a stem cell propagates with maintenance of the undifferentiated state thereof and preserving the pluripotency thereof, in the presence of a polypeptide having a WIF domain (for example, WIF-1) and SCF. Accordingly, in a preferable embodiment, the above-described stem cell survival agent is SCF. In the presence of SCF alone, as shown in the Examples herein, it has been clarified that a stem cell gradually loses the pluripotency thereof although maintains the survival thereof.

In another aspect, the present invention provides a stem cell maintaining an undifferentiated state and having a pluripotency *in vitro.* In a preferable embodiment, the cell of the present invention is a hematopoietic stem cell. Preferably, the stem cell of the present invention having pluripotency with maintenance of the undifferentiated state thereof *in vitro,* preserves its pluripotency for at least three days or at least five days, preferably at least six days, more preferably at least ten days, still more preferably, at least twenty days, thirty days, two months, three months, six months, one year. Naturally, there is a fresh stem cell (for example, a hematopoietic stem cell) as a stem cell without differentiation and such stem cells preserve pluripotency. However, in the absence of an agent of the present invention, such a stem cell immediately differentiates or dies. Therefore, the fact that a stem cell can maintain an undifferentiated state for a long period of time as mentioned above (for example, days to years), is a significant effect which cannot be achieved by the conventional technology. It is also a significant effect that the function of a stem cell just after its isolation from the host thereof can be preserved for a long period of time, which cannot be attained by conventional methods. As used herein, a period for preserving pluripotency refers to a period until the differentiation of the cell is determined for a certain direction. The determination whether or not differentiation has been taken place can be easily conducted by any of a variety of known methods, and such a method includes for example, colony assay. In an embodiment, when the determination is conducted by a colony assay, a stem cell isolated *in vitro* according to the present invention, preserves its pluripotency for at least fourteen days.

In a preferable embodiment, the pluripotency which a cell according to the present invention, comprises includes the capability of differentiation into the blood cells.

In another aspect, the present invention provides a long period pluripotency maintaining cell composition. As used herein the term "long period pluripotency maintaining cell composition" refers to a composition comprising a cell which is preferably a stem cell, and holds its pluripotency and does not differentiate for a long period of time (for example, at least three days, preferably at least five days, more preferably, at least ten days, still more preferably at least twenty days, thirty days, two months, three months, six months, and one year). The long period pluripotency maintaining cell composition comprises a stem cell and a polypeptide having a WIF domain.

In one embodiment of the present invention, the above-mentioned stem cell may be a hematopoietic stem cell. In another embodiment, the above-mentioned stem cell may be another tissue stem cell. In another embodiment, the above-mentioned stem cell may be an embryonic stem cell.

Preferably, in the present composition, a stem cell is present at least at $10^3$ cells, preferably at least at 2 x $10^3$ cells, at least at 5 x $10^3$ cells, at least at $10^4$ cells, at least at 2 x $10^4$ cells, at least at 5 x $10^5$ cells, at least at 2 x $10^5$ cells, at least at 5 x $10^5$ cells, at least at $10^6$ cells, at least at 2 x $10^6$ cells, at least at 5 x $10^6$ cells, at least at $10^7$ cells, at least at 2 x $10^7$ cells, at least at 5 x $10^7$ cells, at least at $10^8$ cells, at least at 2 x $10^8$ cells, at least at 5 x $10^8$

cells, at least at $10^9$ cells, at least at 2 x $10^9$ cells, at least at 5 x $10^9$ cells, at least at $10^{10}$ cells, or more than at least at $10^{10}$ cells. An appropriate amount will vary depending on the circumstances used, and those skilled in the art can appropriately determine such an amount depending on the condition of the agent used present in the composition used, and when used as a medicament, the condition of a patient, the condition of the diseases of the patient, the route of administration, the form of administration and the like.

In a preferable embodiment, the above-mentioned polypeptide having a WIF domain may be WIF-1 (for example, comprising a sequence set forth in SEQ ID Nos: 2, 4, 6, 8 or 10). In a different preferable embodiment, the polypeptide of the present invention may be a polypeptide having an EGF-like repeat and such a polypeptide includes a member of the notch family protein (such as notch-1, notch-2, jagged-1, D11-1, DLK or the like). The polypeptide of the present invention or the polypeptide used in the present invention may be a polypeptide of the natural form, and may be a recombinantly produced polypeptide, a synthetic polypeptide or a variant thereof so long as it is functionally equivalent thereto. The polypeptide of the present invention may be provided to a stem cell in a form of a nucleic acid. In this case, the stem cell may be transformed with a nucleic acid molecule containing a nucleic acid sequence encoding the polypeptide of the present invention. Methods for transformation are well known in the art and described in detail elsewhere in the present specification. Accordingly, those skilled in the art can conduct such transformations according to the teaching of the present specification.

In a preferable embodiment, the above-described polypeptide can be present at least at 0.1 ng/ml. More preferably, the polypeptide may be present at least at 1.0 ng/ml, at least at 2.0 ng/ml, at least at 5.0 ng/ml, at least at 20.0 ng/ml, at least at 50.0 ng/ml, at least at 100.0 ng/ml, at least at 200.0 ng/ml, at least at 500.0 ng/ml, at least at 1.0 μg/ml, at least at 2.0 μg/ml, at least at 10.0 μg/ml, at least at 100.0 μg/ml or at least more than at 1 mg/ml.

In a preferable embodiment, the composition of the present invention further comprises a stem cell survival agent. Preferably, the stem cell survival agent may be SCF. The activity for maintaining pluripotency with an undifferentiated state is augmented by the coexistence of such a stem cell survival agent in addition to the polypeptide of the present invention.

In a preferable embodiment, the above-described stem cell survival agent (for example, SCF) may be present at least at 0.1 ng/ml. More preferably, the stem cell survival agent (for example, SCF) may be present at least at 1.0 ng/ml, at least at 2.0 ng/ml, at least at 5.0 ng/ml, at least at 20.0 ng/ml, at least at 50.0 ng/ml, at least 100.0 ng/ml, at least 200.0 ng/ml, at least t 500.0 ng/ml, at least at 1.0 μg/ml, at least at 2.0 μg/ml, at least at 10.0 μg/ml, at least at 100.0 μg/ml or at least at more than 1 mg/ml.

In another preferable embodiment, the stem cell survival agent may be the TPO. The activity for maintaining pluripotency with an undifferentiated state may be augmented by the coexistence of the TPO in addition to the polypeptide of the present invention. The TPO may augment the activity of maintaining pluripotency with undifferentiated state by the addition thereof in addition to the SCF and/or FL.

In a preferable embodiment, TPO may be present at least at 1.0 ng/ml, at least at 2.0 ng /ml, at least at 5.0 ng /ml, at least at 20.0 ng /ml, at least at 50.0 ng/ml, at least at 100.0 ng/ml, at least at 200.0 ng/ml, at least at 500.0 ng/ml, at least at 1.0 μg/ml, at least at 2.0 μg/ml, at least at 10.0 μg/ml, at least at 100.0 μg/ml or at least more than at 1 mg/ml.

In another preferable embodiment, the stem cell survival agent may be flt-3 ligand (FL). More preferably, the activity for maintaining pluripotency with an undifferentiated state is augmented by the coexistence of a plurality of stem cell survival agents (for example, at least two, preferably three of SCF, TPO and FL and the like) in addition to the polypeptide of the present invention.

In a preferable embodiment, FL may be present at least at 0.1 ng/ml. More preferably, FL may be present 1.0 ng/ml, at least at 2.0 ng/ml, at least at 5.0 ng/ml, at least at 20.0 ng/ml, at least at 50.0 ng/ml, at least at 100.0 ng/ml, at least at 200.0 ng/ml, at least at 500.0 ng/ml, at least at 1.0 μg/ml, at least at 2.0 μg/ml, at least at 10.0 μg/ml, at least at 100.0 μg/ml or at least at more than at 1 mg/ml.

In a preferable embodiment, the present invention provides a composition for preparing differentiated cells. The differentiated cells may be used for treating disorders of the blood cells. A method for preparing differentiated cells from a stem cell is well known in the art, and includes for example, a method for adding a hematopoietic factor and the like.

In another aspect, the present invention provides a method for maintaining pluripotency without differentiating a stem cell. The method comprises 1) providing the stem cell with a polypeptide having a WIF domain.

In an embodiment of a method for maintaining pluripotency of a stem cell of the present invention, the above-described specific elements to be used in preferable embodiments of the above-described composition, may be the specific elements of the method of the present invention.

In one embodiment of the present invention, the above-described polypeptide having a WIF domain (for example, WIF-1 such as those polypeptides comprising a sequence set forth in SEQ ID NO: 2, 4, 6, 8 or 10) may be provided in the form of a polypeptide. In another embodiment, the polypeptide may be provided in the form of a nucleic acid. In the case where provided in the form of a nucleic acid, the method of the present invention further comprises the step of transforming the stem cell with a nucleic acid comprising a nucleic acid sequence encoding the polypeptide.

In another embodiment of the present invention the method of the present invention further comprises the step of providing a stem cell survival agent (for example, SCF) to the stem cell. The stem cell survival agent may be provided before, after or at the same time of the provision of the polypeptide having a WIF domain. The stem cell survival agent may be directly provided in the form of polypeptide. In another embodiment, the stem cell survival agent may be provided in the form of a nucleic acid. In the case where provided in the form of a nucleic acid, the method of the present invention further comprises the step of transforming the stem cell with a nucleic acid molecule comprising a nucleic acid sequence encoding the stem cell survival agent.

In another aspect, the present invention provides a method for producing a long period pluripotency maintaining cell composition. The present method comprises 1) providing a stem cell; 2) providing the stem cell with a polypeptide having a WIF domain; and 3) collecting the stem cell treated.

In embodiments of the method for producing a long period pluripotency maintaining cell composition, the above-described specific elements to be used in preferable embodiments of the above-described composition, may be the specific elements of the method of the present invention.

In an embodiment of the present invention, the polypeptides having a WIF domain (for example, WIF-1) may be directly provided in the form of a polypeptide. In another embodiment, the polypeptide may be in the form of a nucleic acid. In the case where provided in the form of a nucleic acid, the production method of the present invention further comprises the step of transforming the stem cell comprising a nucleic acid molecule comprising a nucleic acid sequence encoding the polypeptide.

In another embodiment of the present invention, the production method of the present invention further comprises the step of providing a stem cell survival agent (for example, SCF) to the stem cell. The stem cell survival agent is provided before, after or at the same time as the provision of the polypeptide having a WIF domain. The stem cell survival agent may be directly provided in the form of a polypeptide. In another embodiment, the stem cell survival agent may be provided in the form of a nucleic acid. In the case where provided in the form of a nucleic acid, the production method of the present invention further comprises the step of transforming the stem cell with a nucleic acid molecule comprising a nucleic acid sequence encoding the stem cell survival agent. The technology such as transformation and the like are well known in the art and described elsewhere herein.

In another aspect, the present invention provides a method for producing differentiated cells in a large scale. The method comprises the steps of 1) providing a stem cell; 2) providing a polypeptide having a WIF domain to the stem cell; 3) collecting the thus treated stem cells; and 4) providing a differentiation factor to the stem cell.

In an embodiment of a method for producing differentiated cells in a large scale of the present invention, the above-described specific elements to be used in preferable embodiments of the above-described composition, may be the specific elements of the method of the present invention.

In another embodiment, the polypeptide having a WIF domain (for example, WIF-1) may be directly provided in the form of polypeptide. In another embodiment, the polypeptide may be provided in the form of a nucleic acid. The differentiation factor may also be provided in the form of polypeptide or nucleic acid. In the case where provided in the form of nucleic acid, a vector construct may be constructed so that the differentiation factor is expressed later than the polypeptide of the present invention and the stem cell may be transformed with the construct. A method for producing and transforming such a vector construct is well known in the art and those skilled in the art can readily carry out such a method based on the teachings of the present application.

In another embodiment of the present invention, the production method of the present invention further comprises the step of providing the stem cell with a stem cell survival agent (for example, SCF). The stem cell survival agent may be provided before, after or at the same time of the provision of the polypeptide having a WIF domain. The stem cell survival agent may be directly provided in the form of polypeptide. In another embodiment, the stem cell survival agent may be provided in the form of a nucleic acid. In the case where provided in the form of a nucleic acid, the production method of the present invention further comprises the step of transforming the stem cell with a nucleic acid molecule comprising a nucleic acid sequence encoding the stem cell survival agent. Technology such as transformation is well known in the art and described elsewhere herein. In one embodiment, nucleic acid sequences encoding the present polypeptide and the stem cell survival agent may be engineered so as to reduce or stop the expression after a certain period of time. In such a case, a differentiation factor may be engineered to be expressed after a certain period of time.

In another aspect, the present invention provides a method for treating a disease or disorder derived from a disorder of differentiated cells. The method comprises the steps of 1) administering a long period pluripotency maintaining cell composition comprising a stem cell and a polypeptide having a WIF domain. The administration method may be any method well known in the art. Administration methods may herein include oral administration and parenteral administration (e.g., intravenous, intramuscular, subcutaneous, intradermal, mucosal, intrarectal, intravaginal, topical to an affected site, to the skin, etc.), direct administration to affected portions, and the like. A prescription for such administration may be provided in any formulation form. Such formulation forms include liquid formulations, injections, sustained preparations, and the like. Preferably, the polypeptide having a WIF domain may include a sequence set forth in SEQ ID NO: 4 or a variant sequence thereof, and preferably may be human recombinant WIF-1.

In an embodiment of a method for treating a disease or disorder derived from the disorder of a differentiated cell of the present invention, the above-described features in preferable embodiments for the above-described composition are also applicable for the specific features of the method of the present invention.

In another aspect, the present invention provides a method for treating a disease or disorder derived from a disorder of a differentiated cell. The method comprises a step of 1) administering a differentiated cell prepared from a stem cell processed with a polypeptide of the present invention, to a subject. Any well known method for administration in the art may be used for this method. Administration methods include: oral administration, parenteral administration (such as intravenous administration, intramuscular administration, subcutaneous administration, intraderal administration, mucous administration, intrarectal administration, intravaginal administration, direct administration to a diseased site, skill administration etc). A formulation for such an administration may be provided in any formulation form. Such formulation formats include for example, liquid agents, injections, sustained-release agents and the like.

In an embodiment of a method for treating a disease or disorder derived from a disorder of a differentiated cell (for example, the blood cells) of the present invention, the above-described specific elements to be used in preferable embodiments of the above-described composition, may be the specific elements of the method of the present invention.

The method for treatment of the present invention can be used for any living organism such as vertebrates or invertebrates so long as the living organism can be subject to a disease or a disorder derived from a differentiated cell. In a preferable embodiment, such a living organism may be vertebrate, more preferably, such a living organism may be a mammal (for example, rodent and the like), and still more preferably may be primates, and most preferably may be a human.

Preparation protocols for preparing such a formulation are known in the art and described in for example, Japanese Pharmacopoeia, U.S. Pharmacopoeia, and pharmacopoeia of the other countries, and the like. Accordingly, based on the description of the present specification, those skilled in the art can determine the amounts of polypeptides and cells to be administered without undue experimentation.

Injection drugs can be prepared using techniques well known in the art. For example, an agent of the present invention is dissolved in an appropriate solvent (physiological saline, buffer (e.g., PBS, etc.), sterilized water, etc.), followed by filter sterilization using a filter, or the like. Thereafter, the solution is placed into a sterile container (e.g., an ampoule, or the like). Thus, an injection drug can be prepared.

The diseases or disorders which can be treated by the present invention may be those related to disorders of differentiated cells into which the stem cells used in the present invention can differentiate. In a preferable embodiment, such a differentiated cell may be blood cells. Examples of the diseases or disorders include, but are not limited to, anemia (e.g., aplastic anemia (particularly, severe aplastic anemia), renal anemia, cancerous anemia, secondary anemia, refractory anemia, etc.), cancer or tumors (e.g., leukemia); and after chemotherapy therefor, hematopoietic failure, thrombocytopenia, acute myelocytic leukemia (particularly, a first remission (high-risk group), a second remission and thereafter), acute lymphocytic leukemia (particularly, a first remission, a second remission and thereafter), chronic myelocytic leukemia (particularly, chronic period, transmigration period), malignant lymphoma (particularly, a first remission (high-risk group), a second remission and thereafter), multiple myeloma (particularly, an early period after the onset), neutropenia and the like. In a preferable embodiment, the diseases or disorders, which can be treated according to the present invention, may be human diseases or disorders.

By the stem cells of the present invention, a number of side effects associated with conventional hematopoietic cell transplantation therapy derived from natural sources (particularly, those associated with foreign substances or foreign cells, such as infection, graft-versus-host disease, and the like) are avoided. This effect is first attained by the present invention in which a technology for maintaining pluripotency of a cell with maintaining an undifferentiated state, and therefore should be noted to be surprising. Further, by maintaining pluripotency of stem cells with an undifferentiated state for a long period of time, it is now possible to produce pluripotent stem cells in a largescale. In addition, it is also now possible to prepare a large number of differentiated cells from such a large number of stem cells cultured in a large scale. Such effects cannot be attained by the conventional methods, and should be noted to be significant.

In another embodiment, the treatment method of the present invention may further comprise a step of administering another drug. Such another drug may be any drug known in the art, and formulations thereof include for example, any formulation known in the pharmaceutical field including antibiotics. Naturally, such drugs may include two or more other drugs. Such drugs include for example those listed in Japanese pharmacopoeia the latest version, the United States pharmacopoeia the latest version, or other country's pharmacopoeia the latest version or the like. Such drugs may preferably be those having effects on the hematopoietic system.

In other embodiment, the above-mentioned cells may include two or more kinds of cells. When two or more kinds of cells are used, cells having identical or similar properties or origin, or different properties or origin may be used.

The amount of an agent or cell used in the method of the present invention can be easily determined by those skilled in the art with reference to the purpose of use, the subject's age, size, sex, and case history, the form or type of agent or cell, and the like.

The frequency of the method of the present invention which is applied to a subject is also determined by those skilled in the art with respect to the purpose of use, the subject's age, size, sex, case history, the progression of the therapy, and the like. Examples of the frequency include once per day to once per several months (e.g., once per week to once per month). Preferably, administration is performed once per week to once per month with reference to the progression.

In another aspect, the present invention provides a pharmaceutical composition for treating a disease or disorder derived from disorders of differentiated cells. The pharmaceutical composition includes a stem cell; a polypeptide having a WIF domain; and a pharmaceutically acceptable carrier. Preferably, the polypeptide having a WIF-1 domain may include the sequence set forth in SEQ ID NO: 4 or a variant sequence thereof, and may be human recombinant WIF-1.

In one embodiment, the object disease or disorder of the pharmaceutical composition may be any one described herein above.

In a variation of the embodiments of a pharmaceutical composition of the present invention, any specific elements to be applied in the preferable embodiments of the above-mentioned composition may be applied to the specific elements of the pharmaceutical composition of the present invention.

In another aspect, the present invention provides a pharmaceutical composition for treating a disease or disorder derived from a disorder of differentiated cells. The pharmaceutical composition comprises a differentiated cell prepared from a stem cell processed using a polypeptide of the present invention; and a pharmaceutically acceptable carrier.

In one embodiment, the objects of the present pharmaceutical composition are the same as described herein above.

In a variation of the embodiments of the present pharmaceutical composition, the particular elements in a number of preferable embodiments of the present composition can be applied to specific elements to be included in the pharmaceutical composition of the present invention.

Pharmaceutically acceptable carriers included in the pharmaceutical composition of the present invention include any material known in the art. Examples of such an appropriate materials or pharmaceutical acceptable agents include, but are not limited to, antioxidants, preservatives, colorants, flavoring agents, diluents, emulsifiers, suspending agents, solvents, fillers, bulking agents, buffers, delivery vehicles, and/or pharmaceutical adjuvants. Representatively, a medicament of the present invention is administered in the form of a composition comprising an active component of the present invention (e.g., a polypeptide, a nucleic acid, or the like) with at least one physiologically acceptable carrier, excipient or diluent. For example, an appropriate vehicle may be an injection solution, a physiological solution, or artificial cerebrospinal fluid, which can be supplemented with other substances which are commonly used for compositions for parenteral delivery.

Examples of appropriate carriers include neutral buffered saline or saline mixed with serum albumin. Preferably, the product is formulated as a lyophilizate using appropriate excipients (e.g., sucrose). Other standard carriers, diluents, and excipients may be included as desired. Other exemplary compositions comprise Tris buffer of about pH 7.0-8.5, or acetate buffer of about pH 4.0-5.5, which may further include sorbitol or a suitable substitute therefor. The pH of the solution or various other pH's should be selected based on the relative solubility of a polypeptide of the present invention.

Solvents for use in preparing a composition may be either aqueous or nonaqueous. Vehicles for the compositions may contain other components for modifying or maintaining pH, osmolarity, viscosity, transparency, color, sterility, stability, isotonicity, disintegration rate, or smell of the formulation. Similarly, a composition of the present invention may contain other components for modifying or maintaining the release rate of an effective component or absorption or permeability of an effective component.

A composition of the present invention may be parenterally administrated. The composition may be intravenously or subcutaneously administrated. When administrated systemically, the therapeutic composition for use in the present invention may be in a form of an aqueous solution which is orally acceptable and pyrogen-free. The preparation of such pharmaceutically acceptable compositions, with due regard to pH, isotonicity, stability and the like, is within the skill of the art.

The therapeutic formulation of the present invention may be prepared for storage by mixing a selected composition having the desired degree of purity with optional physiologically acceptable carriers, excipients, or stabilizers (Japanese Pharmacopeia; Remington's Pharmaceutical Sciences, 18th Edition, A. R. Gennaro, ed., Mack Publishing Company, 1990; and the like), in the form of lyophilized cake or aqueous solutions.

Acceptable carriers, excipients or stabilizers used herein preferably are nontoxic to recipients and are preferably inert at the dosages and concentrations employed, and preferably include phosphate, citrate, or other organic acids; antioxidants (e.g., ascorbic acid); low molecular weight polypeptides; proteins (e.g., serum albumin, gelatin, or immunoglobulins); hydrophilic polymers (e.g., polyvinylpyrrolidone); amino acids (e.g., glycine, glutamine, asparagine, arginine or lysine); monosaccharides, disaccharides, and other carbohydrates (glucose, mannose, or dextrins); chelating agents (e.g., EDTA); sugar alcohols (e.g., mannitol or sorbitol); salt-forming counterions (e.g., sodium); and/or nonionic surfactants (e.g., Tween, pluronics or polyethylene glycol (PEG)).

Cells, compositions or the like of the present invention can be used not only just after the preparation thereof, but also after storage for a certain period of time such as about one day, about two days, about three days, about four days, about five days, about six days, about seven days, about two weeks, about three weeks, about four weeks, about two months, about three months, about four months, about five months, about six months, about one year or the like, under for example, about 4 degree Celcius, about -18 degree Celcius, about -70 degree Celcius, or under liquid nitrogen or the like.

Hereinafter, the present invention will be described by way of examples. Examples described below are provided only for illustrative purposes. Accordingly, the scope of the present invention is not limited except as by the appended claims.

EXAMPLES

(Example 1: Recombinant WIF-1 preparation) 1.

PA-6 cell line was kindly provided from Dr. Hiroshi KODAMA at Ôu University and was cultured in Minumum Essential Medium Alpha Medium ($\alpha$-MEM) (Sigma, St. Louis, USA) with 10 % fetal calf serum added at 37 °C under 5% $CO_2$ until confluent.

PA-6 cells were collected and the mRNA thereof was isolated using a QuickPrep mRNA purification kit (Amersham Pharmacia Biotech, Uppsala, Sweden) and thus cDNA was synthesized using SuperScript® RnaseH-reverse transcriptase (Invtrogen, Groningen, The Netherlands) for use in degenerate PCR.

From the conservative sequences of EGF-like repeats of the Notch family, degenerate primers of SEQ ID Nos: 11 and 12 were synthesized and a partial sequence of the WIF-1 gene was isolated under the following conditions:

a) Reaction Mixture

Primers (SEQ ID Nos: 11 and 12) 10 $\mu$M each; cDNA 1,500 ng/ml; dNTPs 0.25mM each; polymerase, 0.5 U/ml (TakaraTaq, TaKaRa, Shiga, Japan); buffer: 0.15m M $MgCl_2$, 10mM Tris-HCl (pH 8.3), 50 mM KCl.

b) Reaction cycle

(i) 94 °C, 60 seconds, 1 cycle;
(ii) 94 °C, 20 seconds; 60 °C, 20 seconds; 72 °C, 20 seconds; 30 cycles;
(iii) 72 °C, three minutes, 1 cycle.

After gel electrophoresis of the reaction products, a nucleic acid fragment having about 180 bp was collected and sequenced to confirm that the fragment was a fragment derived from the WIF-1 gene.

This fragment was used as a hybridization probe to isolate a full-length clone of the WIF-1 from Mouse Day15 Embryo 5'-Stretch Plus cDNA Library (Clonetech, Palo Alto, California, USA). The sequences thereof are shown in SEQ ID NO: 1 (nucleic acid) and SEQ ID NO:2 (amino acid sequence).

(2. Expression and Purification of Recombinant WIF-1)

The full-length clone of the WIF-1 as isolated in 1. above, as digested with restriction enzymes XhoI and NheI and the resultant fragment was ligated to the pCAGGS-6His vector, in which a 6-His sequence having restriction sites in the pCAGGS vector provided from Dr. Jun-ichi MIYAZAKI, Kumamoto University, was inserted, (SEQ ID NO: 13), and this vector was designated as pCAGGS/mWIF-1-6His.

The pCAGGS/mWIF-1-6His was transduced into COS7 cells by the calcium phosphate method, and the cells were cultured in DMEM (Dulbecco's Modified Eagle Medium) supplemented with 10 % FCS, under 5% $CO_2$, at 37 °C until confluent.

A recombinant WIF-1 was purified from the transduced COS7 cells using the following methods. The culture supernatant was passed through a Nickel-column (His Trap, Amersham Pharmacia Biotech), and thus mWIF-1 was bound to the column. In order to remove nonspecific bound proteins, the column was washed well with phohsphate buffer with 100 mM imidazole added, and then mWIF-1-6His was eluted with a phosphate buffer with 300 mM imidazole added. The eluant included imidazole which is cytotoxic, and thus the buffer was replaced with PBS using gel chromatography (PD-10 column, Amersham Pharmacia Biotech). The resultant purified protein was subjected to electrophoresis using SDS-PAGE and silver-stained to confirm the degree of purification.

(Example 2: Separation of $CD34^-$ KSL cells using FACS)

It is known that with respect to hematopoietic cells in bone marrow, hematopoietic stem cells with high purity can be separated by the use of separation of CD34 negative or weak negative, c-Kit positive, Sca-1 positive, differentiation antigen (Lin) negative cells ($CD34^-$ KSL cells) (Osawa, M. et al., Science, 273: 242-245,1996). In this example, the following method using FACS was applied to separate $CD34^-$KSL cells. The scheme therefor is shown in Figure 2.

(1. Preparation of bone marrow cell suspension)

The femur and tibia were removed from the lower thigh of B6 mice of about eight to ten weeks age. The muscle and fat tissues and the like were removed to separate bone only. Both ends of a tubular bone was subjected to resection and a syringe with a needle connected thereto was used to inject PBS (phosphate buffered saline) from one of the ends to obtain the contents of the bone (bone marrow). The bone marrow was suspended in PBS (phosphate buffered saline) and passed through a nylon filter (70 µm) to remove cell aggregates and tissues such as contaminated muscles. The cell number in the cell suspension, which was filtered by the nylon filter, was counted (usually, 4-5 x $10^7$ cells were obtained), and the cell concentration was controlled to about 1 x $10^7$ cells/ml by adding PBS. About 5 ml of the cell suspension was overlayed to 5ml Ficoll (Lymphoprep, Nycomed (Oslo, Norway)), centrifuged at room temperature, 400xg for 20 minutes to remove erythrocytes and neutrophils. After centrifugation, cells in the boundary surface were collected and washed twice with staining medium (SM; PBS containing 5% bovine fetal serum and 0.05 % $NaN_3$) and the cell number was counted. Usually, about half of the cells before the centrifugation were recovered.

Biotinylated lineage antibodies (0.01-0.1 µg/ml of anti-Mac-1 antibody(clone: M1/70) as a final concentration, 0.01-0.1 µg/ml of anti Gr-1 antibody (clone: RB6-8C5) as a final concentration, 0.01 - 0.1 µg/ml of anti B220 antibody (clone: RA3-6B2) as a final concentration, 0.01 - 0.1 µg/ml of anti CD4 antibody (clone: RM4-5) as a final concentration, 0.01 - 0.1 µg/ml of anti CD8 antibody (clone: 53-6.7) as a final concentration, 0.01 - 0.1 µg/ml of anti erythrocyte antibody (clone: anti erythrocyte antibody TER 119); all available from PharMingen (San Diego, USA)) were added to a cell suspension of about 1 - 10 x $10^7$ cell/ml of the cells and the suspension was subjected to reaction on ice for thirty minutes. The suspension was washed twice with SM, and was resuspended in 100 µl of SM.

In order to purify the cells resuspended in the SM, Dynabeads were used and were pretreated as followed: streptavidin treated Dynabeads (Dynabeads M-280:Dynal; Oslo, Norway) were added so as the concentration thereof became 5-6 beads/cell, and washed with SM using a magnetic stand. After washing, the Dynabeads were resuspended in 10 ml of SM.

The cells resuspended in SM and the pretreated Dynabeads were reacted on ice for thirty minutes. After the addition of 7 ml of SM, a magnetic stand was used to remove Linage positive cells (cells bound to biotinylated lineage antibodies) bound to Dynabeads from the cells were resuspended in SM, and the cell number was counted. Usually, about 5-10 % of the cells before removal remained.

After centrifugation (4 x, 400xg, five minutes), the following was added to the resultant cell pellets:

1) biotinylated lineage antibodies (in the amount of 5-10 % as the amount in which it was used for the first time)
2) 0.01-0.1 µg/ml of phycoerythrin (PE) labeled anti-Sca-1 antibody (clone: E13-161.7) as a final concentration;
3) 0.01-0.1 µg/ml of allophycocyanin (APC) labeled anti c-Kit antibody (clone: ACK2 or 2B8) as a final concentration;
4) 0.01-0.1 µg/ml of flurorescein isothiocyanate (FITC) labeled anti CD34 antibody (clone: RAM34 antibody) as a final concentration;

(2) to (4) were added simultaneously.

After incubation for about thirty minutes on ice, cells were washed and 0.01-0.1 mg/ml of streptavidin - Texas Red was added. After incubation for about thirty minutes on ice, the cells were washed and the cell concentration of the cell suspension was adjusted to about 5 x $10^5$ cells/ml using SM.

FACS Vantage (Becton Dickinson) was used for analysis. Lineage antigen negative cells were selected for Texas Red as indicator thereof (Figure 3A), and then KSL cells were selected by developing with Sca-1 and c-Kit for APC and PE as indicators thereof (Figures 3B), and then less than 10 % of CD negative or CD34 weak negative cells were selected for FITC as an indicator (Figure 3C).

Selected $CD34^-$KSL cells were sorted and used as hematopoietic stem cells.

(Example 3: Human hematopoietic stem cell preparation)

In principle, human hematopoietic stem cells can also be prepared using the same protocols as those used for mice. Accordingly, hematopoietic stem cells can be also separated from the equivalent of anti-Sca-1 antibodies for human as described above.

In Example 3, different experimental methods have been used for demonstrating the utility of the present invention. In human, heterogenous bone marrow transplantation system such as NOD/SCID mice, sheep or the like have to be used for hematopoietic stem cell assays. Human hematopoietic stem cells detected using this method are considered to be substantially equal to the mouse hematopoietic stem cells assayed using the systems of homogenous bone marrow transplantation system. Thus, such a system was used for the demonstration of the present invention. The following is another method for separation of human hematopoietic stem cells.

1) After obtaining informed consent, bone marrow cells or umbilical cord blood, which can be obtained at delivery, from adults (from 18 years old to about 30 years old male and female) were collected in a heparinized syringe. The bone marrow solution or the umbilical cord blood was diluted three times with PBS. About 35ml of the cell suspension was overlaid onto about 15ml of Ficoll (Lymphoprep, Nycomed; Oslo, Norway) and centrifuged for thirty minutes at room temperature at 400xg to remove erythrocytes and neutrophils. After centrifugation, cells in the boundary surface were collected and washed twice with SM and the cell number was counted.

2) Biotinylated lineage antibodies (0.01-0.1 μg/ml of anti-CD2, anti-CD7, anti-CD14, anti-CD16, anti-CD19, antiCD24, anti-CD56, anti-CD66b and anti-Glycophorin A antibodies, respectively, as a final concentration) were added to a cell suspension of about 1 - 10 x $10^7$ cell/ml of the cells and the suspension was subjected to reaction on ice for thirty minutes. The suspension was washed twice with SM, and was resuspended in SM.

3) As in the mouse example, magnetic beads were used to remove lineage marker positive cells. After centrifugation, staining was conducted with 0.01 - 0.1 μg/ml of fluorescein isothiocyanate (FITC) labeled anti-human CD34 antibody (Becon Dickinson) as a final concentration, 0.01 - 0.1 μg/ml of PE labeled anti-human c-Kit antibody as a final concentration, 0.01 - 0.1 μg/ml of APC labeled anti-human CD34 antibody (Beckton Dickinson) as a final concentration. Further, lineage antibodies were again used for restaining.

4) After incubation for about thirty minutes on ice, the cells were washed and 0.01-0.1 mg/ml of streptavidin-Texas Red was added thereto. After the incubation for about thirty minutes on ice, the cells were washed and the cell concentration of the cell suspension was adjusted to 5 x $10^5$ cells/ml using SM.

FACS Vantage (Becton Dickinson) was used for analysis. Lineage antigen negative and CD negative fractions were selectively developed with CD34 and c-Kit. Human hematopoietic stem cells are mainly concentrated in the CD34+Kit+ fractions, however, it recently became apparent that CD34- fractions also include such stem cells as in mouse. Therefore, two kinds, which are CD34+CD38-KL cells and CD34-CD38-KL cells, are subjected to sorting.

(Example 4: Effect of WIF-1 on expansion and differentiation of hematopoietic stem cells)

In order to study the effects of a variety of proteins on expansion and differentiation of hematopoietic stem cells, CD34- KSL cells were used as hematopoietic stem cells, and the expansion (capability of cell division as an indicator thereof) and differentiation (capability of colony formation consisting of matured blood cells as an indicator thereof) were studied.

(1. Method for measuring capabilities of cell division and colony formation of single $CP34^-$ KSL cells)

Round bottom 96-well plates (Corning Inc., New York, USA) were used for culture. 200 μl of Stem-Pro-34 SFM (Life Technologies) containing $5x10^{-5}$M 2-betamercapto ethanol and 2 mM L-glutamine were added to the wells. Added soluble agents were as follows: 1.0ng/ml WIF-1 (as prepared in Example 1), 100 ng/ml mouse SCF, 100 ng/ml human TPO, 10 ng/ml mouse IL-3, 100 ng/ml human IL-6 (PeproTech, London, England), 100 ng/ml human IL-11 (PeproTech, London, England), and 10 ng/ml human GCS-F. SCF, TPO, IL-3 and G-CSF were available from Kirin Brewery Co. Ltd. (Takasaki, Japan). The cells were cultured at 5 % $CO_2$, humidified at 37 °C. Cell number per well was monitored until seven days after culture, and the colony formation was determined on day fourteen of culture.

In the presence of a variety of predetermined proteins, single cell culture was conducted for fourteen days, and cell which underwent at least one cell division, were used as cell-division positive cells. The cells, which underwent a colony formation composed of at least 500,000 cells, were used as a colony formation cell.

(2. The effects of recombinant WIF-1 proteins only on the division and colony formation of $CD34^-$KSL cells)

It was studied whether or not WIF-1 alone induces cell division and/or colony formation of $CD34^-$KSL cells.

It was known that SCF induces cell division of $CD34^-$KSL cells to some extent but fails to induce colony formation thereof (Ema et al., J. Exp. Med. vol 192, Number 9, November 6, 2000). Therefore, SCF was used as a control.

According to the method described above in 1., a number of proteins were added to study the effects of the SCF and WIF-1 on the capability of cell division and colony formation of a single $CD34^-$KSL cell. The results thereof are shown in Table 1.

Table 1.

| The effects of WIF-1 on the capability of cell division and colony formation of the $CD34^-KSL$ cell | | |
|---|---|---|
| Agent(s) | Cell Division | Colony Formation |
| none | 0/96 | 0/96 |
| SCF | 10/96(10.4%) | 0/96 |
| WIF-1 | 0/96 | 0/96 |
| SCF+WIF-1 | 23/96(24.0%) | 0/96 |
| SCF: Stem cell factor | | |

As shown in Table 1, WIF-1 alone did not divide the single $CD34^-KSL$ cell into more than one cell or form colonies. In combination with the SCF, the WIF-1 significantly increased the ratio of cells in which more than one cell existed after cell division (i.e. the ratio of cell division). However, even in the presence of SCF+WIF-1, no colony formation has been observed.

(2. The effects of WIF-1 protein on cell division and secondary colony formation of $CD34^-KSL$ cells in the case of combined with another protein)

In the case where the SCF and FL were combined, effects of the WIF-1 protein on the cell division of the $CD34^-KSL$ cells were studied and it was also studied whether or not colony forming cells remain in the divided cells after fourteen days of culture. In the case where a living colony forming cell was observed, it was determined to be secondary colony positive. The results thereof are shown in Table 2.

Table 2.

| The Effects of WIF-1 on cell division and secondary colony formation of the $CD34^-KSL$ cells in combination of the SCF and/or FL | | |
|---|---|---|
| Agent(s) | Cell Division | Secondary Colony Formation |
| SCF | 5/48 (10.4%) | 0/5 |
| SCF+WIF-1 | 8/48 (16.7%) | 5/7 |
| SCF+FL | 11/48 (22.9%) | 8/9 |
| SCF+WIF-1+FL | 22/48 (45.8%) | 8/8 |
| SCF : Stem cell factor; FL :Flt-3 ligand | | |

The results of Table 2 show that in combination with SCF, WIF-1 supports cell division of the $CD34^-KSL$ cells which are hematopoietic stem cells, and that immature cells having colony formation capability in the divided cells survived at least fourteen days of culture. Accordingly, it has become apparent that WIF-1 induces slight propagation of a hematopoietic stem cell in combination with SCF, the slightly divided cells do have surviving cells, and the surviving cells are not induced for differentiation. Further, it has become apparent that WIF-1 supports the survival of immature cells in a more potent manner when used in combination with SCF and FL.

(Example 5: The effects of WIF-1 for maintaining hematopoietic stem cells which can regenerate *in vivo*)

The isolated hematopoietic stem cells are regenerated into the hematopoietic system by the proliferation and differentiation capability thereof upon transplantation into the living organism. Therefore, CD34-KSL cell as an isolated hematopoietic stem cell was cultured and it was studied whether or not the cultured cells have the capability of renenerating the hematopoietic system in the body after the culture, i.e., maintaining hematopoietic stem cells after culture. WIF-1 was added to the culture solution, and the direct effects of WIF-1 on the maintenance of hematopoietic stem cells were studied.

In order to compare and quantitate the capabilities of propagation and differentiation of the hematopoietic stem cells, a competitive repopulation assay was used and the method thereof is described below.

(1. Competitive repopulating assay)

When hematopoietic stem cells are transplanted into mouse which received a fatal dose of radiation, recipient mouse hematopoietic system is reconstructed for a long period of time by the capability of self-regeneration and pluripotency of the hematopoietic stem cells. That is, by studying the long hematopoietic system repopulation capability, it is possible to determine the presence or absence of hematopoietic stem cells in the cells transplanted into the body. The competitive repopulation assay refers to a method for simultaneously transplanting cells of interest (test cells) and a certain amount of bone marrow cells (competitor cells) into a mouse which has received radiation. This method enables the recipient mouse to survive without the presence of precursor cells required for the survival of the recipient mouse for a shorter period of time, or without the presence of hematopoietic stem cells in the test cells which stem cells are required for longer survival of the recipient mouse. The relative evaluation of the hematopoietic stem cell activity in the test cells can also be made. Specifically, the following experiments were conducted.

Ly5 congenic mouse was used for the competitive repopulation method (D. A. Ogden and H. S. Micklem, (1976) Transplantation. 22: 287-293; aand D. E. Harrison et al., (1978) J. Exp. Med. 147: 1526-1531) to evaluate the prepared hematopoietic stem cells (H. Ema et al., (2000) Blood. 95: 2284-2288). In brief, $CD34^-$KSL cells or $CD34^-$KSL derived cells (B6-Ly5.1) in culture were mixed with $2 \times 10^5$ bone marrow competitor cells (B6-F1) and cultured for a predermined period of time and 200 μl of the cell suspension containing 10 $CD34^-$KSL cells were injected into the tail vein of B6-Ly5.2 mouse which received 9.5 Gy radiation (Figure 5).

After transplantation, the mouse was housed under circumstances of specific pathogen-free (SPF) for a certain period of time, and the mouse was anesthetized by ether inhalation and blood was taken from the orbital cavity vein to fill a 100 μl capillary tube (as a preliminary, a small volume of 0.1 M $EDTA \cdot Na_2/H_2O$ was taken using the capillary phenomenon). The taken blood was transferred to an Eppendorf tube and mixed well and centrifuged at 2,000 rpm for two seconds to remove the blood. 350 μl of distilled water was added thereto and left to stand for thirty second at room temperature. 350 μl of 2 x PBS was added thereto and after sufficient agitation, it centrifuged at 2,000 rpm for two minutes. The supernatant including hemolyzed erythrocytes was discarded and the blood cell pellet was suspended in SM and divided into two portions which were subjected to centrifugation.

The resultant cell pellets were stained using two kinds of antibody stains, namely biotinylated Ly5.1(A20) antibody and FTIC conjugated Ly5.2(104) antibody as described in Example 1. The cells were stained using PE conjugated B220 antibody, or PE conjugated anti-Mac-1 antibody, and PE conjugated anti-Gr-1 antibody or PE conjugated anti-CD4 antibody and PE conjugated anti-CD8 antibody, as described in Example 1. The biotinylated antibodies were developed using streptavidin-APC (Pharmingen, San Diego, USA). After incubation at 4°C for twenty minutes, the cells were washed and were resuspended in 200 μl of SM and analyzed using FACS. Ly5.1 and Ly5.2 were used for developing and cells derived from the test cells and competitor derived cells, as well as the remaining cells of the recipient were detected in a distinguishing manner. Further, the cells derived from the test cells were studied with respect to whether these were granulocytes, macrophages, B lymphocytes, and T lymphocytes.

The % chimerism was determined by using the following formula:

$$\text{Total chimerism (\%)} = \text{\% Ly5.1 cells} \times 100 / (\text{Ly5.1 cells} + \text{\%F1 cells})$$

In the present formula, positive mice are defined to be mice in which 1 % or more chimerism is shown in all of the granulocytes, macrophages, T lymphocytes, and B lymphocytes of the blood system; and negative mice are defined to be mice other than the positive mice.

(2. Effects of the WIF-1 on the maintenance of hematopoietic stem cells)

The SCF and WIF-1 are used to study the maintenance of hematopoietic stem cells using the method described above in 1. The results are shown in Table 3.

Table 3:

| The maintenance of hematopoietic stem cells by WIF-1 | | | | |
|---|---|---|---|---|
| Agent(s) | Culture period | Period after transplantation | Number of positive mice | % chimerism (number of mice) |
| First Round | | | | |
| none | 0 days | 12 weeks | 3/3 | 10.1±7.2(3) |
| SCF | 5 days | 12 weeks | 0/2 | ND |
| SCF+WIF-1 | 5 days | 12 weeks | 8/10 | 34.3±17.7(8) |
| Second Round | | | | |
| none | 0 days | 12 weeks | 7/10 | 24.3±16.8(7) |
| SCF | 5 days | 12 weeks | 0/10 | ND |
| SCF+WIF-1 | 5 days | 12 weeks | 4/10 | 15.8±15.3(4) |
| ND: not detectable | | | | |

As shown in Table 3, WIF-1 in combination with SCF, have lead to the maintenance of the hematopoietic stem cells during the culture period, which was not observed when SCF alone was used.

Next, TPO in combination with SCF (SCF+TPO) was compared with the combination of WIF-1 with SCF and TPO (SCF+TPO+WIF-1).

Further, as shown in Table 4, when no WIF-1 was added but SCF and TPO were added in combination to the $CD34^-$KSL cells culture, no reproducible hematopoietic stem cell maintenance was observed, whereas when WIF-1 was combined with SCF and TPO, the maintenance of hematopoietic stem cells was observed in a reproducible and highly reliable manner.

Table 4:

| The maintenance of hematopoietic stem cells by WIF-1 | | | | |
|---|---|---|---|---|
| Agent(s) | Culture period | Period after transplantation | Number of positive mice | % chimerism (number of mice) |
| First Round | | | | |
| none | 0 days | 24 weeks | 6/10 | 5.6±5.2(6) |
| SCF+TPO | 3 days | 24 weeks | 3/10 | 7.0±6.1(3) |
| SCF+TPO+ WIF-1 | 3 days | 24 weeks | 6/10 | 14.8±11.6(6) |
| Second Round | | | | |
| none | 0 days | 12 weeks | 3/3 | 10.1±7.2(3) |
| SCF+TPO | 6 days | 12 weeks | 0/10 | ND |
| SCF+TPO+ WIF-1 | 6 days | 12 weeks | 10/10 | 15.0±15.0(10) |
| ND: not detectable | | | | |

(Example 6: Gene introduction into a stem cell in the presence of WIF-1)

After the separation of CD34-KSL cells from mouse bone marrow cells, GFP (Green Fluorescence Protein) was introduced to 300 to 600 cells of $CD34^-$KSL cells as a marker gene in the presence or absence of the WIF-1, and the resultant cells were transplanted into five mice which received radiation treatment.

Preparation of retrovirus: 293T cell lines and the Packaging Cell lines PG13, 293GP and 283 GPg were cultured in a DMEM supplemented with 10 % FCS. pGCsa-pEGFP (a retrovirus vector provided by Dr. Masafumi ONODERA at Tsukuba University) was transfected into 293 GP cells using the phosphate calcium method. The culture supernatant comprising the resultant viral particles was collected and infected into the PG13 cells. Further, the culture supernatant of the PG13 cell line was used to infect 293GPg cells (tetracyclin susceptable expression induction system:

tet-off). Culture supernantant obtained by switching into Tet off was centrifuged at 6,000 x g for sixteen hours, and the resultant viral particles were resuspended in Stem-Pro34 serum-free culture media.

Preparation of lentivirus: pCS-CG-PRE(a SIN vector in which a woodchuck posttranscriptional regulatory element was inserted downstream of the GFP), pMDLg/pPRE(packaging construct), pRSV-Rev (Revexpressing construct), pMD.G(VSV-G-expressing construct) were available by Dr. Hiroyuki MIYOSHI at Tsukuba University. These plasmids were transfected into the 293T cell line simultaneously using the calcium phosphate method. Culture supernatant was collected and ultracentrifuged to obtain a viral particle concentrate which was used for infection experiments.

In the presence of 100 ng/ml SCF and 100 ng/ml TPO, D34-KSL cells were cultured in the absence of serum in 96-well plates which wells were coated with vitronectin (TaKaRa) as a control. Further, under the same conditions, 1 ng/ml of WIF-1 was added to additional cutlures (pre-incubation). Retroviral particle concentrates were added to the culture with 5 μg/ml protamine sulfate (Sigma, St. Louis, USA) after 24 hours of culture. After 48 hours, the resultant culture cells were transplanted into mice which had received radiation treatment (competitive hematopoietic system repopulation assay).

In the presence of 100 ng/ml SCF and 100 ng/ml FL, D34-KSL cells were cultured in the absence of serum in a 96-well plates which wells were coated with vitronectin (TaKaRa) as a control. Further, under the same conditions, 1 ng/ml of WIF-1 was added to additional cultures (pre-incubation). Lentivirus particle concentrates were added to the culture with 5 μg/ml protamine sulfate (Sigma, St. Louis, USA) at the beginning of culture. Within 24 hours, the resultant culture cells were transplanted into mice which had received radiation treatment (competitive hematopoietic system repopulation assay).

Repopulation by the cultured cells three months post-transplantation and the gene introduction efficiency into the repopulated blood cells were evaluated. As a result, it became apparent that the group which received the WIF-1 at the time of viral infection had a significantly higher gene introduction rate than the others.

(Example 7: Large scale production of mouse and human WIF-1 recombinant proteins)

In order to study the effects of WIF-1 *in vivo,* it is necessary to produce a large amount of purified protein. Accordingly, cells stably producing mouse and human WIF-1 recombinant proteins were established. Mouse or human cDNA were inserted into the pcDNA 3.1 expression vector (Invitrogen, Groningen, The Netherlands) (pcDNA3.1/mWIF-1, pcDNA3.1/hWIF-1). pcDNA3.1/mWIF-1 and pcDNA3.1/hWIF-1 were transduced into CHO cell lines using calcium phosphate method, and the cells were cultured using DMEM (Dulbecco's Modified Eagle Medium) supplemented with 10 % FCS in the presence of 5% $CO_2$ at 37°C.

Thereafter, a drug resistant cell was obtained by culturing in the presence of Zeosin (Invitrogen, Groningen, The Netherlands). Then a single cell was sorted using FACS. Cell lines were thus cloned. Then the Western blotting method was used to select clones which express WIF recombinant proteins highly.

Clones with high expression were cultured in DMEM (Dulbecco's Modified Eagle Medium) supplemented with 10% FCS in the presence of 5% $CO_2$ at 37°C to produce mouse and human WIF-1 recombinant proteins in a large amount.

(Example 8: The effect of conservatively substituted variants of WIF-1)

In the sequence set forth in SEQ ID NO: 2, variants were made by site directed mutagenesis in which the amino acid number 55 isoleucine was substituted with leucine, the amino acid number 109 leucine was substituted with isoleucine, the amino acid number 244 alanine was substituted with valine, the amino acid number 271 glutamic acid was substituted with aspartic acid (SEQ ID Nos: 15, 17, 19 and 21, respectively). In the site directed mutagenesis, the Quick Change™ kit (Stratagene) was used according to the manual of the manufacturer. In brief, the following was conducted.

1. Double-stranded plasmid DNA was heat denatured and two kinds of primer having mutations introduced therein were annealed to the same base sequence site of the respective complementary DNA strands.

2. The Pfu Turbo DNA Polymerase was used to elongate the primers using the temperature cycling method (Cycling number: 10-16) to synthesize circular DNA strands with nicks introduced therein having the mutation.

3. Dpn I was used to degrade the template DNA. DNA prepared from an *E. coli* cell line which does not requires dam methylase, has methylated, and was degraded by Dpn I which is specific to methylated and hemimethylated DNAs (5' - $^{GM6}$ATC-3'). On the other hand, DNA with the mutation introduced therein is not degraded.

4. An *E. coli* was transformed with the DNA having the nicked mutation. The nick in the plasmid having the mutation was recovered in the E. coli.

Four variants (the nucleotide sequences were set forth in SEQ ID NOs: 14, 16, 18 and 20) were prepared by the above-described method and used for the following experiments.

(The effect of WIF-1 variants against hematopoietic stem cells)

The experiments were conducted as described in Examples 4 and 5, and the effects on the propagation and differentiation of hematopoietic stem cells and the maintance of the hematopoietic stem cells transplanted in vivo, were confirmed.

As for the wild-type sequences, in combination with SCF, the WIF-1 variants showed induction of both cell division and colony formation of $CD34^-KSL$ cells which are hematopoietic stem cells. Accordingly, the WIF-1 variants were demonstrated to have induced both cell proliferation and differentiation of hematopoietic stem cells when combined with SCF. Further, the WIF-1 variants were demonstrated to have augmented the effects of FL which is another factor to induce both proliferation and differentiation of hematopoietic stem cells.

The WIF-1 variants, when combined with SCF, have led to the maintenance of the transplanted hematopoietic cells, which was not observed in the case where only SCF is used.

The above-identified four kinds of variants showed the maintenance of hematopoietic stem cells in a reproducible manner and in a high probability when combined with SCF and TPO.

Accordingly, it was demonstrated that the conservatively substituted variants showed effects attained by the present invention in a manner similar to the wild-type sequences.

(Example 9: The importance of domains)

Next, a variant in which the WIF domain was left, and the other regions were deleted and a variant in which the EGF-like repeat was left and the other regions were deleted, were prepared. Specifically, a variant in which a 6His-Tag was added to the first amino acid numbers 1 to 176 of SEQ ID NO: 2 (the nucleotide sequence thereof is shown in SEQ ID NO: 22, and the amino acid sequence thereof is shown in SEQ ID NO: 23), and a variant in which signal sequence (amino acid numbers 1 to 24) and the EGF-like repeat (amino acid numbers 177 to 379) were fused and a 6His-tag was added (the nucleotide sequence thereof is shown in SEQ ID NO: 24, and the amino acid sequence thereof is shown in SEQ ID NO: 25), were prepared in accordance with well known technologies.

(The importance of domains on hematopoietic stem cells)

Experiments were conducted as described in Examples 4 and 5 to confirm the effects on propagation and differentiation of hematopoietic stem cells, and the maintenance of hematopoietic stem cells transplanted *in vivo*.

As for the wild type sequences, in combination with SCF, the WIF-1 variants showed induction of both cell division and colony formation of $CD34^-KSL$ cells which are hematopoietic stem cells. Accordingly, the WIF-1 variants were demonstrated to have induced both cell proliferation and differentiation of hematopoietic stem cells when combined with SCF. Further, the WIF-1 variants were demonstrated to have augmented the effects of FL which is another factor to induce both proliferation and differentiation of hematopoietic stem cells.

The WIF-1 variants, when combined with SCF, have led to the maintenance of the transplanted hematopoietic cells, which were not observed in the case where only SCF is used.

The above-identified variants showed the maintenance of hematopoietic stem cells in a reproducible manner and in a high probability when combined with SCF and TPO.

The variant having only the WIF domain, and that having only the EGF-like repeat, have been shown to have reduced effects compared to the wild-type sequences. Accordingly, therefore it is understood that both domains should be included in practicing the present invention.

Accordingly, it was demonstrated that the conservatively substituted variants showed the effects to be attained by the present invention in a similar manner to the wild-type sequences.

INDUSTRIAL APPLICABILITY

The present invention attained the maintenance of the pluripotency of stem cells while maintaining the undifferentiated state thereof. This enabled the easy provision of stem cells (for example, hematopoietic stem cells) in a large amount. In addition, the stem cell composition of the present invention is homogenous and impurities therein are significantly reduced. Therefore, the present invention is shown to have significantly improved side effects such as infection, rejection response and the like which haven been problematic in conventional treatments using hematopoietic stem cells. Further, it was shown that the present invention can be applied to effective gene therapies. These can be recognized to be significant effects. Further, compositions, cells, kits, medicaments and the like used for the treatment methods of the present invention have sufficient industrial applicability. The present invention is considered to have sufficient industrially applicability and utilities since for example, pharmaceutical industries except for medical doctors can produce the composition of the present invention as an industry. The present invention *per se* also has industrial applicability since it is useful for clinical trials *per se* which are of industrial purpose *per se,* in addition to medical treatment methods which are for pure medical purposes. Further, the indirect and direct workings of the methods of the present invention are considered to have the possibility of use in industries in the vicinity of medical industry, there is/are sufficient industrial applicability or utilities.

**Claims**

1. A polypeptide having a WIF domain which maintains pluripotency without differentiating a stem cell.

2. The polypeptide according to claim 1, wherein the WIF domain comprises at least five amino acids among about position 30 to about position 180 of the sequence set forth in SEQ ID NO: 4.

3. The polypeptide according to claim 1, wherein the WIF domain comprises the sequence of about position 30 to about position 180 of the sequence set forth in SEQ ID NO: 4.

4. The polypeptide according to claim 1, wherein the polypeptide further includes an EGF like repeat.

5. The polypeptide according to claim 4, wherein the EGF like repeat comprises at least one repeat consisting of $CX_3CX_5CX_5CXCX_8CX_4$ where C is cysteine and X is any amino acid.

6. The polypeptide according to claim 1, wherein the polypeptide has the sequence set forth in SEQ ID NO: 4.

7. The polypeptide according to claim 1, wherein the stem cell is a hematopoietic stem cell.

8. A composition for maintaining pluripotency without differentiating a stem cell comprising a polypeptide having a WIF domain.

9. The composition according to claim 8, wherein the polypeptide further has an EGF like repeat.

10. The composition according to claim 8 wherein the polypeptide has the sequence set forth in SEQ ID NO: 4.

11. The composition according to claim 8 further comprising a stem cell survival agent.

12. The composition according to claim 8, wherein the stem cell survival agent is stem cell factor (SCF).

13. A stem cell which does not differentiate in vitro and maintains pluripotency.

14. The stem cell according to claim 13 which is a hematopoietic stem cell.

15. The stem cell according to claim 13 wherein the period of said pluripotency maintenance is at least six days.

16. The stem cell according to claim 13, wherein the pluripotency comprises a capability of differentiating into blood cells.

17. A long period pluripotency maintaining cell composition comprising a stem cell and a polypeptide having a WIF domain.

18. The long period pluripotency maintaining cell composition according to claim 17, wherein the polypeptide further comprises an EGF like repeat.

19. The long period pluripotency maintaining cell composition according to claim 17, wherein the stem cell is hematopoietic cell.

20. The long period pluripotency maintaining cell composition according to claim 17, wherein at least $10^2$ cells of the stem cell exist therein.

21. The long period pluripotency maintaining cell composition according to claim 17, wherein the polypeptide having the WIF domain comprises the sequence set forth in SEQ ID NO: 4.

22. The long period pluripotency maintaining cell composition according to claim 17, wherein the polypeptide having WIF domain is present at least at 0.1 ng/ml therein.

23. The long period pluripotency maintaining cell composition according to claim 17 further comprising stem cell survival agent.

**24.** The long period pluripotency maintaining cell composition according to claim 23, wherein the stem cell survival agent is SCF.

**25.** The long period pluripotency maintaining cell composition according to claim 23, wherein the stem cell survival agent is FLT-3 ligand.

**26.** The long period pluripotency maintaining cell composition according to claim 23, wherein the stem cell survival agent is present at least at 1 ng/ml therein.

**27.** The long period pluripotency maintaining cell composition according to claim 23 for preparing differentiated cells wherein the differentiated cells are used for treating disorders of blood cells.

**28.** A method for maintaining pluripotency without differentiating a stem cell, comprising the step of: 1) providing the stem cell with a polypeptide having a WIF domain.

**29.** The method according to claim 28 wherein the polypeptide further comprises an EGF like domain.

**30.** The method according to claim 28 wherein the stem cell is a hematopoietic cell.

**31.** The method according to claim 28 wherein the stem cell is present at least at $10^2$ cells.

**32.** The method according to claim 28 wherein the polypeptide having the WIF domain comprises the sequence set forth in SEQ ID NO: 4.

**33.** The method according to claim 27 wherein the polypeptide having the WIF domain is present at least at 0.1 ng/ml.

**34.** The method according to claim 27 further comprising the step of:

2) providing a stem cell survival agent with the stem cell.

**35.** The method according to claim 34, wherein the stem cell survival agent is SCF.

**36.** The method according to claim 34, wherein the stem survival agent is FLT-3 ligand.

**37.** The method according to claim 34 wherein the stem cell survival agent is present at least at 1 ng/ml.

**38.** A method for producing a long period pluripotency maintaining cell composition comprising the steps of:

1) providing a stem cell;
2) treating the stem cell with a polypeptide having a WIF domain; and
3) collecting the stem cell treated.

**39.** The method according to claim 38 wherein the polypeptide having the WIF domain further comprises an EGF like repeat.

**40.** The method according to claim 38 wherein the stem cell is a hematopoietic stem cell.

**41.** The method according to claim 38 wherein the stem cell is present at least at $10^2$ cells.

**42.** The method according to claim 38, wherein the polypeptide having the WIF domain comprises the sequence set forth in SEQ ID NO: 4.

**43.** The method according to claim 38, wherein the polypeptide having the WIF domain is present at least at 0.1 ng/ml.

**44.** The method according to claim 38 further comprising the step of

2) providing the stem cell with a stem cell survival agent.

**45.** The method according to claim 44 wherein the stem cell survival agent is SCF.

**46.** The method according to claim 44 wherein the stem cell survival agent is flt-3 ligand.

**47.** The method according to claim 44, wherein the stem cell survival agent is present at least at 1 ng/ml.

**48.** A method for treating a disease or disorder originating from a disorder of a differentiated cell, comprising the steps of:

1) administering a long period pluripotency maintaining cell composition to a subject wherein the long period pluripotency maintaining cell composition comprises:

a stem cell; and
a polypeptide having a WIF domain.

**49.** The method according to claim 48, wherein the polypeptide having the WIF domain further comprises an EGF-like repeat.

**50.** The method according to claim 48 wherein the differentiated cell is a blood cell.

**51.** The method according to claim 48 wherein the stem cell is a hematopoietic cell.

**52.** The method according to claim 48 wherein the stem cell is present at least at $10^2$ cells.

**53.** The method according to claim 48 wherein the polypeptide having the WIF domain comprises the sequence set forth in SEQ ID NO: 4.

**54.** The method according to claim 48 wherein the polypeptide having the WIF domain is present at least at 0.1 ng/ml.

**55.** The method according to claim 48 wherein the long period pluripotency maintaining cell composition further comprises a stem cell survival agent.

**56.** The method according to claim 55 wherein the stem cell survival agent is SCF.

**57.** The method according to claim 55 wherein the stem cell survival agent is flt-3 ligand.

**58.** The method according to claim 55, wherein the stem cell survival agent is present at least at 1 ng/ml.

**59.** The method according to claim 48 further comprising the step of differentiating the stem cell.

**60.** The method according to claim 48 wherein the subject is a human.

**61.** The method according to claim 48 wherein the polypeptide having the WIF domain is a human recombinant WIF-1 comprising the sequence set forth in SEQ ID NO: 4.

**62.** A pharmaceutical composition for treating a disease or disorder originating from a disorder of a differentiated cell comprising:

a stem cell;
a polypeptide having a WIF domain; and
a pharmaceutically acceptable carrier.

**63.** A pharmaceutical composition according to claim 62 wherein the polypeptide further comprises an EGF-like repeat.

**64.** The pharmaceutical composition according to claim 62 wherein the differentiated cell is a blood cell.

**65.** The pharmaceutical composition according to claim 62 wherein the stem cell is a hematopoietic cell.

**66.** The pharmaceutical composition according to claim 62 wherein the stem cell is present at least at $10^2$ cells.

**67.** The pharmaceutical composition according to claim 62 wherein the polypeptide having the WIF domain is WIF-1.

**68.** The pharmaceutical composition according to claim 62 wherein the polypeptide having the WIF domain is present at least at 0.1 ng/ml.

**69.** The pharmaceutical composition according to claim 62 further comprising a stem cell survival agent.

**70.** The pharmaceutical composition according to claim 62, wherein the stem survival agent is SCF.

**71.** The pharmaceutical composition according to claim 69, wherein the stem cell survival agent is flt-3 ligand.

**72.** The pharmaceutical composition according to claim 69 wherein the stem cell survival agent is present at least at 1 ng/ml.

**73.** The pharmaceutical composition according to claim 69 wherein the disease or disorder is a human disease or disorder.

**74.** The pharmaceutical composition according to claim 62 wherein the polypeptide having the WIF domain is recombinant human WIF-1 comprising the sequence set forth in SEQ ID NO: 4.

**75.** Use of a polypeptide having a WIF domain for maintaining the pluripotency of a stem cell without differentiation.

**76.** The use according to claim 75 wherein the polypeptide further comprises an EGF like repeat.

**77.** The use according to claim 75, wherein the stem cell is a hematopoietic cell.

**78.** The use according to claim 75, wherein the stem cell is present at least at $10^2$ cells.

**79.** The use according to claim 75, wherein the polypeptide having the WIF domain comprises the sequence set forth in SEQ ID NO: 4.

**80.** The use according to claim 75, wherein the polypeptide having the WIF domain is present at least at 0.1 ng/ml.

**81.** The use according to claim 75, combined with the use of a stem cell survival agent.

**82.** The use according to claim 81, wherein the stem cell survival agent is SCF.

**83.** The use according to claim 81, wherein the stem cell survival agent is flt-3 ligand.

**84.** The use according to claim 81, wherein the stem cell survival agent is present at least at 1 ng/ml.

**85.** The use according to claim 75 wherein the disease or disorder is a human disease or disorder.

**86.** The use according to claim 75 wherein the polypeptide having the WIF domain is human recombinant WIF-1 comprising the sequence set forth in SEQ ID NO: 4.

Figure 1A

```
mWIF-1    1  MARRRAFPAFR--IRLWSIL-PCLLLLRADAGQPPEESLYLWIDAHQARVLIGFEEDILI   57
hWIF-1    1  MARRSAFPAAA--LWLWSIL-LCLLALRAEAGPPQEESLYLWIDAHQARVLIGFEEDILI   57
rWIF-1    1  MARRRAFPAFV--IRLWSIL-PCLLLLRADAGQPPEESLYLWIDAHQARVLIGFEEDILI   57
xWIF-1    1  MSLTGYHAAP---LC--SIFLF-ILAHADA-GGQED-SLYMWIDAHQARVLIGFEEDILI   52
zWIF-1    1  ---MA-SRTPSVQLHLKACVLLL-LGGLLEAAYQERGTMMWIDANQARILIGFEEDILI   55

mWIF-1   58  VSEGKMAPFTHDFRKAQQRMPAIPVNIHSMNFTWQAAGQAEYFYEFLSLRSLDKGIMADP  117
hWIF-1   58  VSEGKMAPFTHDFRKAQQRMPAIPVNIHSMNFTWQAAGQAEYFYEFLSLRSLDKGIMADP  117
rWIF-1   58  VSEGKMAPFTHDFRKAQQRMPAIPVNIHSMNFTWQASGQAEYFYEFLSLRSLDKGIMADP  117
xWIF-1   53  VAEGKMAPFTHDFRKAQQRMPAIPVNIHAMNFTWQATGQAEYFYEFLSLRSLDKGIMADP  112
zWIF-1   56  VSEGKMAPFTHDFRKAQQRMPAIPVNIHHVNFTWQATDQAEYFYEFQTLRSLDKDIMDDP  115

mWIF-1  118  TVNVPLLGTVPHKASVVQVGFPCLGKQDGVAAFEVNVIVMNSEGNTILRTPQNAIFFKTC  177
hWIF-1  118  TVNVPLLGTVPHKASVVQVGFPCLGKQDGVAAFEVDVIVMNSEGNTILQTPQNAIFFKTC  177
rWIF-1  118  TVNVPRLGTVPHKASVVQVGFPCLGKQDGVAAFEVNVIVMNSEGNPILRTPQNAIFFKTC  177
xWIF-1  113  TVNMPLLGTVPHKATVIQVGFPCLGNQDGVAAFEVNVIVMNSEGNVILQTPQNAIFFKTC  172
zWIF-1  116  TVNVPLLGSVPHKASVVQVGFPCRGDDDGVAAFEVTILVMDAGGNIILRTPHNAIFFKTC  175
```

Figure 1B

```
mWIF-1   178  QQAECPGGCRNGGFCNERRVCECPDGFYGPHCEKALCIPRCMNGGLCVTPGFCICPPGFY   237
hWIF-1   178  LQAECPGGCRNGGFCNERRICECPDGFHGPHCEKALCTPRCMNGGLCVTPGFCICPPGFY   237
rWIF-1   178  QQAECPGGCRNGGFCNERRVCECPDGFYGPHCEKALCIPRCMNGGLCVTPGFCICPPGFY   237
xWIF-1   173  QQAKCTGGCRNGGFCNDRHVCECPDGFYGPHCEKALCMPRCMNGGLCVTPGLCICPPGYY   232
zWIF-1   176  QRAKCPGGCRNGGYCNERQVCECQDGFYGVHCEKALCSPRCLNGGLCMSPGVCICPPGYF   235

mWIF-1   238  GVNCDKANCSTTCFNGGTCFYPGKCICPPGLEGEQCELSKCPQPCRNGGKCIGKSKCKCP   297
hWIF-1   238  GVNCDKANCSTTCFNGGTCFYPGKCICPPGLEGEQCEISKCPQPCRNGGKCIGKSKCKCS   297
rWIF-1   238  GVNCDKANCSATCFNGGTCFYPGKCICPPGLEGEQCELSKCPQPCRNGGKCIGKSKSV--   295
xWIF-1   233  GINCDKVNCTTHCLNGGTCFYPGKCICHSGYEGEQCETSKCQQPCRNGGKCSGKNKCKCS   292
zWIF-1   236  GSSCERANCSTTCLNGGTCFHPGKCICAVSFEGVRCELSKCRQPCRNGGKCTGRNKCKCS   295

mWIF-1   298  KGYQGDLCSKPVCEPGCGAHGTCHEPNKCQCREGWHGRHCNKRYGASLMHAPRPAGAGLE   357
hWIF-1   298  KGYQGDLCSKPVCEPGCGAHGTCHEPNKCQCQEGWHGRHCNKRYEASLIHALRPAGAQLR   357
rWIF-1   296  ------------CEPGCGAHGTCHEPNKCQCREGWHGRHCNKRYGASLMHAPRPAGAGLE   343
xWIF-1   293  KGYQGDLCSKPVCEPSCGAHGTCIEPNKCQCKEGWNGRYCNKKYGSNLMNALRPTGSRNR   352
zWIF-1   296  KGYHGDLCSKAVCEPSCGAHGTCVEPNRCQCREGWHGRHCNKRFRGGVSNSQRVSPSKHK   355

mWIF-1   358  RHTPSLKKAEDRRDPPES-NYIW   379
hWIF-1   358  QHTPSLKKAEERRDPPES-NYIW   379
rWIF-1   344  RHTPSLKKAEGRRDPPES-NYIW   365
xWIF-1   353  QHTPSPKRTEDRQAIPES-NYIW   374
zWIF-1   356  SPSVAAAKEAPETSQHSETNYVV   378
```

Figure 2

Excision of femur and tibia from mouse

↓

Preparing bone marrow suspension

↓

Isolation of monocyte with gravity centrifuge using Ficoll

↓

Staining with Lineage antibody

↓

Binding to Magnetic beads

↓

Removing Lineage positive cell with Magnetic Stand

↓

Staining by Lineage antibody, c-Kit antibody,
Sca-1 antibody, CD34 antibody

↓

FACS analysis and Sorting

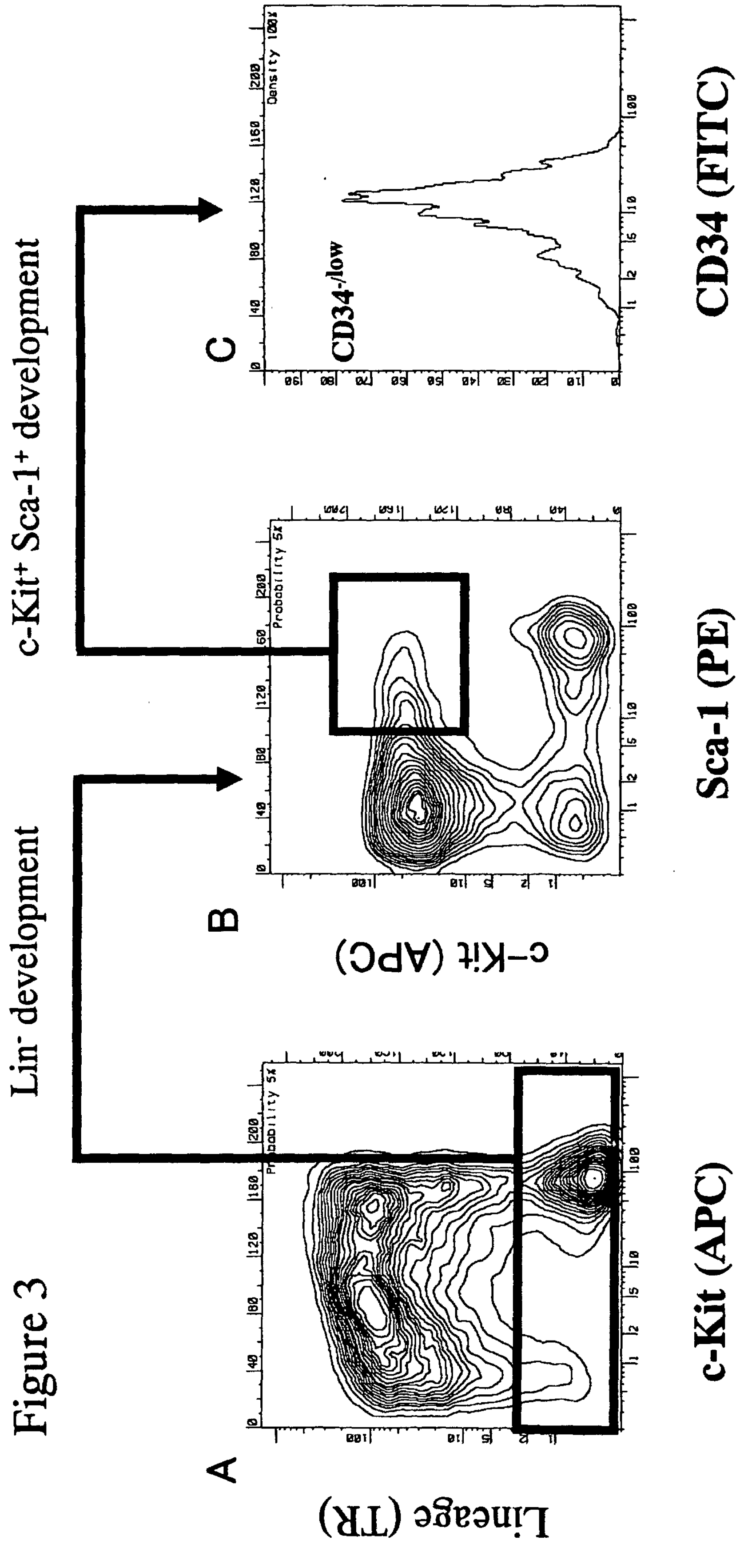
Lin- development
c-Kit+ Sca-1+ development
A
B
C
Lineage (TR)
c-Kit (APC)
c-Kit (APC)
Sca-1 (PE)
CD34 (FITC)
CD34-/low
Probability 5%
Density 100%

Figure 3

Figure 4

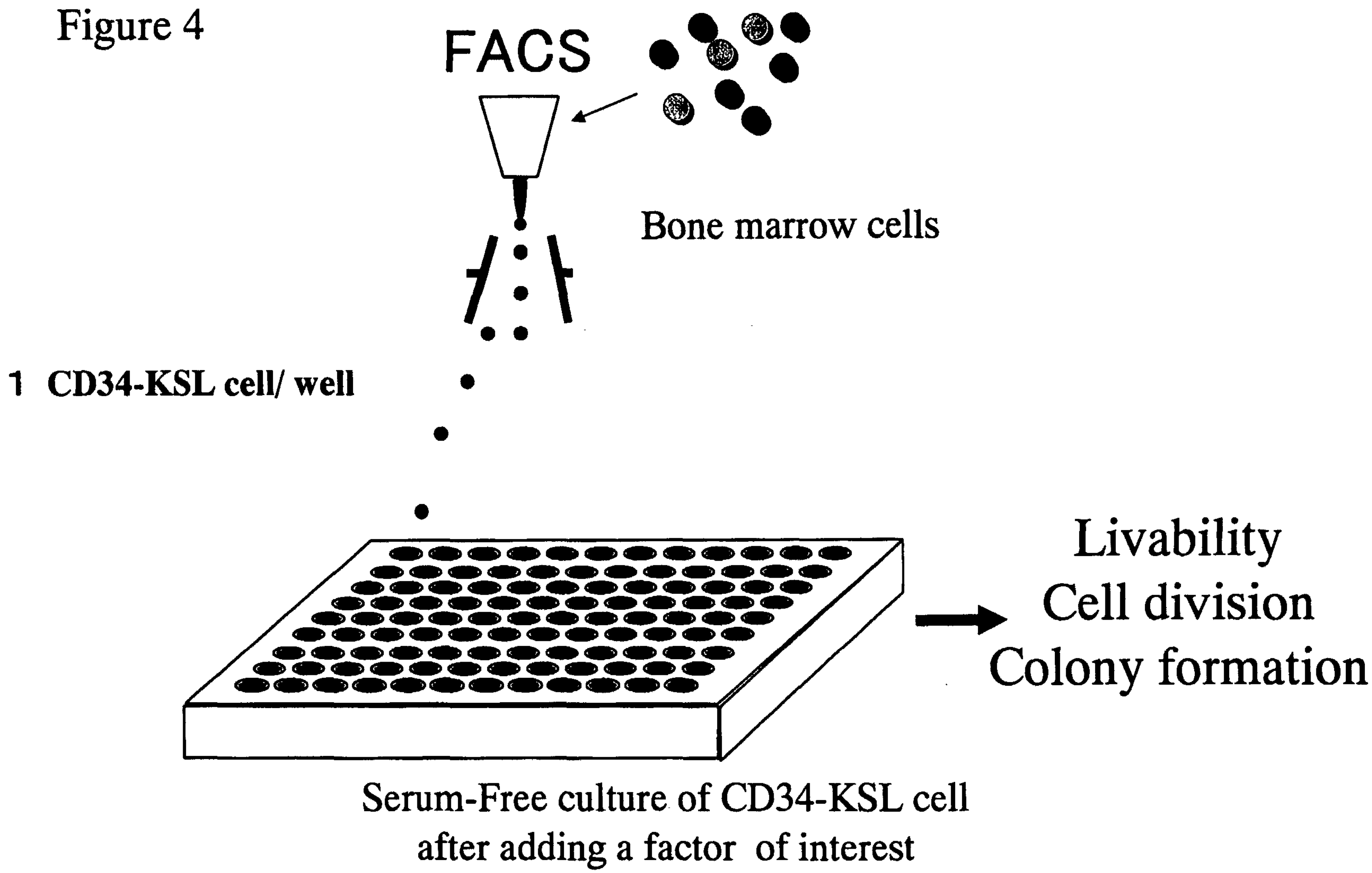
FACS
Bone marrow cells
1 CD34-KSL cell/ well
Serum-Free culture of CD34-KSL cell
after adding a factor of interest
Livability
Cell division
Colony formation

Figure 5

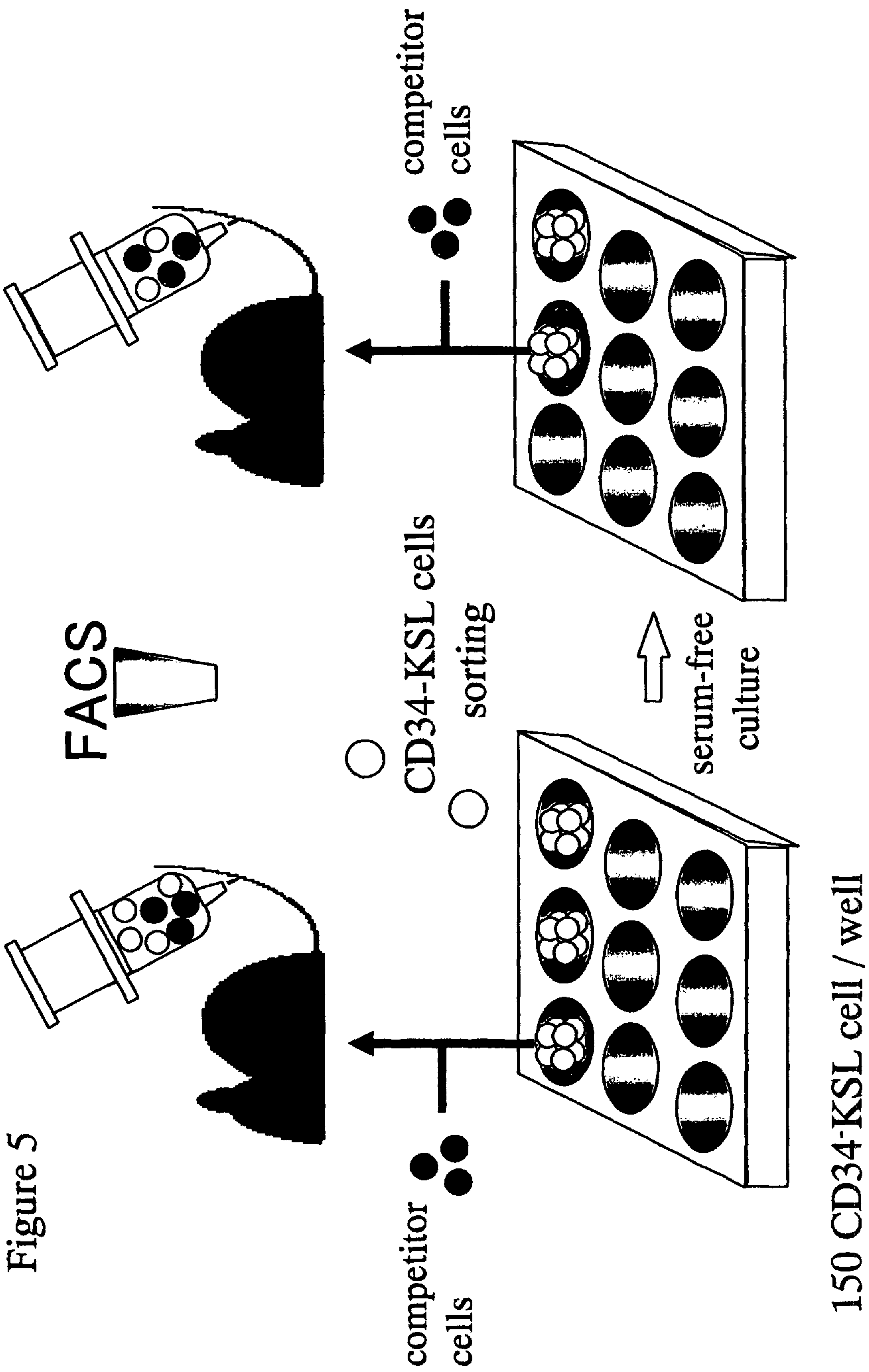

competitor cells
FACS
CD34-KSL cells sorting
serum-free culture
competitor cells
150 CD34-KSL cell / well

| INTERNATIONAL SEARCH REPORT | International application No. PCT/JP02/02265 |
|---|---|

A. CLASSIFICATION OF SUBJECT MATTER
Int.Cl[7] C12N15/09, C07K14/475, C12N5/10, A61K38/17, A61P43/00, A61K35/14, A61K35/12

According to International Patent Classification (IPC) or to both national classification and IPC

B. FIELDS SEARCHED

Minimum documentation searched (classification system followed by classification symbols)
Int.Cl[7] C12N15/09, C07K14/475, C12N5/10

Documentation searched other than minimum documentation to the extent that such documents are included in the fields searched

Electronic data base consulted during the international search (name of data base and, where practicable, search terms used)
MEDLINE(STN), WPI(DIALOG), BIOSIS(DIALOG), JICST FILE(JOIS), GenBank/EMBL/DDBJ/GeneSeq, SwissProt/PIR/GeneSeq

C. DOCUMENTS CONSIDERED TO BE RELEVANT

| Category* | Citation of document, with indication, where appropriate, of the relevant passages | Relevant to claim No. |
|---|---|---|
| X<br>A | HSIEH, JC. et al., A new secreted protein that binds to Wnt proteins and inhibits their activities Nature(1999), Vol.398, pages 431 to 436 | 1-7<br>8-47,62-74 |
| X<br>Y | WO 00/05374 A2 (Incyte Pharm Inc.), 03 February, 2000 (03.02.00), Full text & AU 9951246 A & EP 1095142 A2 | 1-7<br>8-47,62-74 |
| Y | DROUET, M. et al., Cell Cycle Activation of Peripheral Blood Stem and Progenitor Cells Expanded Ex Vivo with SCF, FLT-3 Ligand, TPO, and IL-3 Results in Accelerated Granulocyte Recovery in a Baboon model of Autologous Transplantation but $G_0/G_1$ and $S/G_2/M$ Graft Cell Content Does Not Correlate with Transplantability Stem Cells(2001), Vol.19, No.5, pages 436 to 442 | 8-47,62-74 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 29 May, 2002 (29.05.02) | 11 June, 2002 (11.06.02) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP02/02265

C (Continuation). DOCUMENTS CONSIDERED TO BE RELEVANT

| Category* | Citation of document, with indication, where appropriate, of the relevant passages | Relevant to claim No. |
|---|---|---|
| Y | DROUET, M. et al., The Reduction of In Vitro Radiation-Induced Fas-Related Apoptosis in $CD34^+$ Progenitor Cells by SCF, FLT-3 Ligand, TPO, and IL-3 in Combination Resulted in $CD34^+$ Cell Proliferation and Differentiation Stem Cells(1999), Vol.17, No.5, pages 273 to 285 | 8-47,62-74 |
| A | DEASY, B. M. et al., Mechanisms of Mustle Stem Cell Expansion with Cytokines Stem Cells(January 2002), Vol.20, No.1, pages 50 to 60 | 1-7,8-47, 62-74 |
| X<br>A | Hideo EMA et al., Zoketsu Kansaibo no Junka to Clonal na Kaiseki, Jikken Igaku (2001), Vol.19, No.15, pages 1977 to 1981 | 13-16<br>1-12,17-47, 62-74 |

Form PCT/ISA/210 (continuation of second sheet) (July 1998)

## INTERNATIONAL SEARCH REPORT

International application No.
PCT/JP02/02265

**Box I Observations where certain claims were found unsearchable (Continuation of item 1 of first sheet)**

This international search report has not been established in respect of certain claims under Article 17(2)(a) for the following reasons:

1. [X] Claims Nos.: 48-61, 75-85
because they relate to subject matter not required to be searched by this Authority, namely:
The inventions as set forth in the above claims pertain to methods for treatment of the human body by therapy, as well as diagnostic methods, and thus relate to a subject matter which this International Searching Authority is not required, under the provisions of (continued to extra sheet)

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:
The inventions as set forth in claims 1 to 47 and 62 to 74 relate to a peptide having a WIF domain, a pluripotency-holding stem cell composition and a method of holding the pluripotency of stem cells with the use of a WIF peptide. The invention as set forth in claims 13 to 16 relate to stem cells which are not differentiated *in vitro* and hold the pluripotency. The "stem cells which are not differentiated *in vitro* and hold the pluripotency" involve embryonic stem cells and precultured hematopoietic stem cells. It had been well known that embryonic stem cells hold pluripotency. Also, it had been publicly known prior to the application of the present case that hematopoietic stem cells hold pluripotency (continued to extra sheet)

1. [X] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[X] No protest accompanied the payment of additional search fees.

Form PCT/ISA/210 (continuation of first sheet (1)) (July 1998)

INTERNATIONAL SEARCH REPORT

International application No.
PCT/JP02/02265

Continuation of Box No.I-1 of continuation of first sheet(1)

Article 17(2)(a)(i) of the PCT and Rule 39.1(iv) of the Regulations under the PCT, to search.

Continuation of Box No.II of continuation of first sheet(1)

(Jikken Igaku, Vol. 19, No. 15, p.1977-1981).

Such being the case, there is no special technical matter in the meaning as defined in PCR Rule 13.2 common to all of the claims. Therefore, it is recognized that the inventions as set forth in claims 1 to 47 and 62 to 74 are made up of two groups of inventions, i.e., a group of the inventions as set forth in claims 13 to 16 and another group of the inventions as set forth in claims 1 to 12, 17 to 47 and 62 to 74.

Form PCT/ISA/210 (extra sheet) (July 1998)